# EUROPEAN PATENT SPECIFICATION

(11) **EP 0 986 384 B1**
(45) Date of publication and mention of the grant of the patent: **22.02.2012**
(21) Application number: 98922122.1
(22) Date of filing: 07.05.1998
(51) Int. Cl.: A61K 31/38, A61K 31/39, A61K 31/40, A61K 31/415, A61K 31/42, A61K 31/425, A61K 31/44, A61K 31/445, A61K 31/495, A61K 31/505, A61K 31/095, A61K 31/18, C07D 207/09, C07D 233/54, C07D 239/24, C07D 241/04, C07D 263/02, C07D 277/28, C07D 307/00, C07D 333/00, C07D 209/10, C07C 303/00, C07C 307/00, C07C 309/00, C07C 311/00

(54) **INHIBITORS OF PROTEIN ISOPRENYL TRANSFERASES**
ISOPRENTRANSFERASE PROTEINE INHIBITOREN
INHIBITEURS DE PROTEINES ISOPRENYLE TRANSFERASES

(30) Priority: 07.05.1997 US 852858
(43) Date of publication of application: 22.03.2000
(73) Proprietor: UNIVERSITY OF PITTSBURGH, Pittsburgh, Pennsylvania 15260 (US)
(72) Inventor: SEBTI, Said, M., Tampa, FL 33647 (US); HAMILTON, Andrew, D., Guilford, CT 06437 (US); AUGERI, David, J., Kenosha, WI 53143 (US); BARR, Kenneth, J., San Francisco, CA 94115 (US); DONNER, Bernard, G., Mundelein, IL 60060 (US); FAKHOURY, Stephen, A., Mundelein, IL 60060 (US); JANOWICK, David, A., Beach Park, IL 60087 (US); KALVIN, Douglas, M., Buffalo Grove, IL 60689 (US); LARSEN, John, J., Melrose Park, IL 60164 (US); LIU, Gang, Gurnee, IL 60031 (US); O'CONNOR, Stephen, J., Wilmette, IL 60091 (US); ROSENBERG, Saul, H., Grayslake, IL 60030 (US); SHEN, Wang, Gurnee, IL 60031 (US); SWENSON, Rolf, E., Trenton, NJ 08648-4767 (US); SORENSEN, Bryan, K., Waukegan, IL 60087 (US); SULLIVAN, Gerard M., Round lake Beach, Illinois 60073 (US); SZCZEPANKIEWICZ, Bruce G., Lindenhurst, Illinois 60046-4970 (US); TASKER, Andrew S., Gurnee, Illinois 60031-4017 (US); WASICK, James I., Waterford, Wisconsin 53185 (US)
(74) Representative: Lipscombe, Martin John
(86) International application number: PCT/US1998/009296
(87) International publication number: WO 1998/050029

(56) References cited:
- WO-A-97/17070
- WO-A-98/07692
- DATABASE HCAPLUS ON STN, 1997:247953, "Preparation of 2-Aminomethyl-4-Mercaptopyrrolidines and Analogs as Farnesyl Transferase Inhibitors", XP002913352; & WO,A,97 06138 (BOYLE F.T. et al.) 20-02-97,
- DATABASE HCAPLUS ON STN, 1996:567259, "Peptidomimetic Inhibitors of Prenyl Transferase, Preparation and Activity of the Peptidomimetics and Use for Treating Tumors", XP002913353; & WO,A,96 21456 (SEBTI et al.) 18-07-96.

## Description

### Technical Field

The present invention relates to novel compounds which are useful in inhibiting protein isoprenyl transferases (for example, protein farnesyltransferase and protein geranylgeranyltransferase) and the farnesylation or geranylgeranylation of the oncogene protein Ras and other related small g-proteins, compositions containing such compounds and methods of using such compounds.

### Background of the Invention

Ras oncogenes are the most frequently identified activated oncogenes in human tumors. Transformed protein Ras is involved in the proliferation of cancer cells. The Ras must be famesylated before this proliferation can occur. Farnesylation of Ras by farnesyl pyrophosphate (FPP) is effected by protein famesyltransferase. Inhibition of protein farnesyltransferase, and whereby farnesylation of the Ras protein, blocks the ability of transformed cells to proliferate. Inhibition of protein geranylgeranyltransferase and, thereby, of geranylgeranylation of Ras proteins, also results in down regulation of Ras protein function.

Activation of Ras and other related small g-proteins that are farnesylated and/or geranylated also partially mediates smooth muscle cell proliferation (Circulation, I-3: 88 (1993), which is hereby incorporated herein by reference). Inhibition of protein isoprenyl transferases, and thereby farnesylation or geranylgeranylation of the Ras protein, also aids in the prevention of intimal hyperplasia associated with restenosis and atherosclerosis, a condition which compromises the success of angioplasty and surgical bypass for obstructive vascular lesions.

There is therefore a need for compounds which are inhibitors of protein farnesyltransferase and protein geranylgeranyltransferase.

WO 97/179070 discloses certain inhibitors of protein isoprenyl transferases, which are said to be potentially useful in the treatment of cancer, but none of the compounds disclosed therein have the particular structure recited in the claims of the present application.

WO 97/06138 discloses certain 4-mercaptopyrrolidone derivatives as being farnesyl transferase inhibitors, and again these are said to be potentially useful in the treatment of cancer, by inhibiting the farnesylation of ras oncogene products. The compounds disclosed in WO 97/06138 however have different structures to those recited in the claims of the present application.

### Summary of the Invention

In its principle embodiment the invention provides a compound having the formula I recited in claim 1 of the claims appended hereto.

In yet another aspect of the present invention is disclosed a compound for use in inhibiting or treating cancer in a human or lower mammal, alone or in combination with another chemotherapeutic agent.

In yet another aspect of the present invention is disclosed a compound for use in treating or preventing intimal hyperplasia associated with restenosis and atherosclerosis in a mammal.

The compounds of the invention can comprise asymmetrically substituted carbon atoms. As a result, all stereoisomers of the compounds of the invention are meant to be included in the invention, including racemic mixtures, mixtures of diastereomers, as well as single diastereomers of the compounds of the invention. The terms "S" and "R" configuration, as used herein, are as defined by the IUPAC 1974 Recommendations for Section E, Fundamental Stereochemistry, Pure Appl. Chem. (1976) 45, 13-30, which is hereby incorporated herein by reference.

### Detailed Description

### Definitions of Terms

As used herein the terms "Cys," "Glu," "Leu," "Lys,""Met," "nor-Leu," "nor-Val," "Phe," "Ser" and "Val" refer to cysteine, glutamine, leucine, lysine, methionine, norleucine, norvaline, phenylalanine, serine and valine in their L-, D- or DL forms. As used herein these amino acids are in their naturally occuring L- form.

As used herein, the term "carboxy protecting group" refers to a carboxylic acid protecting ester group employed to block or protect the carboxylic acid functionality while the reactions involving other functional sites of the compound are carried out. Carboxy protecting groups are disclosed in Greene, "Protective Groups in Organic Synthesis" pp. 152-186 (1981), which is hereby incorporated herein by reference. In addition, a carboxy protecting group can be used as a prodrug whereby the carboxy protecting group can be readily cleaved *in vivo* (for example by enzymatic hydrolysis) to release the biologically active parent. T. Higuchi arid V. Stella provide a thorough discussion of the prodrug concept in "Pro-drugs as Novel Delivery Systems", Vol 14 of the A.C.S. Symposium Series, American Chemical Society (1975), which is hereby incorporated herein by reference. Such carboxy protecting groups are well known to those skilled in the art, having been extensively used in the protection of carboxyl groups in the penicillin and cephalosporin fields (as described in U.S. Pat. No. 3,840,556 and 3,719,667, the disclosures of which are hereby incorporated herein by reference). Examples of esters useful as prodrugs for compounds containing carboxyl groups can be found on pages 14-21 of "Bioreversible Carriers in Drug Design: Theory and Application", edited by E.B. Roche, Pergamon Press, New York (1987). Representative carboxy protecting groups are C₁ to C₈ loweralkyl (e.g., methyl, ethyl or tertiary butyl and the like); arylalkyl, for example, phenethyl or benzyl and substituted derivatives thereof such as alkoxybenzyl or nitrobenzyl groups and the like; arylalkenyl, for example, phenylethenyl and the like; aryl and substituted derivatives thereof, for example, 5-indanyl and the like; dialkylaminoalkyl (e.g., dimethylaminoethyl and the like); alkanoyloxyalkyl groups such as acetoxymethyl, butyryloxymethyl, valeryloxymethyl, isobutyryloxymethyl, isovaleryloxymethyl, 1-(propionyloxy)-1-ethyl, 1-(pivaloyloxyl)-1-ethyl, 1-methyl-1-(propionyloxy)-1-ethyl, pivaloyloxymethyl, propionyloxymethyl and the like; cycloalkanoyloxyalkyl groups such as cyclopropylcarbonyloxymethyl, cyclobutylcarbonyloxymethyl, cyclopentylcarbonyloxymethyl, cyclohexylcarbonyloxymethyl and the like; aroyloxyalkyl, such as benzoyloxymethyl, benzoyloxyethyl and the like; arylalkylcarbonyloxyalkyl, such as benzylcarbonyloxymethyl, 2-benzylcarbonyloxyethyl and the like; alkoxycarbonylalkyl or cycloalkyloxycarbonylalkyl, such as methoxycarbonylmethyl, cyclohexyloxycarbonylmethyl, 1-methoxycarbonyl-1-ethyl, and the like; alkoxycarbonyloxyalkyl or cycloalkyloxycarbonyloxyalkyl, such as methoxycarbonyloxymethyl, t-butyloxycarbonyloxymethyl, 1-ethoxycarbonyloxy-1-ethyl, 1-cyclohexyloxycarbonyloxy-1-ethyl and the like; aryloxycarbonyloxyalkyl, such as 2-(phenoxycarbonyloxy)ethyl, 2-(5-indanyloxycarbonyloxy)ethyl and the like; alkoxyalkylcarbonyloxyalkyl, such as 2-(1-methoxy-2-methylpropan-2-oyloxy)ethyl and like; arylalkyloxycarbonyloxyalkyl, such as 2-(benzyloxycarbonyloxy)ethyl and the like; arylalkenyloxycarbonyloxyalkyl, such as 2-(3-phenylpropen-2-yloxycarbonyloxy)ethyl and the like; alkoxycarbonylaminoalkyl, such as t-butyloxycarbonylaminomethyl and the like; alkylaminocarbonylaminoalkyl, such as methylaminocarbonylaminomethyl and the like; alkanoylaminoalkyl, such as acetylaminomethyl and the like; heterocycliccarbonyloxyalkyl, such as 4-methylpiperazinylcarbonyloxymethyl and the like; dialkylaminocarbonylalkyl, such as dimethylaminocarbonylmethyl, diethylaminocarbonylmethyl and the like; (5-(loweralkyl)-2-oxo-1,3-dioxolen-4-yl)alkyl, such as (5-t-butyl-2-oxo-1,3-dioxolen-4-yl)methyl and the like; and (5-phenyl-2-oxo-1,3-dioxolen-4-yl)alkyl, such as (5-phenyl-2-oxo-1,3-dioxolen-4-yl)methyl and the like.

Preferred carboxy-protected compounds of the invention are compounds wherein the protected carboxy group is a loweralkyl, cycloalkyl or arylalkyl ester, for example, methyl ester, ethyl ester, propyl ester, isopropyl ester, butyl ester, sec-butyl ester, isobutyl ester, amyl ester, isoamyl ester, octyl ester, cyclohexyl ester, phenylethyl ester and the like or an alkanoyloxyalkyl, cycloalkanoyloxyalkyl, aroyloxyalkyl or an arylalkylcarbonyloxyalkyl ester.

The term "N-protecting group" or "N-protected" as used herein refers to those groups intended to protect the N-terminus of an amino acid or peptide or to protect an amino group against undersirable reactions during synthetic procedures. Commonly used N-protecting groups are disclosed in Greene, "Protective Groups In Organic Synthesis," (John Wiley & Sons, New York (1981). N-protecting groups comprise acyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, a-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, a,a-dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl and the like; alkyl groups such as benzyl, triphenylmethyl, benzyloxymethyl and the like; and silyl groups such as trimethylsilyl and the like. Preferred N-protecting groups are formyl, acetyl, benzoyl, pivaloyl, t-butylacetyl, phenylsulfonyl, benzyl, t-butyloxycarbonyl (Boc) and benzyloxycarbonyl (Cbz).

The term "alkanoyl" as used herein refers to R₂₉C(O)- wherein R₂₉ is a loweralkyl group. The alkanoyl groups of this invention can be optionally substituted.

The term "alkanoylaminoalkyl" as used herein refers to a loweralkyl radical to which is appended R₇₁-NH- wherein R₇₁ is an alkanoyl group.

The term "alkanoyloxy" as used herein refers to R₂₉C(O)-O- wherein R₂₉ is a loweralkyl group. The alkanoyloxy groups of this invention can be optionally substituted.

The term "alkanoyloxyalkyl" as used herein refers to a loweralkyl radical to which is appended an alkanoyloxy group. The alkanoyloxyalkyl groups of this invention can be optionally substituted.

The term "alkenyl" as used herein refers to a straight or branched chain hydrocarbon containing from 2 to 10 carbon atoms and also containing at least one carbon-carbon double bond. Examples of alkenyl include -CH=CH₂, -CH₂CH=CH₂₉ -C(CH₃)=CH₂, -CH₂CH=CHCH₃, and the like.

The term "alkenylene" as used herein refers to a divalent group derived from a straight or branched chain hydrocarbon containing from 2 to 20 carbon atoms and also containing at least one carbon-carbon double bond. Examples of alkenylene include -CH=CH-, -CH₂CH=CH-, -C(CH₃)=CH-, -CH₂CH=CHCH₂-, and the like.

The term "alkenyloxy" as used herein refers to an alkenyl group attached to the parent molecular group through an oxygen atom.

The term "alkenyloxyalkyl" as used herein refers to a loweralkyl group to which is attached an alkenyloxy group.

The term "alkoxy" as used herein refers to R₃₀O- wherein R₃₀ is loweralkyl as defined above. Representative examples of alkoxy groups include methoxy, ethoxy, t-butoxy and the like. The alkoxy groups of this invention can be optionally substituted.

The term "alkoxyalkyl" as used herein refers to a loweralkyl group to which is attached an alkoxy group. The alkoxyalkyl groups of this invention can be optionally substituted.

The term "alkoxyalkoxy" as used herein refers to R₃₁O-R₃₂O- wherein R₃₁ is loweralkyl as defined above and R₃₂ is an alkylene radical. Representative examples of alkoxyalkoxy groups include methoxymethoxy, ethoxymethoxy, t-butoxymethoxy and the like.

The term "alkoxyalkyl" as used herein refers to an alkoxy group as previously defined appended to an alkyl group as previously defined. Examples of alkoxyalkyl include, but are not limited to, methoxymethyl, methoxyethyl, isopropoxymethyl and the like. The alkoxyalkyl groups of this invention can be optionally substituted.

The term "alkoxyalkylcarbonyloxyalkyl" as used herein refers to a loweralkyl radical to which is appended R₆₅-C(O)-O- wherein R₆₆ is an alkoxyalkyl group.

The term "alkoxyarylalkyl" as used herein refers to a an arylalkyl group to which is attached an alkoxy group.

The term "alkoxycarbonyl" as used herein refers to an alkoxy group as previously defined appended to the parent molecular moiety through a carbonyl group. Examples of alkoxycarbonyl include methoxycarbonyl, ethoxycarbonyl, isopropoxycarbonyl and the like. The alkoxycarbonyl groups of this invention can be optionally substituted.

The term "alkoxycarbonylalkyl" as used herein refers to an alkoxylcarbonyl group as previously defined appended to a loweralkyl radical. Examples of alkoxycarbonylalkyl include methoxycarbonylmethyl, 2-ethoxycarbonylethyl and the like.

The term "alkoxycarbonylaminoalkyl" as used herein refers to a loweralkyl radical to which is appended R₆₉-NH- wherein R₆₉ is an alkoxycarbonyl group.

The term "alkoxycarbonyloxyalkyl" as used herein refers to a loweralkyl radical to which is appended R₆₃-O- wherein R₆₃ is an alkoxycarbonyl group.

The term "alkylamino" as used herein refers to R₃₅NH- wherein R₃₅ is a loweralkyl group, for example, methylamino, ethylamino, butylamino, and the like. The alkylamino groups of this invention can be optionally substituted.

The term "alkylaminoalkyl" as used herein refers a loweralkyl radical to which is appended an alkylamino group.

The term "alkylaminocarbonylaminoalkyl" as used herein refers to a loweralkyl radical to which is appended R₇₀-C(O)-NH- wherein R₇₀ is an alkylamino group. The alkylaminocarbonylaminoalkyl groups of this invention can be optionally substituted.

The term "alkylene" as used herein refers to a divalent group derived from a straight or branched chain saturated hydrocarbon having from 1 to 10 carbon atoms by the removal of two hydrogen atoms, for example methylene, 1,2-ethylene, 1,1-ethylene, 1,3-propylene, 2,2-dimethylpropylene, and the like. The alkylene groups of this invention can be optionally substituted.

The term "alkylsilyloxy" as used herein refers to a loweralkyl group to which is attached -OSiR_{W'}R_{X'}R_{Y'} wherein R_{W'}, R_{X'}, and R_{Y'} are selected from the group consisting of loweralkyl.

The term "alkylsulfinyl" as used herein refers to R₃₃S(O)- wherein R₃₃ is a loweralkyl group.

The term "alkylsulfinylalkyl" as used herein refers to an alkyl group to which is attached a alkylsulfinyl group.

The term "alkylsulfonyl" as used herein refers to R₃₄S(O)₂- wherein R₃₄ is a loweralkyl group.

The term "alkylsulfonylalkyl" as used herein refers to a loweralkyl radical to which is appended an alkylsulfonyl group.

The term alkylthioalkyl as used herein refers to a lower alkyl group as defined herein attached to the parent molecular moiety through a sulfur atom and an alkylene group.

The term "alkynyl" as used herein refers to a straight or branched chain hydrocarbon containing from 2 to 10 carbon atoms and also containing at least one carbon-carbon triple bond. Examples of alkynyl include -C≡CH, -CH₂C≡CH, -CH₂C≡CCH₃, and the like.

The term "alkynylene" as used herein refers to a divalent group derived from a straight or branched chain hydrocarbon containing from 2 to 10 carbon atoms and also containing at least one carbon-carbon triple bond. Examples of alkynylene include -C≡C-, -CH₂C≡C-, -CH₂C≡CCH₂-, and the like.

The term "amino" as used herein refers to -NH₂.

The term "aminocarbonyl" as used herein refers to an amino group attached to the parent molecular group through a carbonyl group.

The term "aminocarbonylalkyl" as used herein refers to an alkyl group to which is attached an aminocarbonyl group.

The term "aminoalkyl" as used herein refers to a loweralkyl radical to which is appended an amino group.

The term "aminothiocarbonyl" as used herein refers to an amino group attached to the parent molecular group through a thiocarbonylcarbonyl (C=S) group.

The term "aroyloxyalkyl" as used herein refers to a loweralkyl radical to which is appended an aroyloxy group (i.e., R₆₁-C(O)O- wherein R₆₁ is an aryl group).

The term "aryl" as used herein refers to a mono- or bicyclic carbocyclic ring system having one or two aromatic rings including, but not limited to, phenyl, naphthyl, tetrahydronaphthyl, indanyl, indenyl and the like. Aryl groups (including bicyclic aryl groups) can be unsubstituted or substituted with one, two or three substituents independently selected from loweralkyl, haloalkyl, alkoxy, thioalkoxy, amino, alkylamino, dialkylamino, hydroxy, halo, mercapto, sulfhydryl, nitro, cyano, carboxaldehyde, carboxy, alkoxycarbonyl, haloalkyl-C(O)-NH-, haloalkenyl-C(O)-NH- and carboxamide. In addition, substituted aryl groups include tetrafluorophenyl and pentafluorophenyl.

The term "arylalkenyl" as used herein refers to an alkenyl radical to which is appended an aryl group.

The term "arylalkenyloxycarbonyloxyalkyl" as used herein refers to a loweralkyl radical to which is appended R₆₈-O-C(O)-O- wherein R₆₈ is an arylalkenyl group.

The term "arylalkoxy" as used herein refers to an alkoxy group to which is attached an aryl group.

The term "arylalkyl" as used herein refers to a loweralkyl radical to which is appended an aryl group. Representative arylalkyl groups include benzyl, phenylethyl, hydroxybenzyl, fluorobenzyl, fluorophenylethyl and the like.

The term "arylalkylcarbonyloxyalkyl" as used herein refers to a loweralkyl radical to which is appended an arylalkylcarbonyloxy group (i.e., R₆₂C(O)O- wherein R₆₂ is an arylalkyl group).

The term "aryloxy" as used herein refers to an aryl group attached to the parent molecular group through an oxygen atom.

The term "aryloxycarbonyl" as used herein refers to an aryloxy group attached to the parent molecular group through a carbonyl group.

The term "aryloyl" as used herein refers to an aryl group attached to the parent molecular group through a carbonyl group.

The term "arylalkyloxycarbonyloxyalkyl" as used herein refers to a loweralkyl radical to which is appended R₆₇-O-C(O)-O- wherein R₆₇ is an arylalkyl group.

The term "aryloxyalkyl" as used herein refers to a loweralkyl radical to which is appended R₆₅-O- wherein R₆₅ is an aryl group.

The term "arylalkoxy" as used herein refers to an alkoxy radical to which is appended R₆₅-O- wherein R₆₅ is an aryl group.

The term "arylalkyloxyalkyl" as used herein refers to a loweralkyl radical to which is appended an arylalkoxy group.

The term "aryloxy" as used herein refers to R₆₅-O- wherein R₆₅ is an aryl group. The aryloxy groups of this invention can be optionally substituted.

The term "(aryl)oyl" as used herein refers to an aryl group attached to the parent molecular group through a carbonyl group.

The term "aryloxythioalkoxyalkyl" as used herein refers to a loweralkyl radical to which is appended R₇₅-S- wherein R₇₅ is an aryloxyalkyl group.

The term "aryloxycarbonyloxyalkyl" as used herein refers to a loweralkyl radical to which is appended R₆₅-O-C(O)-O- wherein R₆₅ is an aryl group.

The term "arylsulfonyl" as used herein refers to R₃₆S(O)₂- wherein R₃₆ is an aryl group.

The term "arylsulfonyloxy" as used herein refers to R₃₇S(O)₂O- wherein R₃₇ is an aryl group.

The term "carboxy" as used herein refers to -COOH.

The term "carboxyalkyl" as used herein refers to a loweralkyl radical to which is appended a carboxy (-COOH) group.

The term "cyanoalkyl" as used herein used herein refers to a loweralkyl radical to which is appended a cyano (-CN) group.

The term "carboxaldehyde" as used herein used herein refers to -CHO.

The term "(carboxaldehyde)alkyl" as used herein used herein refers to a carboxaldehyde group attached to a loweralkyl group.

The terms "cycloalkanoyl" and "(cycloalkyl)oyl" refer to a cycloalkyl group attached to the parent molecular group through a carbonyl group. The (cycloalkyl)oyl groups of this invention can be optionally substituted.

The term "cycloalkanoylalkyl" as used herein refers to a loweralkyl radical to which is appended a cycloalkanoyl group (i.e., R₆₀-C(O)- wherein R₆₀ is a cycloalkyl group).

The term "cycloalkylalkoxyalkyl" as used herein refers to an alkoxyalkyl group to which is attached a cycloalkyl group.

The term "cycloalkenyl" as used herein refers to an alicyclic group comprising from 3 to 10 carbon atoms and containing a carbon-carbon double bond including, but not limited to, cyclopentenyl, cyclohexenyl and the like.

The term "cycloalkoxy" as used herein refers to a cycloalkyl group attached to the parent molecular group through an oxygen atom.

The term "cycloalkoxyalkyl" as used herein refers to a loweralkyl group to which is attached a cycloalkoxy group.

The term "cycloalkoxycarbonyl" as used herein refers to a cycloalkoxy group attached to the parent molecular group through a carbonyl group. The cycloalkoxycarbonyl groups of this invention can be optionally substituted.

The term "cycloalkyl" as used herein refers to an alicyclic group comprising from 3 to 10 carbon atoms including, but not limited to, cyclopropyl, cyclobutyl, cyclopentyl, cyclohexyl, norbornyl, adamantyl and the like. The cycloalkyl groups of this invention can be optionally substituted.

The term "cycloalkylaminocarbonyl" as used herein refers to NHR₆₀-C(O)- wherein R_{60'} is a cycloalkyl group. The cycloalkylaminocarbonyl groups of this invention can be optionally substituted.

The term "cycloalkylaminothiocarbonyl" as used herein refers to NHR_{60'}C(S)-wherein R_{60'} is defined above. The cycloalkylaminothiocarbonyl groups of this invention can be optionally substituted.

The term "cycloalkylalkoxy" as used herein refers to an alkoxy radical to which is appended a cycloalkyl group.

The term "cycloalkylalkoxyalkyl" as used herein refers to an alkyl radical to which is appended a cycloalkylalkoxy group.

The term "cycloalkylalkoxycarbonyl" as used herein refers to a cycloalkylalkoxy radical attached to the parent molecular group through a carbonyl group.

The term "cycloalkylalkyl" as used herein refers to a loweralkyl radical to which is appended a cycloalkyl group. Representative examples of cycloalkylalkyl include cyclopropylmethyl, cyclohexylmethyl, 2-(cyclopropyl)ethyl, adamantylmethyl and the like. The cycloalkylalkyl groups of this invention can be optionally substituted.

The term "cycloalkyloxycarbonyloxyalkyl" as used herein refers to a loweralkyl radical to which is appended R₆₄-O-C(O)-O- wherein R₆₄ is a cycloalkyl group.

The term "dialkoxyalkyl" as used herein refers to a loweralkyl radical to which is appended two alkoxy groups.

The term "dialkylamino" as used herein refers to R₃₈R₃₉N- wherein R₃₈ and R₃₉ are independently selected from loweralkyl, for example dimethylamino, diethylamino, methyl propylamino, and the like.

The term "dialkylaminoalkyl" as used herein refers to a loweralkyl radical to which is appended a dialkylamino group.

The term "dialkyaminocarbonylalkyl" as used herein refers to a loweralkyl radical to which is appended R₇₃-C(O)- wherein R₇₃ is a dialkylamino group.

The term "dioxoalkyl" as used herein refers to a loweralkyl radical which is substituted with two oxo (=O) groups.

The term "dithioalkoxyalkyl" as used herein refers to a loweralkyl radical to which is appended two thioalkoxy groups.

The term "halogen" or "halo" as used herein refers to I, Br, Cl or F.

The term "haloalkenyl" as used herein refers to an alkenyl radical, as defined above, bearing at least one halogen substituent.

The term "haloalkyl" as used herein refers to a lower alkyl radical, as defined above, bearing at least one halogen substituent, for example, chloromethyl, fluoroethyl or trifluoromethyl and the like. Haloalkyl can also include perfluoroalkyl wherein all hydrogens of a loweralkyl group are replaced with fluorides.

The term "heterocyclic ring" or "heterocyclic" or "heterocycle" as used herein refers to a 5-, 6- or 7-membered ring containing one, two or three heteroatoms independently selected from the group consisting of nitrogen, oxygen and sulfur or a 5-membered ring containing 4 nitrogen atoms; and includes a 5-, 6- or 7-membered ring containing one, two or three nitrogen atoms; one oxygen atom; one sulfur atom; one nitrogen and one sulfur atom; one nitrogen and one oxygen atom; two oxygen atoms in non-adjacent positions; one oxygen and one sulfur atom in non-adjacent positions; two sulfur atoms in non-adjacent positions; two sulfur atoms in adjacent positions and one nitrogen atom; two adjacent nitrogen atoms and one sulfur atom; two non-adjacent nitrogen atoms and one sulfur atom; two non-adjacent nitrogen atoms and one oxygen atom. The 5-membered ring has 0-2 double bonds and the 6- and 7-membered rings have 0-3 double bonds. The term "heterocyclic" also includes bicyclic, tricyclic and tetracyclic groups in which any of the above heterocyclic rings is fused to one or two rings independently selected from the group consisting of an aryl ring, a cyclohexane ring, a cyclohexene ring, a cyclopentane ring, a cyclopentene ring and another monocyclic heterocyclic ring (for example, indolyl, quinolyl, isoquinolyl, tetrahydroquinolyl, benzofuryl or benzothienyl and the like). Heterocyclics include: pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidinyl, morpholinyl, thiomorpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, thiazolidinyl, isothiazolyl, triazolyl, tetrazolyl, oxadiazolyl, thiadiazolyl, pyrimidyl, tetrahydrofuranyl, dihydrofuranyl, tetrahydrothienyl, dihydrothienyl, dihydroindolyl, tetrahydroquinolyl, tetrahydroisoquinolyl, pyranyl, dihydropyranyl, dithiazolyl, benzofuranyl and benzothienyl. Heterocyclics also include bridged bicyclic groups wherein a monocyclic heterocyclic group is bridged by an alkylene group, for example, 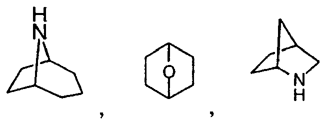 and the like.

Heterocyclics also include compounds of the formula 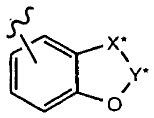 wherein X* is -CH₂-, -CH₂O- or -O- and Y* is -C(O)- or -(C(R")₂)ᵥ - wherein R" is hydrogen or C₁-C₄-alkyl and v is 1, 2 or 3 such as 1,3-benzodioxolyl, 1,4-benzodioxanyl and the like.

Heterocyclics can be unsubstituted or substituted with one, two, three, four or five substituents independently selected from the group consisting of a) hydroxy, b) -SH, c) halo, d) oxo (=O), e) thioxo (=S), f) amino,g) -NHOH, h) alkylamino, i) dialkylamino, j) alkoxy, k) alkoxyalkoxy, l) haloalkyl, m) hydroxyalkyl, n) alkoxyalkyl, o) cycloalkyl which is unsubstituted or substituted with one, two, three or four loweralkyl groups, p) cycloalkenyl which is unsubstituted or substituted with one, two, three or four loweralkyl groups, q) alkenyl, r) alkynyl, s) aryl, t) arylalkyl, u) -COOH, v) -SO₃H w) loweralkyl, x) alkoxycarbonyl, y) -C(O)NH₂, z) -C(S)NH₂, aa) -C(=N-OH)NH₂, bb) aryl-L₁₆-C(O)- wherein L₁₆ is an alkenylene radical, cc) -S-L₁₇-C(O)OR₄₀ wherein L₁₇ is an alkylene radical which is unsubstituted or substituted with one or two substitutents independently selected from the group consisting of alkanoyl, oxo (=O) or methinylamino (=CHNR₄₁R₄₂ wherein R₄₁ is hydrogen or loweralkyl and R₄₂ is loweralkyl) and R₄₀ is hydrogen or a carboxy-protecting group, dd) -S-L₁₈-C(O)NR₄₃R₄₄ wherein L₁₈ is an alkylene radical which is unsubstituted or substituted with one or two substitutents independently selected from the group consisting of alkanoyl, oxo (=O) or methinylamino (=CHNR₄₁R₄₂ wherein R₄₁ is hydrogen or loweralkyl and R₄₃ and R₄₄ are independently selected from the group consisting of hydrogen, loweralkyl and aryl, ee) -S-L₁₉-CN wherein L₁₉ is an alkylene radical, ff) -S-L₂₀-R₄₅ wherein L₂₀ is absent or is an alkylene radical or an alkenylene radical or an alkynylene radical wherein the alkylene, alkenylene or alkynylene radical is unsubstituted or substituted with oxo (=O) and R₄₅ is hydrogen, aryl, arylalkyl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, gg) -O-L₂₁-R₄₆ wherein L₂₁ is absent or is an alkylene radical or an alkenylene radical or an alkynylene radical wherein the alkylene, alkenylene or alkynylene radical is unsubstituted or substituted with one or two substitutents independently selected from the group consisting of alkanoyl, oxo (=O) or methinylamino (=CHNR₄₁R₄₂ wherein R₄₁ is hydrogen or loweralkyl and R₄₆ is hydrogen, aryl, arylalkyl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, hh) -O-S(O)₂-R₄₇ wherein R₄₇ is aryl, arylalkyl, heterocyclic or heterocyclicalkyl wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, ii) -S(O)₂-NH-R₄₈ wherein R₄₈ is aryl, arylalkyl, heterocyclic or heterocyclicalkyl wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, jj) alkylsulfinyl, kk) alkylsulfonyl, 11) arylsulfonyl, mm) arylsulfonyloxy, nn) -C(=NOR₄₉)C(O)OR₅₀ wherein R₄₉ is hydrogen or loweralkyl and R₅₀ is hydrogen or a carboxy-protecting group, oo) alkoxycarbonylalkyl, pp) carboxyalkyl, qq) cyanoalkyl, rr) alkylaminoalkyl, ss) N-protected alkylaminoalkyl, tt) dialkylaminoalkyl, uu) dioxoalkyl, vv) loweralkyl-C(O)-, ww) loweralkyl-C(S)-, xx) aryl-C(O)-, yy) aryl-C(S)-, zz) loweralkyl-C(O)-O-, aaa) loweralkyl-S-C(S)- bbb) N-protected amino, ccc) aminoalkyl-C(O)-, ddd) N-protected aminoalkyl-C(O)- eee) aminoalkyl-C(S)-, fff) N-protected aminoalkyl-C(S)-, ggg) aminoalkyl, hhh) N-protected aminoalkyl, iii) formyl, jjj) cyano, kkk) nitro, lll) spiroalkyl, mmm) oxoalkyloxy, nnn) R₅₃-L₂₂-, wherein L₂₂ is alkenylene or alkynylene and R₅₃ is aryl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, ooo) aryl-NH-C(O)-, ppp) R₅₄-N=N- wherein R₅₄ is aryl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, qqq) =N-R₅₅ wherein R₅₅ is hydrogen, aryl, heterocyclic, -S(O)₂-aryl or -S(O)₂-heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, rrr) diarylalkyl-N=N-, sss) aryl-N(R₅₆)- or arylalkyl-N(R₅₆)- wherein R₅₆ is hydrogen or an N-protecting group, ttt) arylsulfonylalkyl, uuu) heterocyclicsulfonylalkyl wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, vvv) =C(CN)(C(O)NH₂), www) =C(CN)(C(O)O-loweralkyl), xxx) heterocyclic or heterocyclicalkyl wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, yyy) hydroxythioalkoxy, zzz) aryloxyalkyl, aaaa) aryloxyalkylthioalkoxy, bbbb) dialkoxyalkyl, cccc) dithioalkoxyalkyl, dddd) arylalkyl-NH-L₂₃- wherein L₂₃ is an alkylene group, eeee) heterocyclicalkyl-NH-L₂₄- wherein L₂₄ is an alkylene group, ffff) aryl-S(O)₂-NH-L₂₅- wherein L₂₅ is an alkylene group, gggg) heterocyclic-S(O)₂-NH-L₂₆- wherein L₂₆ is an alkylene group, hhhh) aryl-C(O)-NH-L₂₇- wherein L₂₇ is an alkylene group and iiii) heterocyclic-C(O)-NH-L₂₈- wherein L₂₈ is an alkylene group, jjjj) R_{yy}(CH2)ₙ-X-Y-Z-(CH₂)ₘ wherein Ryy is cycloalkyl, aryl and loweralkyl, n amd m are independently 0-2, Z is O or absent, Y is absent, CH₂, CHOH or C(O), with the proviso that when X is O, Z is absent and with the proviso that when Z is O, X is absent and with the proviso that when Y is CHOH. X and Z are absent.

The term "(heterocyclic)alkoxy" as used herein refers to an alkoxy group to which is attached a heterocycle.

The term "(heterocyclic)alkyl" as used herein refers to a heterocyclic group as defined above appended to a loweralkyl radical as defined above. Examples of heterocyclic alkyl include 2-pyridylmethyl, 4-pyridylmethyl, 4-quinolinylmethyl and the like.

The term "(heterocyclic)oxy" as used herein refers to a heterocycle connected to the parent molecular group through an oxygen atom.

The term "(heterocyclic)oxyalkyl" as used herein refers to a loweralkyl group to which is attached a (heterocyclic)oxy group.

The term "(heterocyclic)alkoxyalkyl" as used herein refers to an alkoxyalkyl group to which is attached a heterocycle.

The term "heterocycliccarbonyloxyalkyl" as used herein refers to a loweralkyl radical to which is appended R₇₂-C(O)-O- wherein R₇₂ is a heterocyclic group.

The term "hydroxy" as used herein refers to -OH.

The term "hydroxyalkyl" as used herein refers to a loweralkyl radical to which is appended an hydroxy group.

The term "hydroxyarylalkyl" as used herein refers to a arylalkyl group to which is appended a hydroxy group.

The term "hydroxythioalkoxy" as used herein refers to R₅₁S- wherein R₅₁ is a hydroxyalkyl group.

The term "loweralkyl" as used herein refers to branched or straight chain alkyl groups comprising one to ten carbon atoms, including methyl, ethyl, propyl, isopropyl, n-butyl, t-butyl, neopentyl and the like. The loweralkyl groups of this invention can be optionally substituted.

The term "N-protected alkylaminoalkyl" as used herein refers to an alkylaminoalkyl group wherein the nitrogen is N-protected.

The term "nitro" as used herein refers to -NO₂.

The term "oxo" as used herein refers to (=O).

The term "oxoalkyloxy" as used herein refers to an alkoxy radical wherein the loweralkyl moiety is substituted with an oxo (=O) group.

The term "oxyamino(alkyl)carbonylalkyl" as used herein refers to a -O-NR-C(O)-R' group wherein R and R' are loweralkyl.

The term "oxyamino(arylalkyl)carbonylalkyl" as used herein refers to a -O-NR^{R}₃-C(O)-R group wherein R^{R}3 is arylalkyl and R is loweralkyl.

The term "oxyaminocarbonylalkyl" as used herein refers to -O-NH-C(O)-R group wherein R is loweralkyl.

The term "spiroalkyl" as used herein refers to an alkylene diradical, both ends of which are bonded to the same carbon atom of the parent group to form a spirocyclic group.

The term "sulfhydryl" as used herein refers to -SH.

The term "sulfhydrylalkyl" as used herein refers to a loweralkyl group to which is attached a sulfhydryl group.

The term "thioalkoxy as used herein refers to R₅₂S- wherein R₅₂ is loweralkyl. Examples of thioalkoxy include, but are not limited to, methylthio, ethylthio and the like.

The term "thioalkoxyalkyl" as used herein refers to a thioalkoxy group as previously defined appended to a loweralkyl group as previously defined. Examples of thioalkoxyalkyl include thiomethoxymethyl, 2-thiomethoxyethyl and the like. The thioalkoxyalkyl groups of this invention can be optionally substituted.

The term "thiocycloalkoxy" as used herein refers to a cycloalkyl group attached to the parent molecular group through a sulfur atom.

The term "thiocycloalkoxyalkyl" as used herein refers to a loweralkyl group to which is attached a thiocycloalkoxy group.

### Protein Farnesyltransferase Inhibition

The ability of the compounds of the invention to inhibit protein farnesyltransferase or protein geranylgeranyltransferase can be measured according to the method of Moores, et al., J. Biol. Chem. 266: 14603 (1991) or the method of Vogt, et al., J. Biol. Chem. 270:660-664 (1995). In addition, procedures for determination of the inhibition of farnesylation of the oncogene protein Ras are described by Goldstein, et al., J. Biol. Chem., 266:15575-15578 (1991) and by Singh in United States Patent No. 5,245,061.

In addition, *in vitro* inhibition of protein farnesyltransferase may be measured by the following procedure. Rat brain protein farnesyltransferase activity is measured using an Amersham Life Science commercial scintillation proximity assay kit and substituting a biotin-K Ras B fragment (biotin-Lys-Lys-Ser-Lys-Thr-Lys-Cys-Val-Ile-Met-CO₂H), 0.1 mM final concentration, for the biotin-lamin substrate provided by Amersham. The enzyme is purified according to Reiss, Y., et al., Cell, 62: 81-88 (1990), utilizing steps one through three. The specific activity of the enzyme is approximately 10 nmol substrate farnesylated/mg enzyme/hour. The percent inhibition of the farnesylation caused by the compounds of the invention (at 10 x 10⁻⁶ M) compared to an uninhibited control sample is evaluated in the same Amersham test system.

The % inhibition of protein farnesyltransferase was determined for representative compounds, some being compounds of the invention. The results are summarized in Table 1.

### Tables 1-5

*In Vitro* Potencies of Representative Compounds

**Table 1. Inhibition of farnesyltransferase**

| Example | % inhibition at 1X10⁻⁵ M | Example | % inhibition at 1X10⁻⁵ M |
|---|---|---|---|
| 200 | 93 | 674 | 40 |
| 350 | 53 | 676 | 76 |
| 351 | 82 | 678 | 73 |
| 352 | 52 | 680 | 58 |
| 353 | 62 | 683 | 57 |
| 354 | 47 | 684 | 48 |
| 355 | 43 | 685 | 55 |
| 356 | 58 | 686 | 48 |
| 357 | 56 | 687 | 78 |
| 358 | 45 | 688 | 71 |
| 359 | 36 | 689 | 73 |
| 360 | 88 | 690 | 61 |
| 361 | 97 | 692 | 74 |
| 362 | 83 | 699 | 74 |
| 363 | 96 | 700 | 68 |
| 364 | 69 | 701 | 64 |
| 365 | 97 | 702 | 79 |
| 366 | 83 | 704 | 67 |
| 367 | 81 | 705 | 72 |
| 368 | 71 | 706 | 53 |
| 369 | 87 | 707 | 66 |
| 370 | 86 | 708 | 76 |
| 371 | 66 | 709 | 55 |
| 372 | 69 | 710 | 45 |
| 373 | 76 | 711 | 46 |
| 374 | 61 | 712 | 69 |
| 375 | 68 | 713 | 40 |
| 376 | 80 | 714 | 56 |
| 377 | 71 | 715 | 67 |
| 378 | 54 | 717 | 75 |
| 380 | 45 | 718 | 40 |
| 381 | 79 | 750 | 44 |
| 382 | > 50 | 752 | 58 |
| 383 | > 50 | 753 | 55 |
| 387 | > 50 | 754 | 40 |
| 388 | > 50 | 755 | 44 |
| 390 | > 50 | 756 | 47 |
| 639 | 44 | 757 | 58 |
| 659 | 55 | 758 | 46 |
| 663 | 43 | 759 | 49 |
| 664 | 75 | 952 | > 50 |
| 669 | 52 | 955 | 50 |
| 670 | 78 | 974 | > 50 |
| 672 | 48 | | |

**Table 2. Inhibition of farnesyltransferase**

| Example | % inhibition at 1X10⁻⁶ M | Example | % inhibition at 1X10⁻⁶ M |
|---|---|---|---|
| 157 | 92 | 583 | 98 |
| 158 | 2 | 587 | 97 |
| 159 | 84 | 595 | 97 |
| 160 | 30 | 607 | 96 |
| 161 | 54 | 610 | 94 |
| 162 | 12 | 613 | 97 |
| 163 | 18 | 617 | 99 |
| 164 | 92 | 620 | 98 |
| 165 | 74 | 626 | 61 |
| 166 | 97 | 627 | 85 |
| 167 | 98 | 632 | 43 |
| 168 | 92 | 633 | 32 |
| 183 | 98 | 636 | 72 |
| 184 | 36 | 641 | 34 |
| 185 | 93 | 642 | 48 |
| 186 | 86 | 644 | 54 |
| 187 | 68 | 386 | > 50 |
| 188 | 40 | 399 | > 50 |
| 189 | 88 | 403 | 99 |
| 190 | 4 | 404 | 98 |
| 191 | 28 | 405 | 98 |
| 192 | 95 | 406 | 95 |
| 193 | 4 | 407 | 98 |
| 196 | 43 | 435 | 96 |
| 197 | 1 | 451 | 85 |
| 201 | 63 | 452 | 96 |
| 202 | 31 | 453 | 90 |
| 203 | 76 | 456 | 81 |
| 204 | 98 | 457 | 92 |
| 205 | 98 | 460 | 88 |
| 206 | 67 | 463 | 91 |
| 207 | 98 | 465 | 92 |
| 208 | 98 | 466 | 93 |
| 209 | 74 | 467 | 97 |
| 210 | 5 | 468 | 96 |
| 211 | 98 | 469 | 92 |
| 212 | 12 | 470 | 95 |
| 213 | 98 | 471 | 94 |
| 214 | 97 | 472 | 97 |
| 215 | 82 | 473 | 96 |
| 216 | 67 | 474 | 92 |
| 217 | 99 | 475 | 21 |
| 218 | 89 | 476 | 91 |
| 219 | 56 | 477 | 98 |
| 220 | 92 | 478 | 98 |
| 221 | 55 | 479 | 95 |
| 222 | 41 | 480 | 87 |
| 223 | 63 | 481 | 95 |
| 224 | 41 | 488 | 41 |
| 225 | 93 | 494 | 96 |
| 226 | 23 | 495 | 95 |
| 227 | 94 | 496 | 93 |
| 228 | 39 | 497 | 94 |
| 231 | 50 | 498 | 98 |
| 233 | 65 | 499 | 98 |
| 234 | 4 | 500 | 98 |
| 235 | 95 | 501 | 84 |
| 237 | 98 | 502 | 24 |
| 238 | 22 | 503 | 57 |
| 239 | 97 | 504 | 90 |
| 240 | 98 | 505 | 72 |
| 241 | 41 | 507 | 95 |
| 242 | 99 | 507 | 96 |
| 243 | 23 | 508 | 95 |
| 244 | 21 | 509 | 77 |
| 245 | 50 | 510 | 84 |
| 248 | 79 | 512 | 94 |
| 249 | 77 | 513 | 96 |
| 250 | 96 | 514 | 94 |
| 252 | 98 | 515 | 72 |
| 253 | 99 | 516 | 95 |
| 254 | 96 | 525 | 99 |
| 255 | 98 | 528 | 99 |
| 256 | 98 | 529 | 99 |
| 257 | 98 | 530 | 94 |
| 258 | 98 | 537 | 97 |
| 259 | 98 | 540 | 40 |
| 260 | 98 | 645 | 37 |
| 261 | 98 | 646 | 58 |
| 262 | 98 | 649 | 86 |
| 263 | 99 | 650 | 68 |
| 264 | 98 | 651 | 33 |
| 265 | 98 | 652 | 41 |
| 266 | 97 | 653 | 62 |
| 267 | 96 | 655 | 35 |
| 268 | 98 | 657 | 32 |
| 269 | 98 | 658 | 73 |
| 270 | 98 | 661 | 45 |
| 271 | 84 | 662 | 68 |
| 272 | 96 | 665 | 55 |
| 273 | 96 | 666 | 82 |
| 274 | 94 | 667 | 83 |
| 276 | 98 | 671 | 36 |
| 277 | 98 | 673 | 59 |
| 278 | 99 | 677 | 37 |
| 279 | 99 | 682 | 31 |
| 280 | 98 | 691 | 34 |
| 281 | 98 | 693 | 53 |
| 282 | 76 | 694 | 45 |
| 283 | 98 | 696 | 57 |
| 284 | 83 | 697 | 39 |
| 286 | 84 | 703 | 40 |
| 287 | 24 | 716 | 69 |
| 288 | 22 | 719 | 90 |
| 289 | 23 | 720 | 70 |
| 290 | 74 | 721 | 83 |
| 291 | 23 | 722 | 96 |
| 292 | 36 | 723 | 87 |
| 294 | 98 | 724 | 87 |
| 295 | 94 | 725 | 78 |
| 296 | 89 | 726 | 81 |
| 297 | 65 | 727 | 95 |
| 298 | 43 | 744 | 84 |
| 299 | 94 | 749 | 84 |
| 300 | 22 | 751 | 32 |
| 301 | 98 | 764 | 88 |
| 302 | 31 | 765 | 76 |
| 304 | 99 | 768 | 67 |
| 305 | 99 | 771 | 72 |
| 306 | 99 | 772 | 79 |
| 307 | 82 | 773 | 41 |
| 308 | 62 | 774 | 48 |
| 309 | 98 | 775 | 32 |
| 310 | 98 | 776 | 36 |
| 311 | 97 | 777 | 83 |
| 313 | 94 | 782 | 96 |
| 314 | 97 | 786 | 34 |
| 315 | 93 | 787 | 70 |
| 316 | 63 | 788 | 44 |
| 317 | 54 | 789 | 86 |
| 318 | 98 | 790 | 88 |
| 319 | 98 | 791 | 53 |
| 320 | 93 | 792 | 88 |
| 321 | 90 | 793 | 94 |
| 322 | 98 | 794 | 92 |
| 323 | 98 | 796 | 35 |
| 324 | 98 | 797 | 35 |
| 325 | 99 | 806 | 72 |
| 326 | 91 | 807 | 90 |
| 327 | 97 | 808 | 88 |
| 328 | 96 | 809 | 78 |
| 329 | 98 | 810 | 89 |
| 330 | 98 | 812 | 94 |
| 331 | 98 | 813 | 95 |
| 332 | 26 | 816 | 87 |
| 333 | 99 | 824 | 90 |
| 334 | 93 | 831 | 92 |
| 343 | 72 | 832 | 80 |
| 344 | 95 | 834 | 55 |
| 345 | 91 | 835 | 96 |
| 346 | 98 | 844 | 92 |
| 347 | 95 | 846 | 85 |
| 348 | 66 | 850 | 90 |
| 349 | 99 | 862 | 95 |
| 379 | 21 | 866 | 62 |
| 541 | 37 | 867 | 71 |
| 542 | 67 | 868 | 89 |
| 544 | 35 | 872 | 74 |
| 545 | 88 | 878 | 95 |
| 546 | 97 | 879 | 95 |
| 547 | 91 | 886 | 35 |
| 550 | 96 | 889 | 95 |
| | 78 | 902 | 85 |
| 728 | | | |
| 552 | 88 | 903 | 78 |
| 553 | 92 | 908 | 88 |
| 554 | 96 | 910 | 42 |
| 555 | 85 | 911 | 65 |
| 556 | 99 | 918 | 97 |
| 557 | 93 | 923 | 78 |
| 560 | 91 | 924 | 77 |
| 561 | 91 | 925 | 87 |
| 564 | 98 | 926 | 69 |
| 565 | 94 | 936 | |
| | | | 69 |
| 566 | 98 | 937 | 95 |
| 568 | 93 | 962 | > 50 |
| 569 | 91 | 964 | > 50 |
| 572 | 91 | 979 | 26 |
| 575 | 70 | 982 | 64 |
| 576 | 88 | 987 | 93 |
| 577 | 94 | 988 | 92 |
| 582 | 99 | 989 | 88 |

**Table 3. Inhibition of farnesyltransferase**

| Example | % inhibition at 1X10⁻⁷ M | Example | % inhibition at 1X10⁻⁷ M |
|---|---|---|---|
| 434 | 93 | 623 | 96 |
| 436 | 89 | 729 | 73 |
| 437 | 89 | 730 | 96 |
| 438 | 90 | 731 | 65 |
| 439 | 80 | 732 | 84 |
| 440 | 92 | 733 | 60 |
| 441 | 91 | 734 | 49 |
| 442 | 88 | 735 | 96 |
| 443 | 97 | 736 | 96 |
| 444 | 95 | 737 | 95 |
| 445 | 94 | 738 | 54 |
| 446 | 91 | 739 | 83 |
| 447 | 91 | 740 | 94 |
| 448 | 92 | 741 | 89 |
| 449 | 91 | 742 | 87 |
| 450 | 96 | 743 | 51 |
| 455 | 83 | 745 | 93 |
| 458 | 87 | 746 | 84 |
| 459 | 92 | 747 | 68 |
| 461 | 93 | 748 | 56 |
| 462 | 91 | 769 | 90 |
| 464 | 86 | 770 | 91 |
| 482 | 96 | 781 | 91 |
| 483 | 95 | 785 | 96 |
| 484 | 97 | 795 | 87 |
| 485 | 96 | 798 | 95 |
| 486 | 97 | 799 | 96 |
| 487 | 81 | 800 | 74 |
| 489 | 86 | 801 | 87 |
| 490 | 70 | 802 | 88 |
| 491 | 94 | 811 | 85 |
| 492 | 95 | 814 | 81 |
| 493 | 51 | 815 | 71 |
| 511 | 82 | 817 | 60 |
| 519 | 89 | 818 | 78 |
| 520 | 97 | 822 | 93 |
| 521 | 94 | 823 | 75 |
| 522 | 93 | 825 | 79 |
| 523 | 97 | 839 | 63 |
| 524 | 99 | 849 | 66 |
| 526 | 96 | 854 | 78 |
| 527 | 97 | 855 | 92 |
| 531 | 74 | 856 | 97 |
| 532 | 88 | 857 | 92 |
| 533 | 91 | 859 | 86 |
| 534 | 84 | 861 | 65 |
| 535 | 89 | 863 | 72 |
| 536 | 79 | 864 | 84 |
| 539 | 89 | 865 | 95 |
| 548 | 86 | 869 | 92 |
| 549 | 98 | 874 | 90 |
| 551 | 93 | 875 | 92 |
| 558 | 87 | 876 | 92 |
| 559 | 96 | 891 | 94 |
| 562 | 95 | 893 | 87 |
| 563 | 95 | 894 | 89 |
| 570 | 92 | 895 | 92 |
| 571 | 88 | 896 | 96 |
| 573 | 72 | 900 | 95 |
| 574 | 81 | 906 | 88 |
| 578 | 90 | 912 | 85 |
| 579 | 92 | 913 | 89 |
| 580 | 90 | 914 | 91 |
| 581 | 96 | 917 | 78 |
| 584 | 96 | 919 | 91 |
| 585 | 96 | 921 | 82 |
| 589 | 91 | 929 | 81 |
| 590 | 95 | 931 | 98 |
| 592 | 93 | 933 | 91 |
| 593 | 86 | 935 | 72 |
| 594 | 95 | 940 | 92 |
| 597 | 75 | 941 | 90 |
| 600 | 93 | 945 | 80 |
| 601 | 92 | 947 | 79 |
| 602 | 97 | 948 | 75 |
| 604 | 86 | 949 | 57 |
| 609 | 95 | 950 | 71 |
| 611 | 95 | 951 | 71 |
| 615 | 94 | 959 | > 50 |
| 616 | 95 | 983 | 66 |
| 618 | 89 | 984 | 86 |
| 621 | 98 | 990 | 84 |
| 622 | 95 | 993 | 90 |

**Table 4. Inhibition of farnesyltransferase**

| Example | % inhibition at 1X10⁻⁸ M | Example | % inhibition at 1X10⁻⁸ M |
|---|---|---|---|
| 384 | 91 | 851 | 82 |
| 397 | 50 | 852 | 79 |
| 398 | >50 | 853 | 85 |
| 400 | 98 | 858 | 60 |
| 401 | 66 | 860 | 85 |
| 408 | > 95 | 870 | 91 |
| 409 | 84 | 871 | 94 |
| 410 | 94 | 873 | 97 |
| 517 | 92 | 877 | 68 |
| 518 | 90 | 880 | 95 |
| 567 | 69 | 881 | 69 |
| 586 | 90 | 882 | 79 |
| 588 | 68 | 883 | 91 |
| 591 | 82 | 884 | 94 |
| 599 | 86 | 885 | 95 |
| 603 | 94 | 887 | 92 |
| 605 | 68 | 888 | 86 |
| 606 | 93 | 892 | 59 |
| 608 | 91 | 897 | 76 |
| 612 | 96 | 898 | 82 |
| 614 | 92 | 899 | 88 |
| 619 | 95 | 901 | 84 |
| 760 | 95 | 904 | 85 |
| 762 | 84 | 905 | 86 |
| 763 | 92 | 907 | 79 |
| 766 | 95 | 909 | 79 |
| 767 | 97 | 916 | 96 |
| 779 | 70 | 920 | 96 |
| 780 | 71 | 922 | 96 |
| 803 | 95 | 927 | 74 |
| 804 | 95 | 928 | 84 |
| 805 | 96 | 930 | 66 |
| 819 | 76 | 932 | 60 |
| 820 | 66 | 934 | 71 |
| 821 | 75 | 938 | 61 |
| 826 | 92 | 939 | 72 |
| 827 | 77 | 942 | 58 |
| 828 | 87 | 943 | 79 |
| 829 | 92 | 944 | 88 |
| 833 | 78 | 946 | 52 |
| 836 | 95 | 954 | > 50 |
| 837 | 91 | 958 | > 50 |
| 838 | 92 | 960 | > 50 |
| 840 | 73 | 985 | 89 |
| 841 | 93 | 986 | 95 |
| 842 | 88 | 991 | 69 |
| 843 | 96 | 992 | 93 |
| 845 | 85 | 994 | 83 |
| 847 | 85 | 995 | 92 |
| 848 | 87 | 996 | 80 |

**Table 5. Inhibition of geranylgeranyltransferase I.**

| Example | Activity |
|---|---|
| 387 | > 50% inhibition at 1 X 10⁻⁶ M |
| 388 | > 50% inhibition at 1 X 10⁻⁷ M |
| 389 | > 50% inhibition at 1 X 10⁻⁶ M |
| 390 | > 50% inhibition at 1 X 10⁻⁵ M |
| 392 | > 50% inhibition at 1 X 10⁻⁵ M |
| 399 | > 50% inhibition at 1 X 10⁻⁶ M |
| 953 | > 50% inhibition at 1 X 10⁻⁶ M |
| 955 | > 50% inhibition at 1 X 10⁻⁷ M |
| 962 | > 50% inhibition at 1 X 10⁻⁷ M |
| 964 | > 50% inhibition at 1 X 10⁻⁶ M |
| 966 | > 50% inhibition at 1 X 10⁻⁶ M |
| 967 | > 50% inhibition at 1 X 10⁻⁶ M |
| 969 | > 50% inhibition at 1 X 10⁻⁵ M |
| 974 | > 50% inhibition at 1 X 10⁻⁵ M |

**Table 6. Inhibition of farnesyltransferase at concentrations of 10 mM and 1 mM unless specified as * (0.1 mM) or ** (0.01 mM)**

| Example | % inhibition 10 mM | % inhibition 1 mM | Example | % inhibition 10 mM | % inhibition 1 mM |
|---|---|---|---|---|---|
| 997 | | 91** | 1199 | | 71 |
| 998 | | 79** | 1200 | | 97* |
| 999 | | 90 | 1201 | | 73* |
| 1000 | | 82* | 1202 | | 96** |
| 1001 | | 92** | 1203 | | 84* |
| 1002 | | 82** | 1204 | | 93* |
| 1003 | | 92* | 1205 | | 55** |
| 1004 | | 92** | 1206 | | 63** |
| 1005 | | 95** | 1207 | | 91* |
| 1006 | | 95** | 1208 | | 89* |
| 1007 | | 85** | 1209 | | 87* |
| 1008 | | 95** | 1210 | | 64** |
| 1009 | | 86** | 1211 | | 94 |
| 1010 | | 90* | 1212 | | 86* |
| 1011 | | 92** | 1213 | | 79** |
| 1012 | | 88* | 1214 | | 92** |
| 1013 | | 80* | 1215 | | 17 |
| 1014 | | 91 | 1216 | | 88** |
| 1015 | | 59* | 1217 | | 87* |
| 1016 | | 92* | 1218 | | 54** |
| 1017 | | 51* | 1219 | | 85** |
| 1018 | | 97 | 1220 | | |
| 1019 | | 70 | 1221 | | 82** |
| 1020 | | 39 | 1222 | | 89* |
| 1021 | | 93* | 1223 | | 91** |
| 1022 | | 91** | 1224 | | 88* |
| 1023 | | 89** | 1225 | | 92** |
| 1024 | | 89** | 1226 | | 69** |
| 1025 | | 91** | 1227 | | 91 |
| 1026 | | 74** | 1228 | | 88* |
| 1027 | | 81** | 1229 | | 66** |
| 1028 | | 92** | 1230 | | 77** |
| 1029 | | 82** | 1231 | | 93* |
| 1030 | | 92** | 1232 | | 68** |
| 1031 | | 90** | 1233 | | 77** |
| 1032 | | 93** | 1234 | | 71** |
| 1033 | | 76** | 1235 | | 86** |
| 1034 | | 77 | 1236 | | 83** |
| 1035 | | 76 | 1237 | | 89** |
| 1036 | | 79 | 1238 | | 91** |
| 1037 | | 88 | 1239 | | 85* |
| 1038 | | 57 | 1240 | | 64** |
| 1039 | | 89** | 1241 | | 74* |
| 1040 | | 90** | 1242 | | 75* |
| 1041 | | 48 | 1243 | | 95* |
| 1042 | | 88 | 1244 | | 84 |
| 1043 | | 90* | 1245 | | 92 |
| 1044 | | 76* | 1246 | | 82 |
| 1045 | | 86* | 1247 | | 95* |
| 1046 | | 93 | 1248 | | 88 |
| 1047 | | 95 | 1249 | | 89 |
| 1048 | | 78** | 1250 | | 79** |
| 1049 | | 93** | 1251 | | 91** |
| 1050 | | 62** | 1252 | | 84* |
| 1051 | | 79** | 1253 | | 76* |
| 1052 | | 91** | 1254 | | 67 |
| 1053 | | 60** | 1255 | | 82* |
| 1054 | | 89** | 1256 | | 95* |
| 1055 | | 85** | 1257 | | 93** |
| 1056 | | 75** | 1258 | | 97** |
| 1057 | | 82* | 1259 | | 89** |
| 1058 | | 89 | 1260 | | 90** |
| 1059 | | 92* | 1261 | | 94 |
| 1060 | | 42 | 1262 | | 95 |
| 1061 | | 88* | 1263 | | 85* |
| 1062 | | 93 | 1264 | | 83** |
| 1063 | | 92** | 1265 | | 90 |
| 1064 | | 95** | 1266 | | 85* |
| 1065 | | 78* | 1267 | | 96 |
| 1066 | | 73** | 1268 | | 95* |
| 1067 | | 93* | 1269 | | 84** |
| 1068 | | 79** | 1270 | | 91** |
| 1069 | | 74* | 1271 | | 78** |
| 1070 | | 93** | 1272 | | 73** |
| 1071 | | 95* | 1273 | | 94* |
| 1072 | | 82* | 1274 | | 89* |
| 1073 | | 93** | 1275 | | 86** |
| 1074 | | 82 | 1276 | | 88** |
| 1075 | | 90** | 1277 | | 90** |
| 1076 | | 69** | 1278 | | 68 |
| 1077 | | 93** | 1279 | | 87** |
| 1078 | | 86* | 1280 | | 78** |
| 1079 | | 90 | 1281 | | 81* |
| 1080 | | 87 | 1282 | | 69* |
| 1081 | | 61 | 1283 | | 74* |
| 1082 | | 84* | 1284 | | 86 |
| 1083 | | 88 | 1285 | | 94 |
| 1084 | | 76** | 1286 | | 85** |
| 1085 | | 93* | 1287 | | 95** |
| 1086 | | 87* | 1288 | | 69* |
| 1087 | | 76* | 1289 | | 93 |
| 1088 | | 73* | 1290 | | 80 |
| 1089 | | 86* | 1291 | | |
| 1090 | | 81** | 1292 | | |
| 1091 | | 87* | 1293 | | |
| 1092 | | 74** | 1294 | | |
| 1093 | | 95** | 1295 | | |
| 1094 | | 96** | 1296 | | |
| 1095 | | 76* | 1297 | | |
| 1096 | | 86* | 1298 | | 97** |
| 1097 | | 80** | 1299 | | 96** |
| 1098 | | 60* | 1300 | | 97* |
| 1099 | | 87** | 1301 | | 97* |
| 1100 | | 82** | 1302 | | 93** |
| 1101 | | 86* | 1303 | | 91** |
| 1102 | | 84** | 1304 | | 90** |
| 1103 | | 92* | 1305 | | 91** |
| 1104 | | 89** | 1306 | | 85** |
| 1105 | | 91** | 1307 | | 85** |
| 1106 | | 67** | 1308 | | 91** |
| 1107 | | 88** | 1309 | | 96* |
| 1108 | | 95** | 1310 | | 90** |
| 1109 | | 74** | 1311 | | 95** |
| 1110 | | | 1312 | | 91** |
| 1111 | | 63** | 1313 | | 91** |
| 1112 | | 62 | 1314 | | 96* |
| 1113 | | 55 | 1315 | | 86* |
| 1114 | | 83** | 1316 | | 78* |
| 1115 | | 94* | 1317 | 99 | 96 |
| 1116 | | 91** | 1318 | | |
| 1117 | | 92* | 1319 | | 79** |
| 1118 | | 86* | 1320 | | 79 |
| 1119 | | 84** | 1321 | | |
| 1120 | | 93 | 1322 | | |
| 1121 | | 72* | 1323 | | |
| 1122 | | 92** | 1324 | | |
| 1123 | | 90* | 1325 | | |
| 1124 | | 90* | 1326 | | |
| 1125 | | 92* | 1327 | | |
| 1126 | | 87 | 1328 | | |
| 1127 | | 90* | 1329 | | |
| 1128 | | 86* | 1330 | | |
| 1129 | | 92** | 1331 | | |
| 1130 | | 88** | 1332 | | 92** |
| 1131 | | 96** | 1333 | | 95* |
| 1132 | | 97* | 1334 | | 72** |
| 1133 | | 75* | 1335 | | 90* |
| 1134 | | 95** | 1336 | | 74 |
| 1135 | | 88* | 1337 | | 83** |
| 1136 | | 91 | 1338 | | 65* |
| 1137 | | 83** | 1339 | | |
| 1138 | | 65* | 1340 | | 77* |
| 1139 | | 92* | 1341 | | 89 |
| 1140 | | 77** | 1342 | | |
| 1141 | | 80* | 1343 | | 88 |
| 1142 | | 84** | 1344 | | 93** |
| 1143 | | 92* | 1345 | | 94** |
| 1144 | | 76* | 1346 | | 94* |
| 1145 | | 83* | 1347 | | 81** |
| 1146 | | 61** | 1348 | | 78** |
| 1147 | | 93* | 1349 | | 92** |
| 1148 | | 79** | 1350 | | |
| 1149 | | 94* | 1351 | | |
| 1150 | | 92* | 1352 | | |
| 1151 | | 91* | 1353 | | |
| 1152 | | 96* | 1354 | | 38 |
| 1153 | | 89* | 1355 | | 46 |
| 1154 | | 93* | 1356 | | 80 |
| 1155 | | 91* | 1357 | | 78 |
| 1156 | | 87 | 1358 | | |
| 1157 | | 66** | 1359 | | |
| 1158 | 75 | | 1360 | | 98** |
| 1159 | | 72* | 1361 | | 96* |
| 1160 | | 83* | 1362 | | 83** |
| 1161 | | 87* | 1363 | | 88** |
| 1162 | | 84* | 1364 | | |
| 1163 | | 73** | 1365 | | |
| 1164 | | 94 | 1366 | | 79* |
| 1165 | | 84* | 1367 | | 93* |
| 1166 | | 74** | 1368 | | 92** |
| 1167 | | 91* | 1369 | | 94* |
| 1168 | | 88* | 1370 | | 86** |
| 1169 | | 77 | 1371 | | 94* |
| 1170 | | 74* | 1372 | | 95** |
| 1171 | | 74** | 1373 | | 95** |
| 1172 | | 38* | 1374 | | 93** |
| 1173 | | 89** | 1375 | | 80** |
| 1174 | | 79** | 1376 | | 86** |
| 1175 | | 96 | 1377 | | 95* |
| 1176 | | 97* | 1378 | | 68 |
| 1177 | | 19 | 1379 | | 41 |
| 1178 | | 88** | 1380 | | 87** |
| 1179 | | 85* | 1381 | | 65** |
| 1180 | | 93* | 1382 | | 86** |
| 1181 | | 82* | 1383 | | 88* |
| 1182 | | 92** | 1384 | | 69** |
| 1183 | | 79** | 1385 | | 93* |
| 1184 | | 84** | 1386 | | 88* |
| 1185 | | 85** | 1387 | | 82** |
| 1186 | | 93** | 1392 | | 93* |
| 1187 | | 93** | 1397 | | 87** |
| 1188 | | 93** | 1398 | | 81* |
| 1189 | | 74** | 1399 | | 94 |
| 1190 | | 95** | 1400 | | 95 |
| 1191 | | 85** | | | |
| 1192 | | 91* | | | |
| 1193 | | 95** | | | |
| 1194 | | 78** | | | |
| 1195 | | 94* | | | |
| 1196 | | 87* | | | |
| 1197 | | 85* | | | |
| 1198 | | 86* | | | |

| | | | | | |
|---|---|---|---|---|---|
| * % inhibition at 0.1 µM ** % inhibition at 0.01 µM | | | | | |

Additional methods for the measurement of *in vitro* inhibition of protein prenylation (i.e., inhibition of farnesyltransferase or geranygeranyltransferase) are described below.

Assays are performed using the glass fiber filter binding assay procedure with either rabbit reticulocyte lysate or FTase or GGTase I fractions isolated from bovine brains using a combination of hydrophobic and DEAE column chromatography procedures. Protein substrates are purchased from Panvera Corporation (H-ras for FTase, H-ras-CVLL for GGTase I). Tritium labeled prenyl lipid substrates (FPP or GGPP) are obtained from Amersham Life Science.

### FTase

³H-Farnesyldiphosphate (final concentration 0.6 µM), H-Ras (final concentration 5.0 µM) and the test compound (various final concentrations from a stock solution in 50% DMSO/water; final concentration DMSO < 2%) were mixed in buffer (50 mM HEPES (pH 7.5), 30 mM MgCl₂, 20 mM KCI, 10 µM ZnCl₂, 5 mM DTT, 0.01 % Triton X-100) to give a final volume of 50 µL. The mixture was brought to 37 °C, enzyme was added, and the reaction is incubated for 30 minutes. 1 mL of 1 M HCl/ethanol was added to stop the reaction, and the mixture was allowed to stand for 15 minutes at room temperature then diluted with 2 mL of ethanol. The reaction mixture was filtered through a 2.5 cm glass microfiber filter from Whatman and washed with four 2 mL portions of ethanol. The glass filter was transferred to a scintillation vial and 5 mL of scintillation fluid was added. The radioisotope retained on the glass fiber filter was counted to reflect the activity of the enzymes. The IC₅₀ value was calculated by measuring the activity of the enzyme over a suitable range of inhibitor concentrations.

### GGTase I

³H-geranylgeranyldiphosphate (final concentration 0.5µM), H-Ras-CVLL (final concentration 5.0 µM) and the test compound (various final concentrations from a stock solution in 1:1 DMSO/water; final concentration DMSO < 2%) were mixed in buffer (50 mM Tris-HCl (pH 7.2), 30 mM MgCl₂, 20 mM KCI, 10 µM ZnCl₂, 5 mM DTT, 0.01% Triton X-100) to give a final volume of 50 µL. The mixture was brought to 37 °C, treated with enzyme, and incubated for 30 minutes. 1 mL of 1 M HCl/ethanol was added to stop the reaction, and the mixture was allowed to stand for 15 minutes at room temperature then diluted with 2 mL of ethanol. The reaction mixture was filtered through a 2.5 cm glass microfiber filter from Whatman and washed with four 2 mL portions of ethanol. The glass filter was transferred to a scintillation vial, and 5 mL scintillation fluid was added. The radioisotope retained on the glass fiber filter was counted to reflect the activity of the enzymes. The IC₅₀ value was calculated by measuring the activity of the enzyme over a suitable range of inhibitor concentrations.

Additionally, the ability of the compounds of the invention to inhibit prenylation in whole cells, inhibit anchorage-independent tumor cell growth and inhibit human tumor xenograft in mice could be demonstrated according to the methods described in PCT Patent Application No. WO95/25086, published September 21, 1995.

### Pharmaceutical Compositions

The compounds of the present invention can be used in the form of pharmaceutically acceptable salts derived from inorganic or organic acids. These salts include trifluoroacetate.

Basic addition salts can be prepared in situ during the final isolation and purification of the compounds of formula (I)-(XII) or separately by reacting the carboxylic acid function with a suitable base such as the hydroxide, carbonate or bicarbonate of a pharmaceutically acceptable metal cation Such pharmaceutically acceptable salts include cations based on the alkali metal, lithium.

The compounds of the invention are useful (in humans and other mammals) for inhibiting protein isoprenyltransferases (i.e, protein farnesyltransferase and/or protein geranylgeranyltransferase) and the isoprenylation (i.e., farnesylation and/or geranylgeranylation) of Ras. These inhibitors of protein isoprenyltransferases are also useful for inhibiting or treating cancer in humans and other mammals. Examples of cancers which may be treated with the compounds of the invention include, but are not limited to, carcinomas such as lung, colorectal, bladder, breast, kidney, ovarian, liver, exocrine pancreatic, cervical, esophageal, stomach and small intestinal; sarcomas such as oesteroma, osteosarcoma, lepoma, liposarcoma, hemanioma and hemangiosarcoma; melanomas such as amelanotic and melanotic; mixed types of cancers such as carcinosarcoma, lymphoid tissue type, follicular reticulum, cell sarcoma and Hodgkins disease and leukemias, such as myeloid, acute lymphoblastic, chronic lymphocytic, acute myloblastic and chronic mylocytic.

The ability of the compounds of the invention to inhibit or treat cancer can be demonstrated according to the methods of Mazerska Z., Woynarowska B., Stefanska B., Borowski S., Drugs Exptl. Clin. Res. 13(6), 345-351 (1987) Bissery, M.C., Guenard F., Guerritte-Voegelein F., Lavelle F., Cancer Res. 51, 4845-4852 (1991) and Rygaard J., and Povlsen C., Acta Pathol. Microbiol. Scand. 77, 758 (1969).

These inhibitors of protein isoprenyltransferases are also useful for treating or preventing restenosis in humans and other mammals. The ability of the compounds of the invention to treat or prevent restenosis can be demonstrated according to the methods described by Kranzhofer, R. et al. Circ. Res. 73: 264-268 (1993), Mitsuka, M. et al. Circ. Res. 73: 269-275 (1993) and Santoian, E.C. et al. Circulation 88: 11-14(1993).

For use as a chemotherapeutic agent, the total daily dose administered to a host in single or divided doses may be in amounts, for example, from 0.01 to 500 mg/kg body weight daily, preferably in amounts from 0.1 to 20 mg/kg body weight daily and more preferably in amounts from 0.5 to 10 mg/kg body weight daily. Dosage unit compositions may contain such amounts of submultiples thereof to make up the daily dose.

For treatment or prevention of restenosis, the total daily dose administered to a host in single or divided doses may be in amounts, for example, from 0.001 to 1000 mg/kg body weight daily and more preferred from 1.0 to 50 mg/kg body weight daily. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of the present invention may be administered orally, parenterally, sublingually, by inhalation spray, rectally or topically in dosage unit formulations containing conventional nontoxic pharmaceutically acceptable carriers, adjuvants, and vehicles. Topical administration may also involve the use of transdermal administration such as transdermal patches or iontophoresis devices. The term parenteral as used herein includes subcutaneous injections, intravenous, intramuscular, intrasternal injection or infusion techniques.

Injectable preparations, for example sterile injectable aqueous or oleagenous suspensions, may be formulated according to the known art using suitable dispersing or wetting and suspending agents. The sterile injectable preparation may also be a sterile injectable solution or suspension in a nontoxic parenterally acceptable diluent or solvent (as in a solution in 1,3-propanediol, for example). Among the acceptable vehicles and solvents that may be employed are water, Ringer's solution and isotonic sodium chloride solution. Additionally, sterile, fixed oils are conventionally employed as a solvent or suspending medium. For this purpose, any bland fixed oil may be employed including synthetic mono- or diglycerides. Fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at rectal temperature and will therefore melt in the rectum and release the drug.

Solid dosage forms for oral administration may include capsules, tablets, pills, powders and granules. In such solid dosage forms, the active compound may be admixed with at least one inert diluent such as sucrose, lactose or starch. These dosage forms may also comprise additional substances other than inert diluents such as lubricating agents like magnesium stearate. With capsules, tablets and pills, the dosage forms may also comprise buffering agents. Tablets and pills mayalso be prepared with enteric coatings.

Liquid dosage forms for oral administration may include pharmaceutically acceptable emulsions, solutions, suspensions, syrups and elixirs containing inert diluents commonly used in the art such as water. Such compositions may also comprise adjuvants such as wetting agents, emulsifying and suspending agents and sweetening, flavoring, and perfuming agents.

The compounds of the present invention can also be administered in the form of liposomes. As is known in the art, liposomes are generally derived from phospholipids or other lipid substances. Liposomes are formed by mono- or multi-lamellar hydrated liquid crystals dispersed in an aqueous medium. Any non-toxic, physiologically aceptable and metabolizable lipid capable of forming liposomes can be used. The present compositions in liposome form can contain, in addition to a compound of the present invention, stabilizers, preservatives, excipients and the like. The preferred lipids are the phospholipids and phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq*.*

While the compounds of the invention can be administered as the sole active pharmaceutical agent for the treatment of cancer, they can also be used in combination with one or more other chemotherapeutic agents.

Representative examples of chemotherapeutic agents are described in Holleb, et al., Clinical Oncology, American Cancer Society, United States (1991) p 56 et seq*.* These agents include alkylating agents such as the nitrogen mustards (mechloethamine, melphalan, chlorambucil, cyclophosphamide and ifosfamide), nitrosoureas (carmustine, lomustine, semustine, streptozocin), alkyl sulfonates (busulfan), triazines (dacarbazine) and ethyenimines (thiotepa, hexamethylmelamine); folic acid analogues (methotrexate); pyrimidine analogues (5-fluorouracil, cytosine arabinoside); purine analogues (6-mercaptopurine, 6-thioguanine); antitumor antibiotics (actinomycin D, the anthracyclines (doxorubicin), bleomycin, mitomycin C, methramycin); plant alkaloids such as vinca alkaloids (vincristine and vinblastine) and etoposide (VP-16); hormones and hormone antagonists (tamoxifen and corticosteroids); and miscellaneous agents (cisplatin, taxol and brequinar).

The above compounds to be employed in combination with the isoprenyl protein transferase inhibitor of the invention will be used in therapeutic amounts as indicated in the Physicians' Desk Reference (PDR) 47th Edition (1993), or by such therapeutically useful amounts as would be known to one of ordinary skill in the art.

The compounds of the invention and the other chemotherapeutic agent can be administered at the recommended maximum clinical dosage or at lower doses. Dosage levels of the active compounds in the compositions of the invention may be varied to obtain a desired therapeutic response depending on the route of administration, severity of the disease and the response of the patient.

When administered as a combination, the therapeutic agents can be formulated as separate compositions which are given at the same time or different times, or the therapeutic agents can be given as a single composition.

### Preparation of the Compounds of the Invention

In general, the compounds of the invention can be prepared by the processes illustrated in the following Schemes 1-16. In these general schemes compounds of the formula I are used to exemplify the methods, but the methods are intended to be applicable to all of the compounds of the invention.

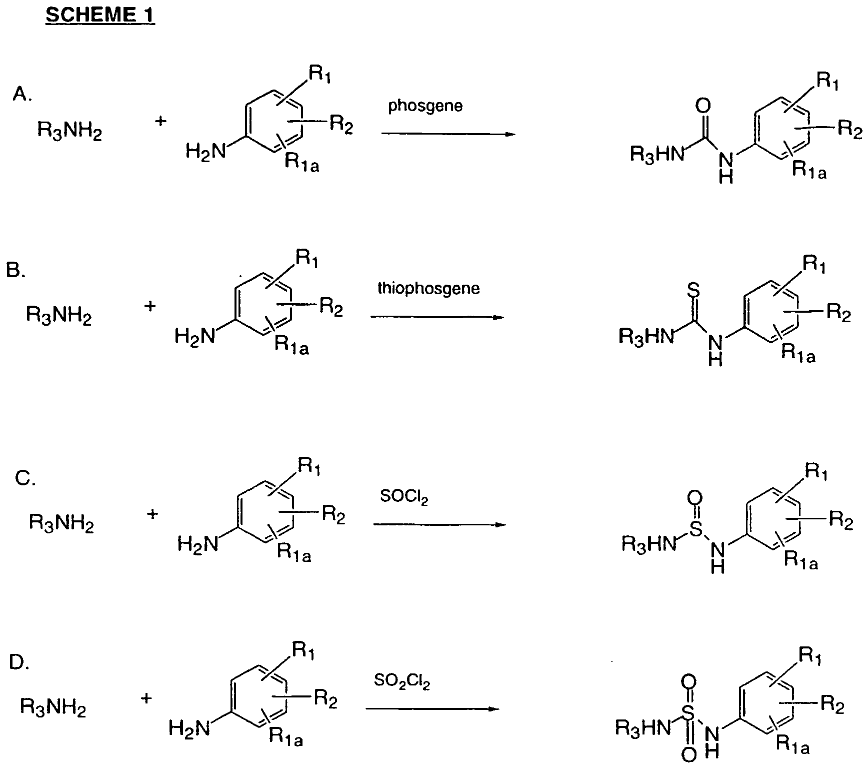

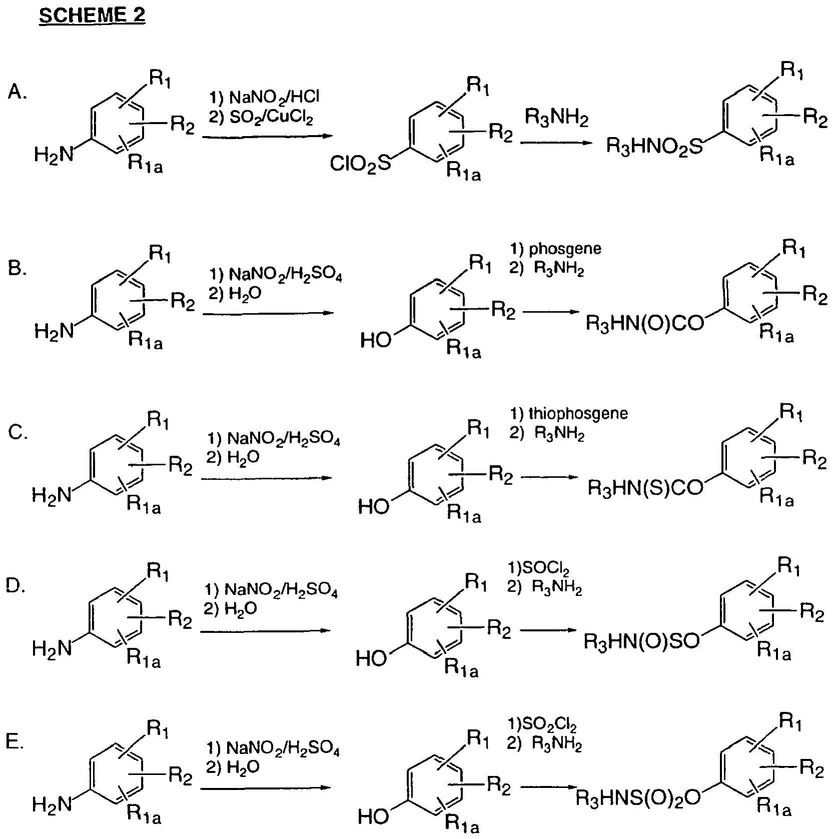

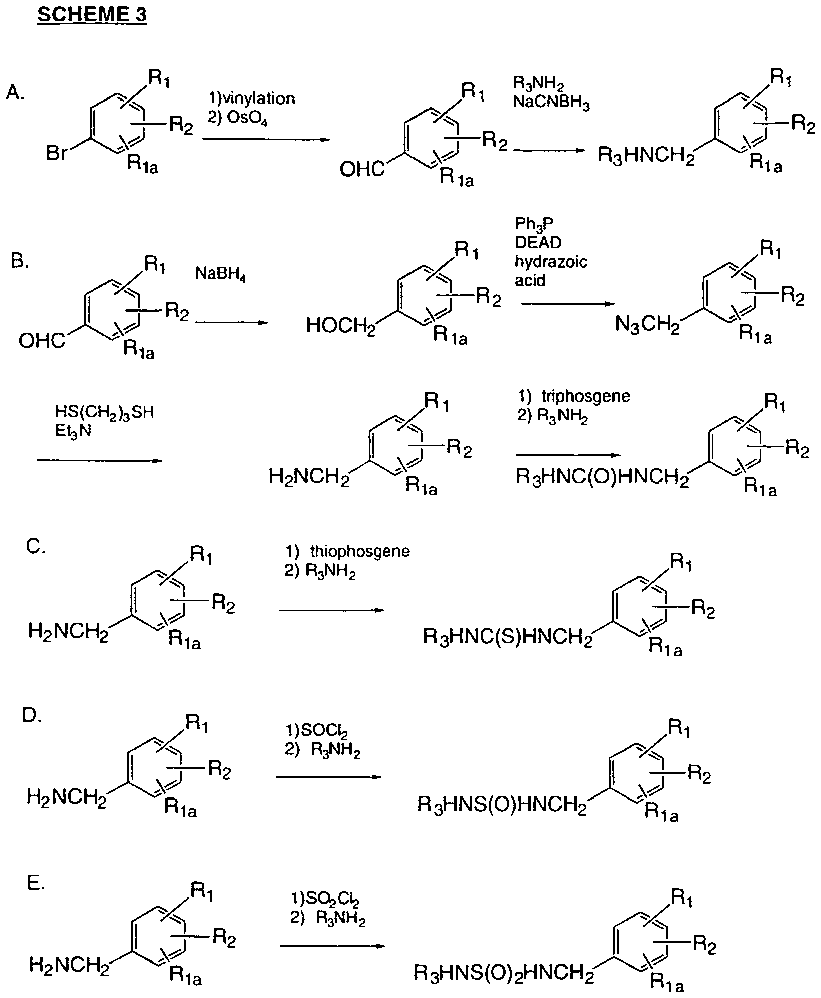

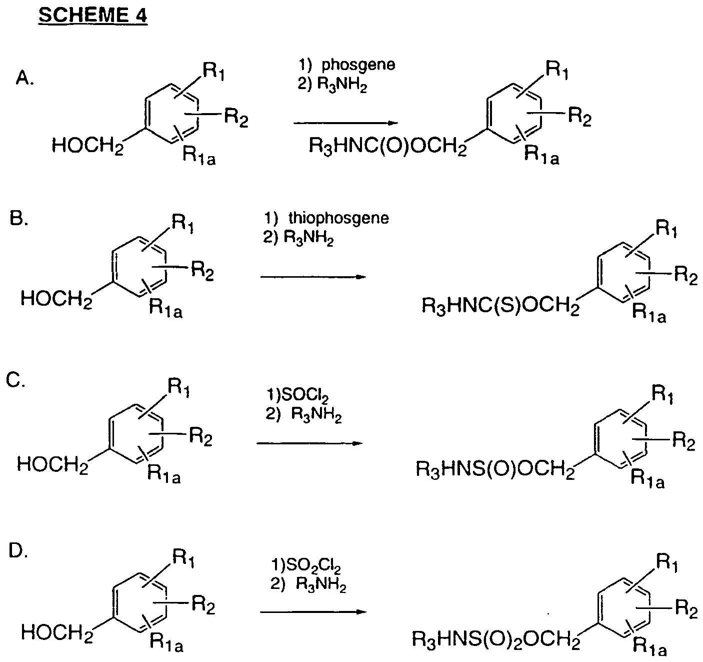

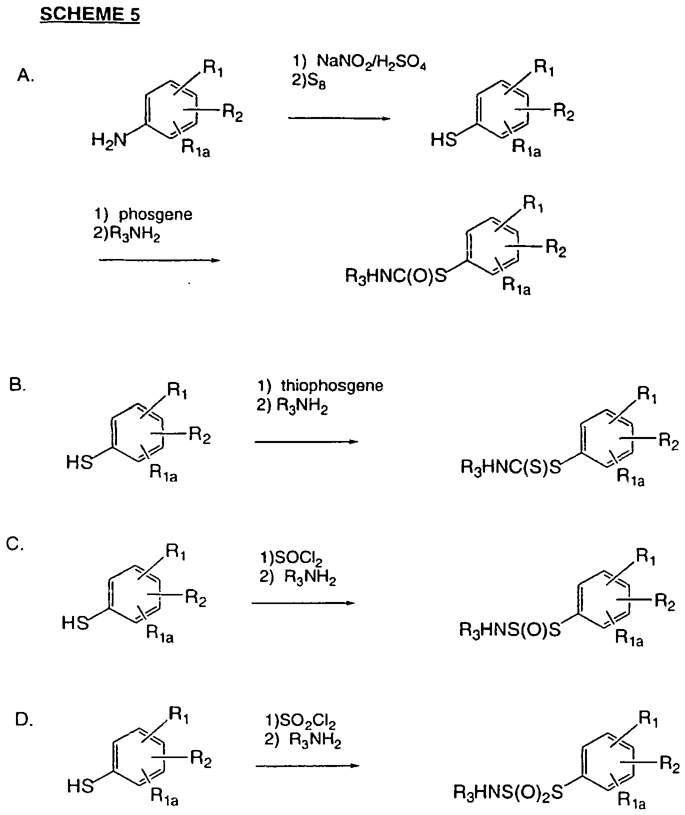

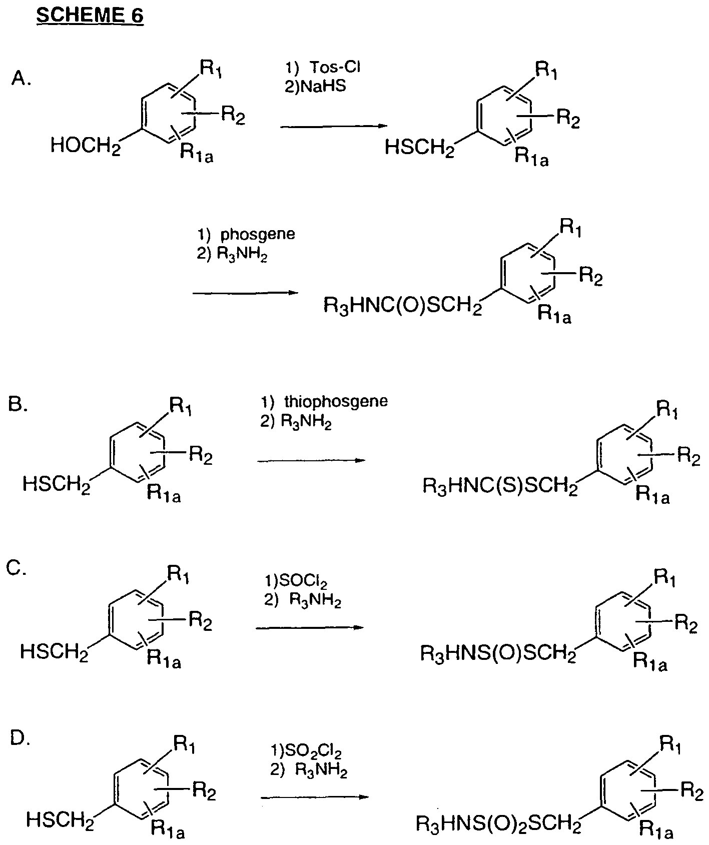

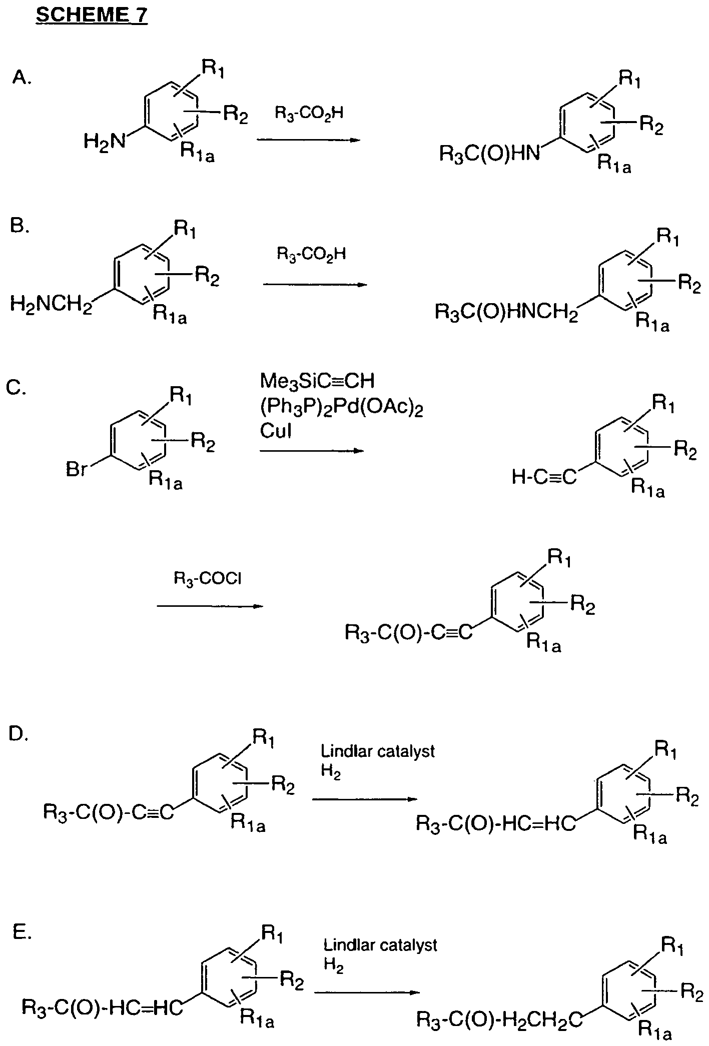

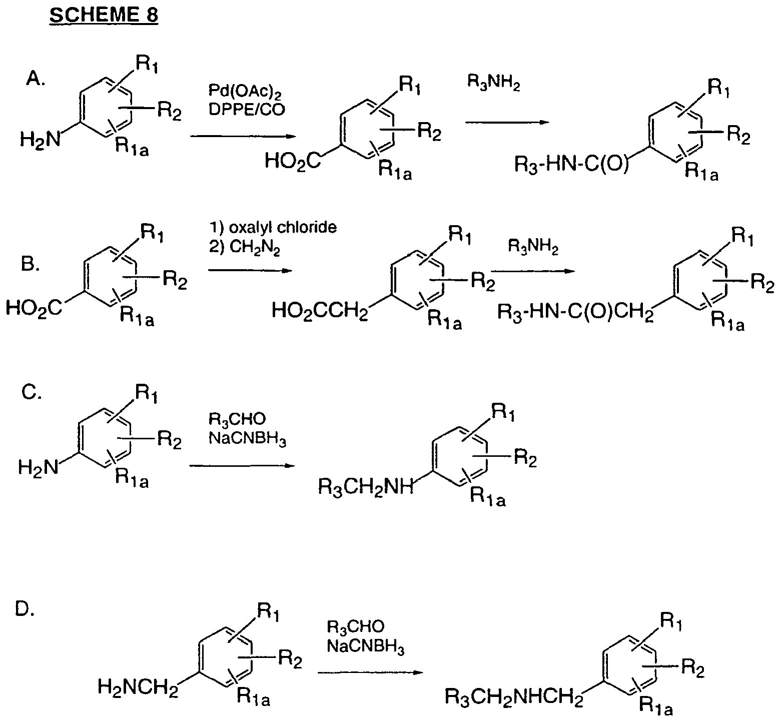

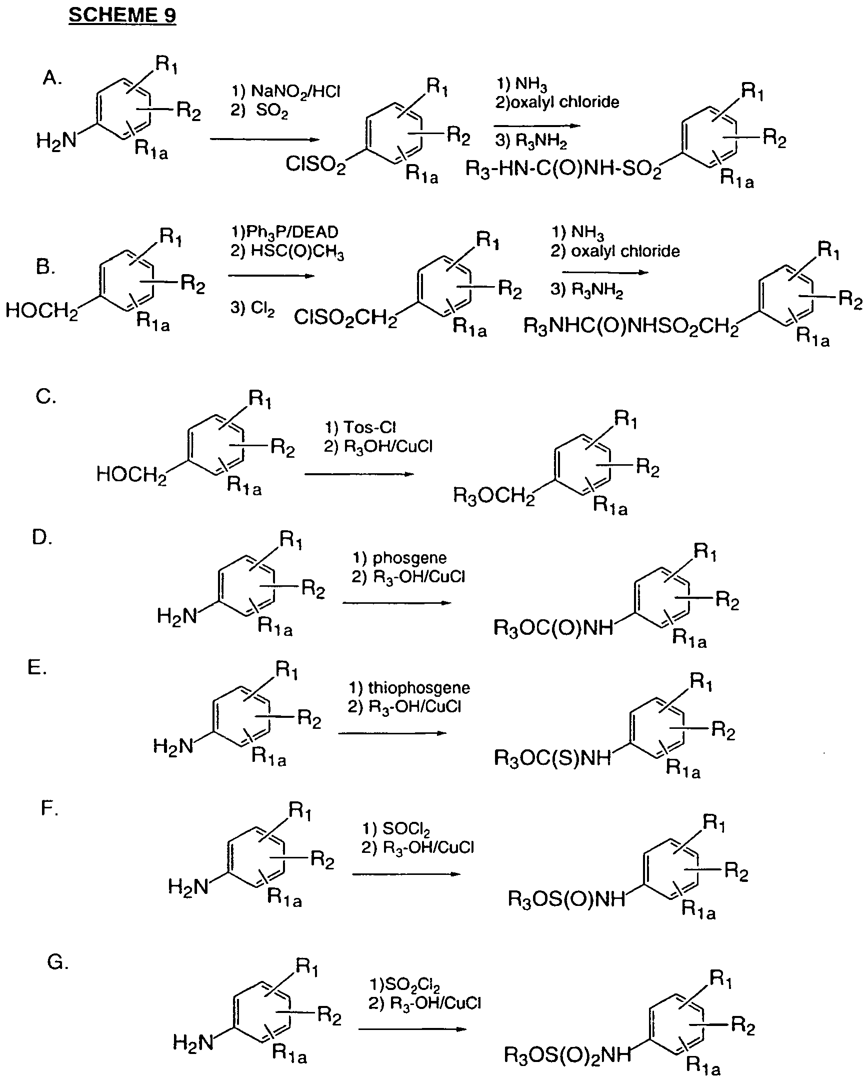

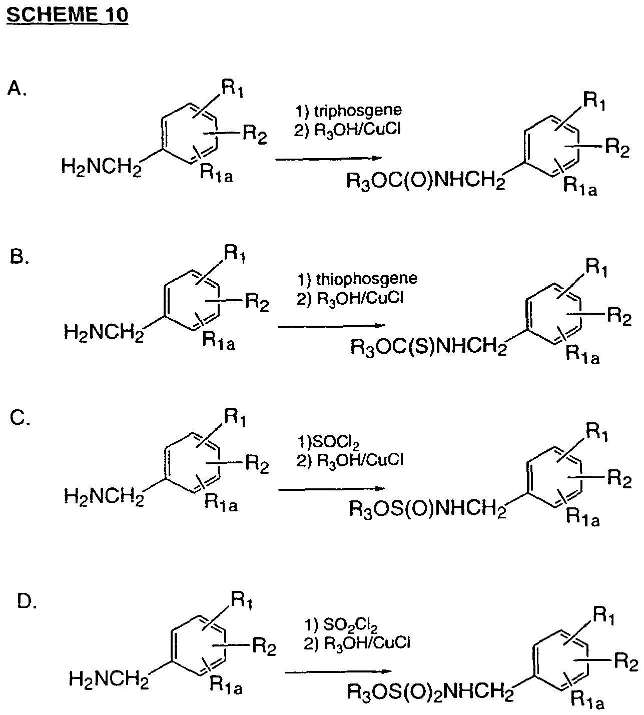

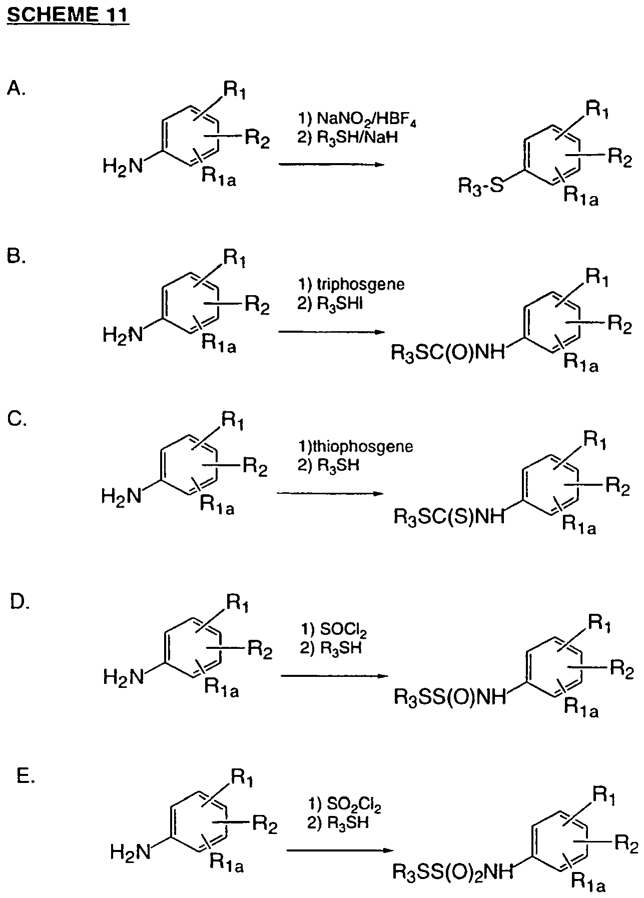

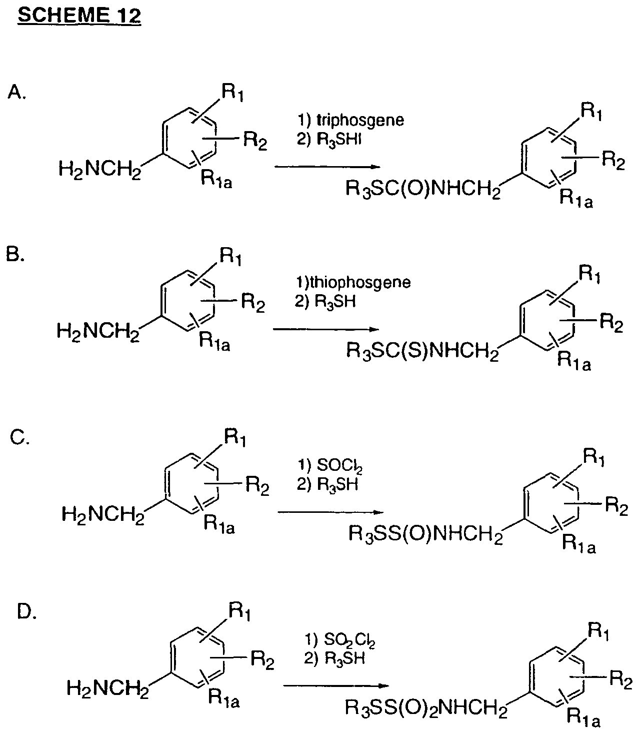

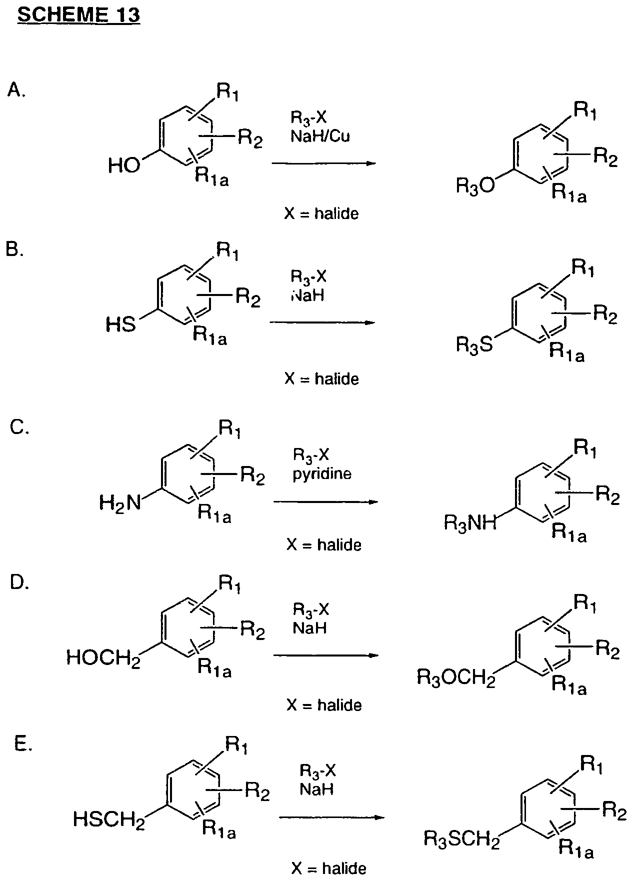

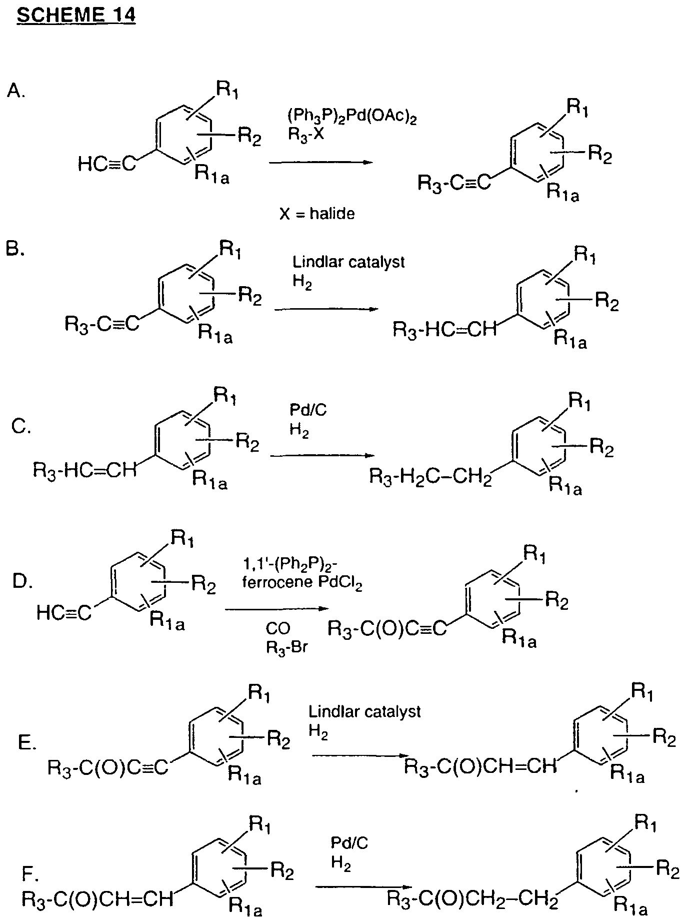

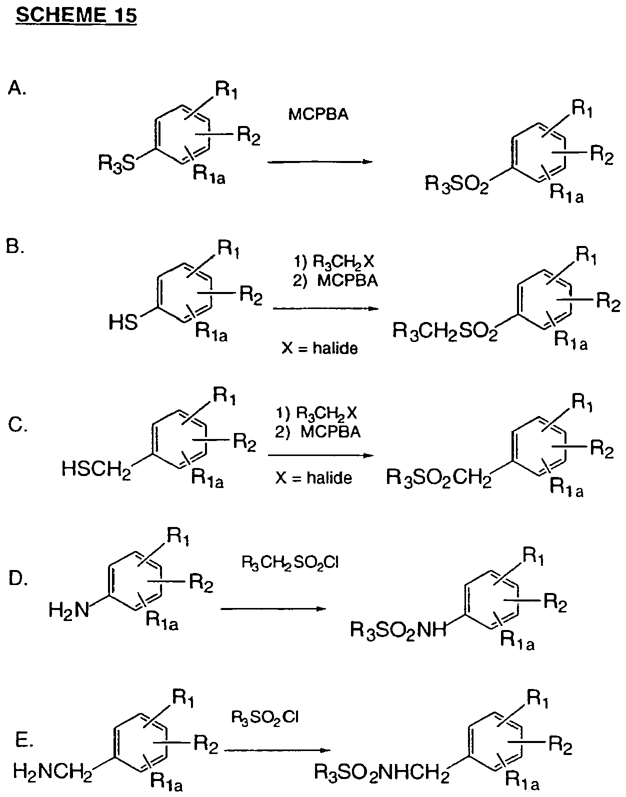

Scheme 16 illustrates an alternative method for preparing compounds wherein R₂ is -C(O)NH-CH(R₁₄)-C(O)OR₁₅ or 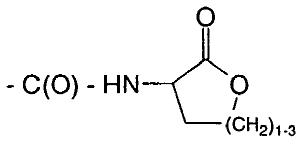 as defined above.

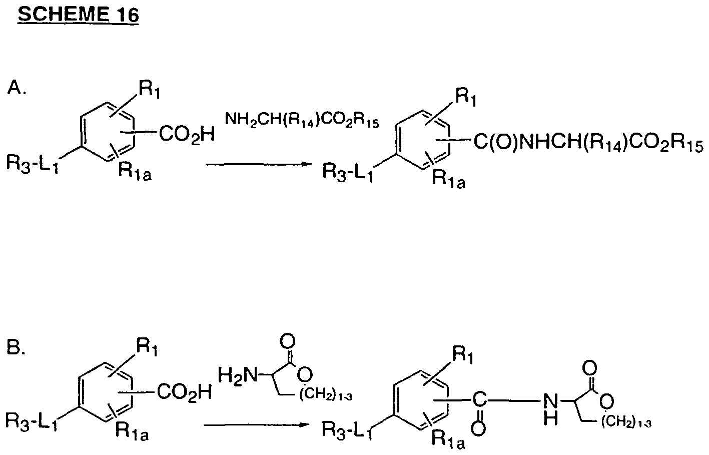

The foregoing may be better understood by reference to the following examples which are provided for illustration and not intended to limit the scope of the inventive concept.

In Tables 2-10, the abbreviation bz=benzoyl, bn=benzyl, Ph=phenyl, BOC=t-butyloxycarbonyl and TS=p-toluenesulfonyl.

### Compound 1

### (3-(Aminomethyl)benzoyl)-Met-OCH₃

### Step A

### (3-(Chloromethyl)benzoyl)-Met-OCH₃

To a solution of methionine methyl ester hydrochloride (2.0 g, 10 mmol) and 3-(chloromethyl)benzoyl chloride (2.08 g, 11.0 mmol) in methylene chloride (50 mL) was slowly added triethylamine (3.07 mL, 22.0 mmol) at ice bath temperature for 2 hours. The mixture was washed with 0.5 N HCl (50 mL x 2), brine (50 mL x 2) and water (50 mL x 2) then dried over anhydrous MgSO₄ and concentrated under reduced pressure. The residue was purified by flash column chromatography (30% ethyl acetate in hexanes) to give the desired product (3.03 g) as a white solid: m.p. 82-83°C;
¹H NMR (CDCl₃) d 7.82 (1H, s), 7.74 (1H, d, *J*=7.7 Hz), 7.53 (1H, d, *J*=7.7 Hz), 7.42 (1H, t, *J*=7.7 Hz), 7.06 (1H, br d, *J*=7.6Hz), 4.92 (1H, ddd, *J*=7,6, 7.1, 5.1 Hz), 4.59 (2H, s), 3.78 (3H, s), 2.58 (2H, t, *J*=7.1Hz) 2.26 (1H, sm), 2.15 (1H, m), 2.10 (3H, s); ¹³C NMR (CDCl₃) d 172.59, 166.54, 138.13, 134.25, 131.95, 129.12, 127.42, 126.97, 52.72, 52.14, 45.55, 31.47, 30.12, 15.55.

### Step B

### (3-(Azidomethyl)benzoyl)-Met-OCH₃

A suspension of (3-(chloromethyl)benzoyl)-Met-OCH₃ (1.58 g, 5.0 mmol) and sodium azide (1.3 g, 20.0 mmol) in DMSO (40 mL) was stirred at 80°C for 7 hours. The mixture was diluted with methylene chloride (100 mL), washed with brine (70 mL x 2) and water (70 mL x 2), and then dried over anhydrous MgSO₄. The solvent was evaporated under reduced pressure to give a yellow residue. Chromatography on silica gel (30% ethyl acetate in hexanes) to provide the desired product (1.45 g) as a colorless solid: m.p. 48-49°C; ¹H NMR (CDCl₃) d 7.78 (2H, m), 7.49 (2H, m), 6.99 (1H, br d, *J*=7.4 Hz), 4.49 (1H, ddd, *J*=7.4, 7.1, 5.2 Hz), 4.42 (2H, s), 3.80 (3H,s), 2.60 (2H, t, *J*=7.4 Hz), 2.29 (1H, m), 2.17 (1H, m), 2.12 (3H, s); ¹³C NMR (CDCl₃) d 177.50. 166.54, 135.97, 134.06, 131.18, 128.89, 126.84, 126.71, 54.09, 52.47, 51.95, 31.38, 30.00,15.30.

### Step C

### (3-(Aminomethyl)benzoyl)-Met-OCH₃

A suspension of (3-(azidomethyl)benzoyl)-Met-OCH₃ (1.29 g, 4.0 mmol) and 5% palladium on carbon (0.2 g) in methanol (40 mL) was stirred under a hydrogen atmosphere (1 atm) for two days at room temperature. The catalyst was removed by filtration through celite (1.5 g) and the solvent was evaporated in vacuo. The residue was washed with water (5 mL x 2) and dried to give the desired product (1.12 g) as a colorless foam. ¹H NMR (CDCl₃) d 7.81 (1H, s), 7.68 (1H, d, *J*=7.4 Hz), 7.45 (1H, d, *J*=6.5 Hz), 7.36 (1H, t, *J*=7.4 Hz), 4.91 (1H, ddd, *J*=7.3, 7.1, 5.1 Hz), 3.90 (2H, s), 3.77 (3H, s), 3.21 (2H, br s), 2.59 (2H, t, *J*=7.4 Hz), 2.20 (1H, m), 2.12 (1H, m), 2.09 (3H, s).

### Compound 2

### (4-(Aminomethyl)benzoyl)-Met-OCH₃

The title compound is prepared according to the procedure used to prepare Compound 1 but replacing 3-(chloromethyl)benzoyl chloride with 4-(chloromethyl)benzoyl chloride.

### Compound 3

### (3-Aminobenzoyl)-Met-OCH₃

The title compound was prepared according to the procedure described in J. Biol. Chem. 269 12410-12413 (1994).

### Compound 4

### (4-Aminobenzoyl)-Met-OCH₃

### Step A

### N-BOC-4-Aminobenzoic acid

4-Aminobenzoic acid (10 g, 72.9 mmol) was placed into a mixture of dioxane (145.8 mL) and 0.5 M NaOH (145.8 mL). The solution was cooled to 0°C and di-t-butyl dicarbonate (23.87 g, 109.5 mmol) was added. The reaction mixture was allowed to warm to room temperature and stirred overnight. The next day, the dioxane was removed, the residue was made acidic and extracted into ethyl acetate. The ethyl acetate fractions were combined and washed with 1N HCl to remove any unreacted starting material. The solution was dried over Na₂SO₄ and the solvent was removed in vacuo. The crude material was recrystallized from ethyl acetate/hexanes to provide the desired product (12.2 g): m.p. 189-190°C; ¹H NMR (CD₃OD) d 1.52 (9H, s), 7.49 (2H, d, *J*=8.6 Hz), 7.91 (2H, d, *J*=8.6 Hz), 9.28 (1H, s); ¹³C NMR (CD₃OD) d 28.59, 81.29, 118.54, 125.30, 131.81, 145.70, 155.00, 169.80; Anal. Calc. for C₁₂H₁₅NO₄, C: 60.76, H: 6.37, N: 5.90; Found, C: 60.52, H: 6.43, N: 5.83; HRMS Calc. for C₁₂H₁₅NO₄, 237.0961, Found, 237.1001.

### Step B

### (N-BOC-4-Aminobenzoyl)-Met-OCH₃

Into a dried, nitrogen filled flask was placed N-BOC-4-aminobenzoic acid (8.77 g, 36.97 mmol) in dry methylene chloride (148 mL) along with methionine methyl ester hydrochloride (8.12 g, 40.66 mmol). This solution was cooled in an ice bath and triethylamine (6.7 mL), EDCI (7.80 g, 40.66 mmol) and hydroxybenzotriazole (HOBT, 5.50 g, 40.66 mmol) were added. The mixture was stirred overnight, diluted with more methylene chloride and was extracted three times each with 1 M HCl, 1M NaHCO₃ and water. The methylene chloride was dried over MgSO₄ and the solvent was removed in vacuo. The resulting solid was recrystallized from ethyl acetate/hexanes to yield the desired product (9.72 g): m.p. 184-185°C; ¹H NMR (CDCl₃) d 1.53 (9H, s), 2.06-2.18 (4H, m), 2.23-2.33 (1H, m), 2.59 (2H, t, *J*=7.6 Hz), 3.80 (3H, s), 4.92 (1H, m), 7.45 (2H, d, *J*=8.7 Hz), 7.77 (2H, d, *J*=8.7 Hz); ¹³C NMR (CDCl₃) d 15.59, 28.34, 30.15, 31.64, 52.10, 52.73, 81.20, 117.73, 127.8, 128.33, 141.88, 152.33, 166.50, 172.75; Anal. Calc. for C₁₈H₂₆N₂O₅S, C: 56.53, H: 6.85, N: 7.29; Found, C: 56.47, H: 6.86, N: 7.29; m/z (EI) 382 (M).

### Step C

### (4-Aminobenzoyl)-Met-OCH₃ hydrochloride

N-BOC-4-aminobenzoyl-Met-OCH₃ (3.53 g, 9.59 mmol) was placed into methylene chloride (30-35 mL) and to it was added 3M HCl/EtO₂ (38.4 mL). After standing, a white precipitate formed. After two hours the solution was decanted and the crystals were collected by centrifugation. The crystals were then washed several times with fresh ether and dried overnight on the vacuum pump. Meanwhile, the filtrate was left to stand overnight to allow additional product to precipitate. The second fraction was washed with ether and dried overnight on the vacuum pump. The total yield of the desired product was 2.87 g: m.p.158-164°C; ¹H NMR (CDCl₃) d 2.10 (3H, s), 2.12-2.29 (1H, m), 2.52-2.71 (1H, m), 2.59 (2H, t, *J*=7.6 Hz), 3.75 (3H, s), 4.79 (1H, m), 7.02 (2H, d, *J*=8.6 Hz), 7.55 (2H, d, *J*=8.6 Hz); ¹³C NMR (CDCl₃) d 15.23, 31.43, 31.53, 52.91, 52.43, 124.35, 130.56, 135.31, 135.76, 168.95, 173.87; HRMS Calc. for C₁₃H₁₈N₂O₃S, 282.1038, Found 282.1009.

### Compound 5

### (4-Amino-3-methylbenzoyl-Met-OCH₃

### Step A

### N-BOC-4-Amino-3-methylbenzoic acid

4-Amino-3-methylbenzoic acid (5 g, 33.1 mmol) was reacted according to the same procedure as that used in the process for preparing N-BOC-4-aminobenzoic acid. The resulting orange-brown solid was recrystallized from ethyl acetate and hexanes to provide the desired product (4.99 g) as tan prismatic crystals: m.p. 180-182°C; 1H NMR (CD₃OD) d 1.51 (9h, s), 2.27 (3H, s), 7.66 (1H, d, *J*=8.1 Hz), 7.79-7.82 (2H, m), 8.32 (1H, s); 13C NMR (CD3OD) d 17.98, 28.62, 81.47, 123.12, 127.05, 129.14, 130.65, 132.99, 142.45, 155.33, 168.70; Anal. Calc. for C₁₃H₁₇NO₄, C: 62.15, H: 6.82, N: 5.58; Found C: 62.07, H: 6.86, N: 5.46; m/z (EI) 251; HRMS Calc. for C₁₃H₁₇NO₄, 251.1158; Found, 251.1153.

### Step B

### (N-BOC-4-Amino-3-methylbenzoyl)-Met-OCH₃

N-BOC-4-amino-3-methylbenzoic acid (2.00 g, 7.96 mmol) was reacted with with methionine methyl ester hydrochloride (1.75 g, 8.76 mmol), triethylamine (1.4 mL), EDCI (1.68 g, 8.76 mmol) and hydroxybenzotriazole (HOBT, 1.18 g, 8.76 mmol) in dry methylene chloride (31.8 mL) according to the procedure described for the preparation of N-BOC-4-aminobenzoyl)-Met-OCH₃. The resulting solid was recrystallized from ethyl acetate/hexanes to yield the desired product (2.61 g): m.p. 163-165°C; ¹H NMR (CDCl₃) d 1.54 (9H, s), 2.06-2.18 (4H, m), 2.23-2.34 (4H, m), 2.59 (2H, t, *J*=6.8 Hz), 3.80 (3H, s), 4.92 (1H, m), 6.45 (1H, s), 6.88 (1H, d, *J*=7.5 Hz), 7.63 (1H, d, *J*=8.6 Hz), 7.66 (1H, s), 8.05 (1H, d, *J*=8.6 Hz); ¹³C NMR (CDCl₃) d 15.47, 17.61, 28.22, 30.03, 31.55, 51.93, 52.57, 81.04, 118.73, 125.62, 127.66, 129.54, 139.89, 152.34, 166.58, 172.66.

### Step C

### (4-Amino-3-methylbenzoyl)-Met-OCH₃ hydrochloride

N-BOC-4-Amino-3-methylbenzoyl-Met-OCH₃ (0.99 g, 2.59 mmol) was dissolved in methylene chloride (15-20 mL) and precipitated with 3M HCl/Et₂O (20.7 mL). A pale orange precipitate was obtained, washed with ether and dried overnight on the vacuum pump. The total yield of the desired product was 0.83 g: m.p. 157-159°C; ¹H NMR (CD₃OD) d 2.04 (3H, s), 2.11-2.25 (1H, m), 2.47 (3H, s), 2.52-2.68 (3H, m), 3.74 (3H, s), 4.75-4.80 (1H, m), 7.48 (1H, d, *J*=8.2 Hz), 7.81 (2H, d, *J*=8.2 Hz), 7.87 (1H, s); ¹³C NMR (CD₃OD) d 15.23, 17.28, 31.43, 31.51, 52.91, 53.37, 124.41, 127.85, 131.99, 133.63, 134.14, 135.65, 169.05, 173.84; Anal. Calc. for C₁₄H₂₁N₂O₃S, C: 50.52, H: 6.36, N: 8.42; Found C: 50.71, H: 6.40, N: 8.34.

### Compound 6

### (4-Amino-3-methoxybenzoyl)-Met-OCH₃

### Step A

### N-BOC-4-Amino-3-methoxybenzoic acid

4-Amino-3-methoxybenzoic acid (1 g, 5.98 mmol) was reacted according to the same procedure as that used in the process for preparing N-BOC-4-aminobenzoic acid. The resulting solid was recrystallized from ethyl acetate and hexanes to provide the desired product (1.5 g) as tan crystals: m.p. 176-178°C; ¹H NMR (CD₃OD d 1.52 (9H, s), 3.92 (3H, s), 7.56 (1H, s), 7.62 (1H, d, *J*=8.4Hz), 7.96 (1H, s), 8.03 (1H, d, *J*=8.4 Hz); ¹³C NMR (CD₃OD d 28.53, 56.35, 81.78, 112.01, 118.58, 124.20, 125.76, 133.84, 149.04, 154.20, 169.60; HRMS Calc. for C₁₃H₁₇NO₅, 267.1107; Found, 267.1103.

### Step B

### (N-BOC-4-Amino-3-methoxybenzoyl)-Met-OCH₃

N-BOC-4-amino-3-methoxybenzoic acid (0.35 g, 1.31 mmol) was reacted with with methionine methyl ester hydrochloride (0.9 g, 1.43 mmol) using EDCI according to the procedure described for the preparation of (N-BOC-4-aminobenzoyl)-Met-OCH₃. The resulting solid was recrystallized from ethyl acetate/hexanes to yield the desired product (0.36 g): m.p. 163-165°C; ¹H NMR (CDCl₃) d 1.53 (9H, s), 2.09-2.18 (4H, m), 2.23-2.35 (1H, m), 2.60 (2H, t, *J*=6.9 Hz), 3.80 (3H, s), 3.93 (3H, s), 4.92 (1H, br s), 6.93 (1H, d, *J*=7.6 Hz), 7.25(1H, m), 7.31 (1H, d, *J*=10.2 Hz), 7.44 (1H, s), 8.15 (1H, d, *J*=8.5 Hz); ¹³C NMR (CDCl₃) d 15.47, 28.23, 30.09, 31.48, 52.06, 52.54, 55.81, 80.82, 98.06, 109.38, 116.66, 119.31, 131.52, 147.23, 152.31, 166.57, 172.58; m/z (FAB)413(M+1).

### Step C

### (4-Amino-3-methoxybenzoyl)-Met-OCH₃ hydrochloride

N-BOC-4-Amino-3-methoxybenzoyl-Met-OCH₃ (0.71 g, 1.79 mmol) was dissolved in methylene chloride (4 mL) and precipitated with 3M HCl/Et₂O (12 mL). A reddish precipitate was obtained, washed with ether and dried overnight on the vacuum pump. The total yield of the desired product was 0.55 g: m.p. 176-177°C; ¹H NMR (CD₃OD) d 2.08 (3H, s), 2.21 (2H, m), 2.61 (2H, m), 3.74 (3H, s), 4.02 (3H, s), 4.79 (1H, m), 7.50 (1H, d, *J*=8.2 Hz), 7.57 (1H, d, *J*=4.1 Hz), 7.67 (1H, s); ¹³C NMR (CD₃OD) d 15.26, 31.34, 31.42, 52.95, 53.38, 57.12, 112.29, 121.43, 124.57, 124.77, 136.15, 153.67, 168.79, 173.81.

### Compound 7

### (4-Amino-1-naphthoyl)-Met-OCH₃

### Step A

### 4-Amino-1-naphthoic acid

4-Amino-1-naphthalenecarbonitrile (1.5 g, 8.91 mmol) was suspended in a 50% KOH solution (18 mL). The heterogeneous solution was heated at reflux for 2-3 days. Once the solution became homogeneous and TLC showed no more starting material, the deep red solution was cooled and poured over 200 mL of water. The resulting solution was then filtered and the desired product was precipitated with concentrated HCl. The resulting red crystals were filtered and the filtrate was refiltered to give pink crystals. The first fraction of crystals was treated with activated carbon to remove some of the red color. A total of 1.51 g of the desired product was obtained: m.p. 169-171°C; ¹H NMR (CD₃OD) d 6.69 (1H, d, *J*=8.2 Hz), 7.38-7.43 (1H, m), 7.48-7.54 (1H, m), 8.03 (1H, d, *J*=8.5 Hz), 8.13 (1H, d, *J*=8.2 Hz), 9.09 (1H, d, *J*=8.5 Hz); ¹³C NMR (CD₃OD) d 107.39, 114.61, 122.99, 123.92, 125.21, 127.40, 128.48, 135.04, 151.35, 171.44; HRMS Calc. for C₁₁H₇NO₂, 187.0633; Found, 187.0642.

### Step B

### N-BOC-4-Amino-1-naphthoic acid

4-Amino-1-naphthoic acid (0.86 g, 4.61 mmol) was dissolved in dioxane (9.2 mL). Di-t-butyl dicarbonate (1.11 g, 5.07 mmol) was added and the mixture was stirred overnight. The reaction mixture was worked up as described above for N-BOC-4-aminobenzoic acid to give 0.76 g of the desired product as a reddish pink solid: m.p. 194-195°C; ¹H NMR (CD₃OD) d 1.56 (9H, s), 7.53-7.62 (2H, m), 7.79 (1H, d, *J*=8.1 Hz), 8.12 (1H, d, *J*=8.0 Hz), 8.22 (1H, d, *J*=8.18 Hz), 9.02 (1H, d, *J*=8.9 Hz); ¹³C NMR (CD₃OD) d 26.68, 81.62, 119.06, 123.40, 124.57, 127.03, 127.37, 128.49, 128.77, 131.89, 133.76, 139.86, 155.95, 170.73; Anal. Calc. for C₁₇H₁₇NO₄, C: 66.90, H: 5.96, N: 4.88; Found C: 66.49, H: 6.08, N: 4.79; m/z (EI), 289; HRMS Calc. for C₁₆H₁₇NO₄, 287.1158; Found, 287.1151.

### Step C

### (N-BOC-4-Amino-1-naphthoyl)-Met-OCH₃

N-BOC-4-Amino-naphthoic acid (0.46 g, 1.60 mmol), methionine methyl ester hydrochloride (0.35 g, 1.76 mmol), EDCl (0.43 g, 1.76 mmol), HOBT (0.24 g, 1.76 mmol) and triethylamine (0.27 mL) in methylene chloride (6.4 mL) were reacted as described above for N-BOC-4-aminobenzoyl-Met-OCH3. After workup and recrystallization from ethyl acetate hexanes, the desired product (0.44 g) was obtained as pale pink crystals: m.p. 131-132°C; ¹H NMR (CDCl₃) d 1.57 (9H, s), 2.11-2.21 (4H, m), 2.29-2.41 (1H, m), 2.65 (2H, t, *J*=7.1 Hz), 3.83 (3H, s), 4.99-5.06 (1H, m), 6.68 (1H, d, *J*=8.0 Hz), 7.02 (1H, s), 7.56-7.59 (2H, m) 7.69 (1H, d, *J*=7.9 Hz), 7.87-7.90 (1H, m), 8.02 (1H, d, *J*=7.9 Hz), 8.44-8.48 (1H, m); ¹³C NMR (CDCl₃) d 15.56, 28.31, 30.19, 31.65, 52.06, 52.64, 81.17, 115.82, 120.18, 125.79, 126.37, 126.53, 127.18, 131.02, 135.65, 152.93, 169.04, 172.40; HRMS Calc. for C₂₂H₂₈N₂O₅S, 432.1719; Found, 432.1702; m/z (FAB) 433 (M+1).

### Step D

### (4-Amino-1-naphthoyl)-Met-OCH₃ hydrochloride

(N-BOC-4-Amino-l-naphtholyl)-Met-OCH₃ (0.57 g, 1.31 mmol) was deprotected with HCl/ether to yield the desired product (0.31 g) as a white solid: m.p. 178-181°C; ¹H NMR (CD₃OD) d 2.08-2.16 (4H, m), 2.20-2.30 (1H, m) 2.57-2.75 (2H, m) 3.82 (3H, s), 4.87-4.91 (1H, m), 7.59 (1H, d, *J*=7.5 Hz), 7.67 (1H, d, *J*=7.5 Hz) 7.71-7.80 (2H, m), 8.03 (1H, dd, *J*=7.1, 2.0 Hz), 8.35 (1H, dd, *J*=6.8, 1.8 Hz); ¹³C NMR (CD₃OD) d 15.23, 31.40, 53.01, 53.33, 119.90, 122.20, 126.15, 127.41,127.77, 129.09, 129.31, 131.50, 132.33, 135.64, 171.77, 173.83; m/z (FAB), 369 (M+1).

### Compound 8

### (4-Amino-2-phenylbenzoyl)-Met-OCH₃

### Step A

### 4-Nitro-2-phenyltoluene

2-Bromo-4-nitrotoluene (2.16 g, 10.00 mmol) and phenylboric acid (1.46 g, 12.00 mmol) were dissolved in anhydrous DMF (25 mL) under nitrogen. To this mixture was added Pd(Ph₃P)₄ (0.58 g, 5%). The mixture was heated at 100°C overnight. The solution was poured onto 1N HCl and extracted with Et₂O. The crude product was chromatographed on silica gel using hexanes as eluent. After recrystallization from ethanol, the desired product (1.23 g) was obtained as pale orange needles: m.p. 69-71°C; ¹H NMR (CDCl₃) d 2.36 (3H, s), 7.29-7.40 (2H, m), 7.41-7.49 (5H, m), 8.07-8.10 (2H, m); ¹³C NMR (CDCl₃) d 20.68, 121.96, 124.51, 127.78, 128.41, 128.83, 131.06, 139.06, 139.44, 142.97, 143.48, 146.05; Anal. Calc. for C₁₃H₁₁NO₂, C: 73.26, H: 5.20, N: 6.57; Found, C: 73.10, H: 5.12, N: 6.50; m/z (EI) 213; HRMS Calc. for C₁₃H₁₁NO₂, 213.0790; Found, 213.0793.

### Step B

### 4-Nitro-2-phenylbenzoic acid

4-Nitro-2-phenyltoluene (0.5 g, 2.34 mmol) was dissolved in water (4.6 mL) and pyridine (2.3 mL). The mixture was heated to reflux and KMnO₄ (1.85 g, 11.7 mmol) was added. The reaction mixture was heated overnight and the solution was filtered and washed several times with boiling water. The aqueous solution was made acidic and the product was extracted into ethyl acetate. The ethyl acetate solution was dried over Na₂SO₄ and the solvent removed in vacuo to provide the desired product (0.37 g): m.p. 174-176°C, ¹H NMR (CD₃OD) d 7.38-7.48 (5H, m), 7.96 (1H, d, *J*=8.5 Hz), 8.21 (1H, d, *J*=2.3 Hz), 8.28 (1H, dd, *J*=8.48, 2.37 Hz); ¹³C NMR (CD₃OD) d 122.95, 126.09, 129.27, 129.42, 129.49, 131.56, 139.26, 140.42, 144.41, 150.17, 170.52; m/z (EI) 243 (M).

### Step C

### (4-Nitro-2-phenylbenzoyl-Met-OCH₃

4-Nitro-2-phenylbenzoic acid (0.3 g, 1.23 mmol), methionine methyl ester hydrochloride salt (0.27 g, 1.35 mmol), EDCI (0.26 g, 1.35 mmol), HOBT (0.18 g, 1.35 mmol) and triethylamine (0.19 mL) in dry methylene chloride (4.9 mL) were reacted according the procedure described above for (N-BOC-4-aminobenzoyl)-Met-OCH₃. After recrystallization of the product from ethyl acetate hexanes, the desired product (0.41 g) was obtained: m.p. 98-101°C; ¹H NMR (CDCl₃) d 1.62-1.73 (1H, m), 1.79-1.88 (1H, m), 1.91 (3H, s), 1.99 (2H, t, *J*=7.2 Hz), 3.59 (3H, s), 4.53 (1H, m), 6.45 (1H, d, *J*=7.8 Hz), 7.33-7.40 (5H, m), 7.67 (1H, d, *J*=8.3 Hz), 8.07-8.12 (2H, m); ¹³C NMR (CDCl₃) d 14.92, 29.11, 30.67, 51.51, 52.29, 121.86, 124.74, 128.27, 128.60, 128.69, 129.52, 137.50, 140.56, 141.02, 148.09, 167.23, 171.23; m/z (FAB), 389 (M+1).

### Step D

### (4-Amino-2-phenylbenzoyl)-Met-OCH₃

(4-Nitro-2-phenylbenzoyl)-Met-OCH₃ (0.35 g, 0.90 mmol) was dissolved in ethyl acetate (9.0 mL). To this mixture was added SnCl₂ · 2H₂O (1.02 g, 4.5 mmol) and the reaction mixture was heated under nitrogen at reflux for one hour. The mixture was poured onto ice, the solution was made basic using NaHCO₃ and the product was extracted into ethyl acetate several times (7-8). The ethyl acetate solutions were combined, washed with brine and dried over Na₂SO₄. The solvent was removed in vacuo to the desired product (0.24 g) as a yellow solid: ¹H NMR (CDCl₃) d 1.58-1.70 (1H, m), 1.80-1.92 (1H, m), 1.98 (3H, s), 2.06 (2H, t, *J*=7.7 Hz), 3.62 (3H, s), 4.00 (2H, br s), 4.56-4.63 (1H, m), 5.84 (1H, d, *J*=7.7 Hz), 6.50 (1H, s), 6.61 (1H, d, *J*=8.4 Hz) 7.29-7.42 (5H, m), 7.58 (1H, d, *J*=8.3 Hz); ¹³C NMR (CDCl₃) d 15.02, 29.25, 31.25, 51.57, 52.15, 113.27, 115.88, 123.52, 127.56, 128.37, 128.44, 130.92, 140.66, 141.44, 148.53, 168.58, 171.91.

### Compound 9

### (4-Amino-2-(2-thienyl)benzoyl)-Met-OCH₃

The title compound can be prepared according to the method used to prepare Compound 8, only substituting thiophene-2-boronic acid for phenyl boronic acid.

### Compound 10

### (4-Amino-2-(1-naphthyl)benzoyl)-Met-OCH₃

The title compound can be prepared according to the method used to prepare Compound 8, only substituting 1-naphthylboronic acid for phenylboronic acid.

### Compound 11

### 4-Amino-3'-methylbiphenyl

The title compound was prepared by Suzuki coupling of 1-bromo-4-nitrobenzene and 1-bromo-3-methylbenzene.

### Compound 12

### 4-Amino-4'-biphenyl carboxylic acid

### Step A

### 4-Nitro-4'-methylbiphenyl

The title compound was prepared by Suzuki coupling of 1-bromo-4-nitrobenzene and 1-bromo-4-methylbenzene.

### Step B

### 4-Nitro-4'-biphenyl carboxylic acid

The title compound was prepared by KMnO₄ oxidation of 4-nitro-4'-methylbiphenyl.

### Step C

### 4-Amino-4'-biphenyl carboxylic acid

The title compound can be prepared by palladium catalyzed hydrogenation of 4-nitro-4'-biphenyl carboxylic acid.

### Compound 13

### 4-Amino-3'-biphenyl carboxylic acid

### Step A

### 4-Nitro-3'-methylbiphenyl

The title compound was prepared by Suzuki coupling of 1-bromo-4-nitrobenzene and 1-bromo-3-methylbenzene.

### Step B

### 4-Nitro-3'-biphenyl carboxylic acid

The title compound was prepared by KMnO₄ oxidation of 4-nitro-3'-methylbiphenyl.

### Step C

### 4-Amino-3'-biphenyl carboxylic acid

The title compound can be prepared by palladium catalyzed hydrogenation of 4-nitro-3'-biphenyl carboxylic acid.

### Compound 14

### 4-Amino-2-methoxy-3'-biphenyl carboxylic acid

### Step A

### 2-Methoxy-4-nitro-3'-methylbiphenyl

The title compound was prepared by reaction of 1-bromo-2-methoxy-4-nitrobenzene with 3-methylphenylboronic acid in the presence of palladium acetate.

### Step B

### 2-Methoxy-4-nitro-3'-biphenylcarboxylic acid

The title compound was prepared by KMnO₄ oxidation of 2-methoxy-4-nitro-3'-methylbiphenyl.

### Step C

### 4-Amino-2-methoxy-3'-biphenyl carboxylic acid

The title compound can be prepared by palladium catalyzed hydrogenation of 2-methoxy-4-nitro-3'-biphenyl carboxylic acid.

### Compound 15

### 4-Amino-2-isopropyloxy-3'-biphenyl carboxylic acid

The title compound can be prepared by methods analogous to those used to prepare Compound 14.

### Compound 16

### 4-Amino-2-phenyl-3'-biphenylcarboxylic acid

The title compound can be prepared by methods analogous to those used to prepare Compound 14.

### Compound 17

### (4-Amino-2-(3,5-dimethylphenyl)benzoyl)-Met-OCH₃

### Step A

### 2-Bromo-4-nitrobenzoic acid

2-Bromo-4-nitrotoluene (5.0 g, 23.14 mmol) was dissolved in pyridine (23 mL) and water (46 mL). The heterogeneous mixture was heated to 60°C and KMnO₄ (18.29 g, 115.7 mmol) was added carefully. The mixture was then heated under reflux overnight. The reaction mixture was filtered and washed with boiling water. The solution was then made acidic and extracted into ethyl acetate, dried over Na₂SO₄ and the solvent was removed in vacuo. The crude product was dissolved in aqueous NaOH and washed with hexanes. The aqueous phase was made acidic and the product was extracted into ethyl acetate. The ethyl acetate solutions were combined and dried over Na₂SO₄ and the solvent was removed in vacuo to provide the desired product (3.72 g): m.p. 158-160°C; ¹H NMR (CD₃OD) d 7.81 (1H, d, *J*=8.5 Hz), 8.08 (1H, d, *J*=8.5 Hz), 8.30 (1H, s); ¹³C NMR (CD₃OD) d 121.96, 122.75, 129.36, 132.24, 139.52, 149.54, 167.75; Anal. Calc. for C₇H₄BrNO₄ ·0.1 ethyl acetate, C: 34.88, H: 1.90, N: 5.50; Found, C: 34.68, H: 1.86, N: 5.82.

### Step B

### 3.5-Dimethylphenylboronic acid

Magnesium turnings (1.44 g, 59.43 mmol) were coverd with dry THF (18.8 mL) in a dried, nitrogen filled flask fitted with an addition funnel and reflux condenser. To this was added 5-bromo-m-xylene (10 g, 54.03 mmol) in THF (15 mL) after initiation of the Grignard reaction. The addition was carried out over several minutes and the reacton mixture was heated at reflux for 1-2 hours until most of the magnesium had reacted. The reaction mixture was then cooled and transferred to an addition funnel fitted to an nitrogen filled flask containing triisopropyl borate (24.9 mL) at -70°C. The dropwise addition was carried out over several minutes and the mixture warmed to room temperature and stirred overnight. The grey solution was poured onto 2 M HCl and immediately turned yellow. The solution was extracted with Et₂O and the Et₂O fractions were combined, dried over MgSO₄ and the solvent was removed in vacuo to provide the desired product (2.41 g): m.p.249-251°C; ¹H NMR (CDCl₃) d 2.44 (6H, s), 7.23 (1H, s), 7.84 (2H, s); ¹³C NMR (CD₃OD) d 21.36, 133.28, 134.39, 137.48.

### Step C

### 4-Nitro-2-(3,5-dimethylphenyl)benzoic acid

2-Bromo-4-nitrobenzoic acid (0.43 g, 2.03 mmol) and 3,5-dimethylphenyl boronic acid (0.334 g, 2.23 mmol) were dissolved in anhydrous DMF (25 mL) under nitrogen. To this mixture was added Cs₂CO₃ (1.66 g, 5.08 mmol) followed by Pd(Ph₃P)₄ (0.12 g, 5%). The mixture was heated at 100°C overnight. The solution was poured onto 1N HCl and extracted with Et₂O. It was dried over MgSO₄ and the solvent was removed in vacuo. The crude product was chromatographed on silica gel using a 9:1 mixture of hexanes and ethyl acetate to provide the desired product (0.34 g): ¹H NMR (CDCl₃) d 2.36 (6H, s), 6.99 (2H, s), 7.07 (1H, s), 8.03 (1H, d, *J*=9.0 Hz), 8.23-8.25 (2H, m); ¹³C NMR (CDCl₃) d 21.28, 121.68, 123.68, 125.74, 126.07, 130.22, 131.19, 131.31, 135.04, 138.21, 144.74, 170.75.

### Step D

### (4-Nitro-2-(3,5-dimethylphenl)benzoyl)-Met-OCH₃

4-Nitro-2-(3,5-dimethylphenyl)benzoic acid (0.15 g, 0.55 mmol), methionine methyl ester hydrochloride (0.11 g, 0.55 mmol), EDCI (0.11 g, 0.55 mmol), HOBT (0.07 g, 0.55 mmol) and triethylamine (0.08 mL) in dry methylene chloride (2.2 mL) were reacted and worked up according to the procedure for (N-BOC-4-aminobenzoyl)- Met-OCH₃ as described above. After recrystallization from ethyl acetate and hexanes, the desired product was obtained (0.13 g): m.p. 122-124°C; ¹H NMR (CDCl₃) d 1.2-1.84 (1H, m), 1.85-1.97 (1H, m), 2.01 (3H, s), 2.05 (3H, t, *J*=7.7Hz), 2.38 (6H, s), 3.70 (3H, s), 4.67-4.74 (1H, m), 6.03 (1H, d, *J*=7.9 Hz), 7.05 (2H, s), 7.09 (1H, s), 7.84-7.87 (1H, m), 7.84-7.87 (1H, m) 8.23-8.26 (2H, m); ¹³C NMR (CDCl₃) d 15.20, 21.26, 29.22, 31.15, 51.79, 52.57, 122.07, 125.11, 126.27, 130.03, 130.53, 137.77, 138.82, 140.29, 141.56, 148.41, 167.14, 171.53.

### Step E

### (4-Amino-2-(3,5-dimethylphenyl)benzoyl)-Met-OCH₃

(4-Nitro-2-(3,5-dimethylphenyl)benzoyl)-Met-OCH₃ (0.11 g, 0.26 mmol) was dissolved in ethyl acetate (3.0 mL). To this mixture was added SnCl₂ · 2H₂O (0.3 g, 1.30 mmol) and the reacton was heated under nitrogen at reflux for 6 hours. The mixture was worked up as described above for (4-amino-2-phenylbenzoyl)-Met-OCH₃ to give the desired product (0.15 g): ¹H NMR (CDCl₃) d 1.60-1.70 (1H, m), 1.80-1.90 (1H, m), 1.99 (3H, s), 2.05 (2H, t, *J*=7.6 Hz), 2.33 (6H, s), 3.64 (3H, s), 3.93 (2H, br s), 4.61-4.64 (1H, m), 5.82 (1H, d, *J*=7.7 Hz), 6.49 (1H, d, *J*=2.3 Hz) 6.62 (1H, dd, *J*=8.4, 2.4 Hz), 6.98 (2H, s), 7.00 (1H, s), 7.65 (1H, d, *J*=8.3 Hz); ¹³C NMR (CDCl₃) d 15.08, 21.17, 29.28, 31.49, 51.70, 52.18, 113.30, 115.94, 123.55, 126.36, 129.32, 131.23, 138.15, 140.72, 141.92, 148.40, 168.45, 172.01.

### Preparation 1

### Anilines of the formula B-NH₂

The anilines from Table 1, entries 10-126 (B-NH₂) are prepared using the procedures for Compounds 1-18 with the exception that methionine methyl ester is replaced by methioninesulfone methyl ester, (S-Me)cysteine methyl ester, serine methyl ester, (O-Me)serine methyl ester, (O-Me)homoserine methyl ester, homoserine lactone, isoleucine methyl ester, leucine methyl ester, norleucine methyl ester, norvaline methyl ester, cyclohexylalanine methyl ester, phenylalanine methyl ester, or glutamic acid dimethyl ester.

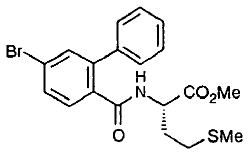

### Preparation 2

### 4-Bromo-2-phenylbenzoyl methionine methyl ester

### Preparation 2A

### 4-Bromo-2-phenylbenzoic acid methyl ester

A solution of methyl 4-amino-2-phenylbenzoic acid (1.0 equivalent) in dilute aqueous HBr is treated with NaNO₂ (1.1 equivalents) to form the diazonium salt. The reaction is treated with CuBr (1.1 equivalents) and heated. When judged complete by TLC analysis, the mixture is extracted into ethyl acetate which is dried and evaporated. The title arylbromide is purified by chromatography on silica gel.

### Preparation 2B

### 4-Bromo-2-phenylbenzoic acid

To a solution of the resultant compound from Preparation 2A (1.0 equivalent) in a 3:1 mixture of tetrahydrofuran (THF) and water is added an excess (1.5 equivalents) of LiOH. When hydrolysis is judged complete by TLC analysis, the solvent is evaporated and the remaining aqueous layer is acidified to pH = 3 and extracted into ethyl acetate which is dried and evaporated prior to purification by chromatography on silica gel.

### Preparation 2C

### 4-Bromo-2-phenylbenzoyl methionine methyl ester

To a solution of the resultant compound from Preparation 2B (1.0 equivalent) in dimethylformamide (DMF) is added 3-hydroxy-1,2,3-benzotriazin-4(3H)-one (1.5 equivalents) followed by methionine methyl ester (1.0 equivalent) and 1-(3-dimehtylaminopropyl)-3-ethylcarbodiimide hydrochloride (1.5 equivalents). When judged complete by TLC analysis, the reaction is taken up in ethyl acetate which is washed by 1N HCl and saturated brine, and then is dried and evaporated. The crude reaction mixture is purified by column chromatography to afford the title product.

### Preparation 2D

### 4-Bromo-2-phenylbenzoyl methionine methyl ester alternate procedure

A solution of 4-amino-2-phenylbenzoyl methionine methyl ester (1.0 equivalent) in dilute aqueous HBr is treated with NaNO₂ (1.1 equivalents) to form the diazonium salt. The reaction is treated with CuBr (1.1 equivalents) and heated. When judged complete by TLC analysis, the mixture is extracted into ethyl acetate which is dried and evaporated. The title arylbromide is purified by chromatography on silica gel.

### Preparation 3

### Arylbromides of the formula B-Br

The anilines from Table 1 (B-NH₂) are reacted according to the procedures of Preparation 2 to provide the arylbromides listed in Table 2.

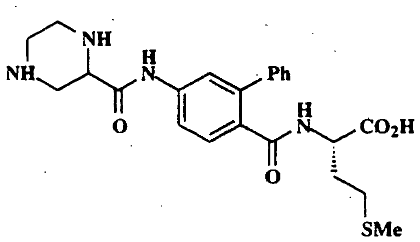

### Example 185

### [4-(piperidin-2-ylcarboxyamino)-2-phenylbenzoyl]methionine hydrochloride.

### Example 185A

### di-tert-butoxycarbonylpiperidine-2-carboxylic acid

Di-*tert*-butyl dicarbonate (15.5 g, 70.2 mmol) was added to a solution of piperazine-2-carboxylic acid (4.85 g, 23.4 mmol) and NaOH (98 mL of a 1 M aqueous solution, 98 mmol) in THF (100 mL). The cloudy mixture was stirred for 16 hours and then concentrated under reduced pressure to remove THF. The residue was saturated with solid NaHCO₃ and extracted with ether (2 x 30 mL). The aqueous layer was cooled to 0 °C and then adjusted to pH = 3 with 2 M aqueous HCl. A precipitate developed. The mixture was extracted with CH₂Cl₂ (3 x 75 mL), and the organic extracts were dried over MgSO₄, filtered, and concentrated under reduced pressure to provide 7.61 g (98%) of di-*tert-*butoxycarbonylpiperidine-2-carboxylic acid as a tan solid. ¹H NMR (CDCl₃) δ 1.45 (s, 18 H), 2.80-2.98 (br, I H), 3.04-3.36 (br comp. 2 H), 3.70-3.83 (br, 1 H), 3.94-4.05 (br, I H), 4.44-4.65 (br comp, 2 H), 4.80-4.95 (br, 1 H). LRMS (CI): 292. 331 (M+1)⁺, 348 (M+NH₄)⁺.

### Example 185B

### [4-(di-tert-butoxycarbonylpiperidint-2-ylcarboxyamino)-2-phenylbenzoyl]methionine methyl ester.

The desired compound was prepared by coupling di-*tert*-butoxycarbonylpiperidine-2-carboxylic acid with [4-amino-2-phenylbenzoyl]methionine methyl ester (compound 8) according to the procedure of Example 184A.

### Example 185C

### [4-(di-tert-butoxycarbonylpiperidin-2-ylcarboxyamino)-2-phenylbenzoyl]methionine.

Lithium hydroxide hydrate (0.411 g, 9.60 mmol) was added to a solution of [4-(di-*tert*-butoxycarbonylpiperidin-2-yl)carboxyamino-2-phenylmethionine methyl ester (*ca* 0.8 g, 1.20 mmol), prepared in Example 185B, in THF/H₂O (4:1, 12 mL). The solution was stirred for 20 hours and then treated with 1 M aqueous HCl (10 mL). The mixture was extracted with ethyl acetate (5 x 10 mL), and the organic extracts were rinsed with-1:1 brine/ I N HCl (10 mL), dried over Na₂SO₄, and concentrated under reduced pressure to provide [4-(di-tert-butoxycarbonylpiperidin-2-yl)carboxyamino-2-phenylmethionine (0.72 g) as a white foam (est. 89%). ¹H NMR (CD₃OD) δ 1.3-1.5 (br, 18 H), 1.7-1.9 (br comp, 2 H), 2.0 (br s, 3 H), 2.1-2.3 (br comp, 2 H), 2.9-4.8 (br comp, 8 H), 7.3-7.5 (br comp, 6 H), 7.5-7.6 (br m, 1 H), 7.6-7.7 (br m, 1 H). LRMS (CI): 657 (M+1)⁺, 457, 330.

### Example 185D

### [4-(piperidin-2-ylcarboxyamino)-2-phenylbenzoyl]methionine hydrochloride.

[4-(di-*tert*-butoxycarbonylpiperidin-2-ylcarboxyamino)-2-phenylbenzoyl]methionine (0.72 g, 1.07 mmol), prepared in Example 185C, was treated with HCl (9.6 mL of a 4 M solution in dioxane, 38.5 mmol) and the solution was stirred for 5 minutes, at which time a pink precipitate was observed. The mixture was treated with pentane (10 mL) and the precipitate was isolated by filtration to afford [4-(piperidin-2-yl)carboxyamino-2-phenylbenzoyl]methionine hydrochloride (0.448 g, 86%). ¹H NMR (CD₃OD) δ 1.73-1.88 (m, 1 H), 1.93-2.05 (comp, 4 H), 2.05-2.14 (m, I H), 2.14-2.26 (m, 1 H), 3.32-3.64 (comp, 5 H), 3.68-3.85 (comp, 2 H), 3.97 (dd, 1 H), 4.13 (dd, 1 H), 4.73 (dd, 1 H), 7.35-7.50 (comp, 5 H), 7.51-7.59 (m, 1 H), 7.74-7.80 (m, 1 H). LRMS (CI): 457 (M+1)⁺.

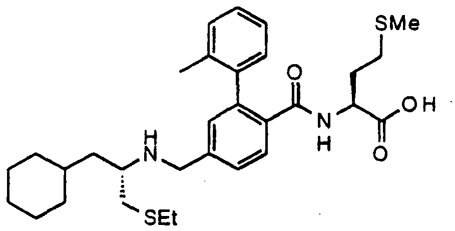

### Example 403

### [4-(1-ethylthio-3-cyclohexylprop-2-ylaminomethyl)-2-(2-methylphenyl)benzoyl] methionine.

The desired compound was prepared according to the method of Example 349A except substituting (S)-(+)-1-ethylthio-3-cyclohexyl-2-propylamine hydrochloride for (S)-(+)-2-amino-3-cyclohexyl-1-propanol hydrochloride. ¹H NMR (DMSO-d₆, 300 MHz) δ 8.02 (m, 1H), 7.50-7.38 (m, 2H), 7.22-7.05 (m, 4H), 4.21 (m, 1H), 3.88-3.78 (m, 2H), 2.74-2.60 (-m, 2H), 2.51 (s, 3H), 2.44 (q, *J*=7.5 Hz, 2H), 2.22-1.95 (m, 5H), 1.88-1.50 (m, 7H), 1.45-1.25 (m, 4H), 2.21-1.02 (m, 3H), 1.12 (t, *J*=7.5 Hz, 3H), 0.90-0.70 (m, 2H). MS (CI/NH₃) m/e: 557 (M+H)⁺ Anal calcd for C₃₁H₄₄N₂O₃S₂ • 1.15 H2O: C, 64.47; H, 8.08; N, 4.85. Found: C, 64.48; H, 7.84; N, 4.72.

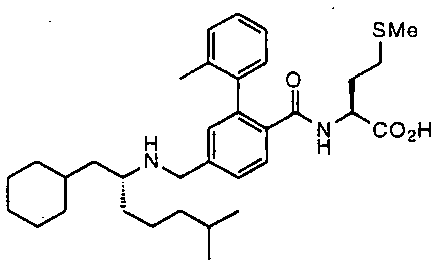

### Example 527

### N-[4-N-(1-Cyclohexyl-6-methylheptan-2-yl)aminomethyl-2-(2-methylphenyl)benzoyl]methioniine lithium salt

The desired compound was prepared according to the method of Example 158 ¹H NMR (300 MHz, DMSO) δ 0.80 (d, *J*=5 Hz, 3H), 0.82 (d, *J*=5 Hz, 3H), 1.02-1.40 (m, 12H), 1.40-1.65 (m, 12H), 1.75-1.83 (m, 1H), 1.92 (s, 3H), 1.99 (m, 1H), 2.16 (m, 1H), 2.43 (m, 1H), 3.60-3.77 (m, 3H), 6.86-6.95 (m. 1H), 7.08-7.22 (m, 5H), 7.35 (d, *J*=8.0 Hz, 1H), 7.47 (d, *J*=8.0 Hz, 1H). MS (APCI(+)) m/z 567 (M+H)⁺. Anal calcd for C₃₄H₄₉N₂O₃SLi•1.15H₂O: C, 66.99; H, 8.48; N, 4.60. Found: C, 67.03; H, 8.62; N, 4.49.

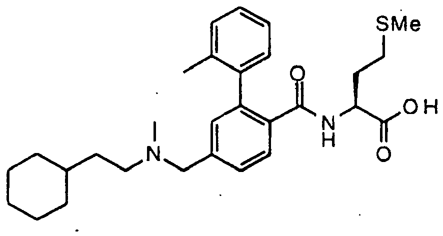

### Example 608

### N-[4-(N-(2-cyclohexylethyl)-N-methylaminomethyl)-2-(2-methylphenyl)benzoyl]methionine Lithium Salt

The desired compound was prepared according to the method of Example 158. MS (CI/NH₃) m/z: (M+H)⁺ 497; ¹H NMR (DMSO-d₆, 300 MHz) δ 7.49 (d, *J*=8 Hz, 1H), 7.32 (dd, *J*=8, 1Hz, 1H), 7.25-7.06 (m, 4H), 6.93 (d, *J*=6 Hz, 1H), 3.73-3.64 (m, 1H), 3.49 (s, 2H), 2.32 (t, *J*=7 Hz, 2H), 2.15 (m, 1H), 2.12 (s, 3H), 2.06-1.80 (m, 3H), 1.92 (s, 3H), 1.74-1.50 (m, 7H), 1.35-1.05 (m, 7H), 0.90-0.76 (m, 2H); Anal. Calcd for C₂₉H₃₉LiN₂O₃S·1.05 H₂O: C, 66.78; H, 7.94; N, 5.37. Found: C, 66.81; H, 7.75; N, 5.07.

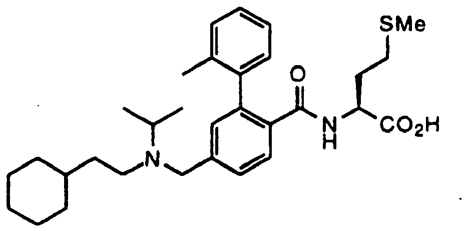

### Example 915

### N-[4-(N-(2-Cyclohexylethyl)-N-isopropylaminomethyl)-2-(2-methylphenyl)-benzoyl]methionine

The desired compound was prepared according to the method of Example 158. ¹H (300MHz), CDCl₃. δ) 7.80-7.60 (2H, m), 7.30-7.00 (5H, m), 6.50 (1H, d, *J*=8Hz), 4.38 (1H, m), 4.03 (2H, m), 3.67 (1H, m), 2.88 (2H, m), 2.20-2.00 (7H, m), 2.00 (3H, s), 1.80-1.40 (8H, m), 1.33 (6H, d, *J*=7Hz), 1.30-1.00 (3H, m), 1.00-0.80 (2H, m). *m*/*z* (ESI) 525 (MH⁺) Anal.calc. for C₃₁H₄₄N₂O₃S·0.50 H₂O C 69.76, H 8.50, N 5.25 Found C 69.90, H 8.26, N 5.57

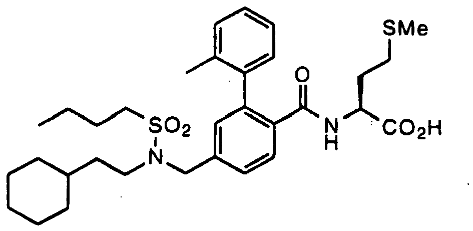

### Example 916

### N-[4-(N-Butanesulfonyl-N-(2-cyclohexylethyl)aminomethyl)-2-(2-methylphenyl)-benzoyl]methionine

The desired compound was prepared according to the method of Example 157. ¹H (300MHz, CDCl₃, δ) 7.99 (1H, m), 7.45 (1H, dd, *J*=9&2Hz), 7.40-7.10 (5H, m), 5.92 (1H, m), 4.56 (1H, m), 4.44 (2H, s), 3.20 (2H, m), 2.96 (2H, m), 2.20-2.05 (5H, m), 2.02 (3H, s), 2.00-1.70 (3H, m), 1.70-1.30 (10H, m), 1.30-1.00 (4H, m), 0.95 (3H, t, *J*=8Hz), 0.83 (2H, m). *m*/*z* (ESI) 603 (MH⁺) Anal.calc. for C₃₂H₄₆N₂O₅S₂·0.25 H₂O C 63.28, H 7.72, N 4.61 Found C 63.27, H 7.73, N 4.50

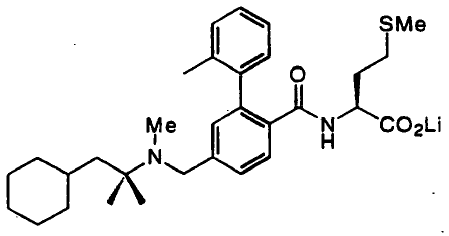

### Example 983

### N-[4-(N-Methyl-N-(1,1-dimethyl-2-cyclohexylethyl)aminomethyl)-2-(2-methylphenyl)-benzoyl]methionine lithium salt

The desired compound was prepared according to the method of Example 158 ¹H NMR (300 MHz, DMSO) δ 0.85-1.17 (m, 6H), 1.03 (brs, 6H), 1.30-1.35 (m, 2H), 1.51-1.77 (m, 10H), 1.93 (s, 3H), 1.97-2.18 (m, 3H), 2.02 (s, 3H), 3.56 (brs, 2H), 3.59-3.74 (m, 1H), 6.92 (d, J=5.0 Hz, 1H), 7.11-7.23 (m, 5H), 7.34 (d, J=7.7 Hz, 1H), 7.46 (d, J=7.8 Hz, 1H). MS (APCI(+)) *m*/*z* 525 (M+H); Analysis calc'd for C₃₁H₄₃LiN₂O₃S+0.80H₂O: C, 68.31; H, 8.25; N, 5.14; found: C, 68.29; H, 8.23; N, 5.04.

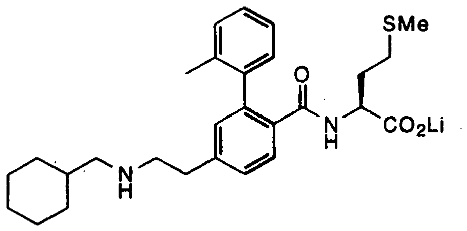

### Example 1021

### N-[4-(N-cyclohexylmethylamino-ethyl)-2-(2-methylphenyl)benzoyl]methionine, lithium salt

N-[4-(N-(cyclohexylmethyl)aminoethyl)-2-(2-methylphenyl)benzoyl methionine methyl ester (84 mg, 0.17 mmol) was treated with LiOH (1 M, 85 µL) in methanol to provide the title compound.
MS m/e 481 (M-H)-.

¹H NMR (d₆-DMSO, 300 MHz) δ 0.83 (m, 2H), 1.15 (m, 4H), 1.36 (m, 1H), 1.62 (m, 9H), 1.98 (m, 10H), 3.7 (m, 2H), 4.27 (m, 1H), 6.90 (m, 1H), 7.00 (m, 1H), 7.1-7.3 (m, 4H), 7.44 (m, 1H), 8.24 (m, 1H).

### N-[4-(N-(cyclohexylmethyl)aminoethyl)-2-(2-methylphenyl)benzoyl methionine methyl ester

Methyl 4-(N-(cyclohexylmethyl)aminoethyl)-2-(2-methylphenyl)benzoate hydrochloride (1.33 g, 3.31 mmol) was treated with sat. LiOH (1.3 mL, 6.95 mmol) in 50 mL methanol at 60 °C until no starting material remained by tlc. The solution was evaporated to dryness and treated with Met-OMe·HCl (0.99 g, 4.96 mmol), EDAC (1.26 g, 6.6 mmol), HOBt (1.5 g, 9.9 mmol), and TEA (to pH 6~7) in 25 mL DMF. Standard aqueous workup followed by flash chromatography (100 % EtOAc) provided 1.5 g of the title compound.
MS m/e 497 (M-H)-.
¹H NMR (CDCl₃, 300 MHz) δ 0.88 (m, 2H), 1.2 (m, 4H), 1.6 (m, 8H), 2.1 (m, 8H), 2.47 (m, 2H), 2.9 (m, 4H), 3.68 (s, 3H), 4.63 (m, 1H), 5.89 (d, 1H, J = 7 Hz), 7.04 (s, 1H), 7.19 (m, 1H), 7.3 (m, 4H), 7.91 (m, 1H).

### Methyl 4-(N-(cyclohexylmethyl)aminoethyl)-2-(2-methylphenyl)benzoate

Methyl 4-(propan-3-al)-2-(2-methylphenyl)benzoate (5.0 g, 18.6 mmol) and cyclohexylmethylamine (2.32 g, 10.5 mmol) were dissolved in 250 mL I % acetic acid in methanol. After 10 minutes, sodium cyanoborohydride (1.76 g, 28 mmol) was added. The mixture stirred overnight at room temperature before evaporating to dryness. The residue was dissolved in ether and washed with 5 % NaHCO₃, water, and brine, dried over Na₂SO₄, and treated with anh. HCl. The oily product was crystalized from methanol and ether.
MS m/e 366 (M+H)⁺.
¹H NMR (CDCl₃, 300 MHz) δ 0.88 (m, 2H), 1.2 (m, 4H), 1.6 (m, 6H), 2.06 (s, 3H), 2.48 (d, 2H, J = 7 Hz), 2.92 (s, 4H), 3.61 (s, 3H), 7.06 (m, 1H), 7.23 (m, 5H), 7.92 (m, 1H).

### Methyl 4-(propan-3-al)-2-(2-methylphenyl)benzoate

Methyl 4-(prop-2-enyl)-2-(2-methylphenyl)benzoate (5.23 g, 19.6 mmol), osmium tetroxide (0.02 mmol/mL t-BuOH, 29.5 mL), and sodium periodate (10.5 g, 49.1 mmol) were combined in 200 mL acetone with 50 mL water. After stirring at ambient temperature for 1 hour, the mixture was diluted with water and extracted with EtOAc. The combined organic extracts were washed with brine and dried over Na₂SO₄ to give the desired product which was used directly in the next step.
MS m/e 286 (M+NH₄)⁺.
¹H NMR (CDCl₃, 300 MHz) δ 2.06 (m, 3H), 3.61 (s, 3H), 3.8 (m, 2H), 7.1 (m, 1H), 7.25 (m, 5H), 7.95 (m, 1H), 9.80 (m, 1H).

### Methyl 4-(prop-2-enyl)-2-(2-methylphenyl)benzoate

Methyl 4-iodo-2-(2-methylphenyl)benzoate (10.0 g, 28.4 mmol), allyltributyl tin (11.3 g, 34.1 mmol), and dichlorobis(triphenylphosphine)palladium (II) (1.0 g, 1.42 mmol) were combined in 50 mL toluene and 20 mL NMP and heated at 125°C for 18 hours. The reaction was diluted with EtOAc, washed with water and brine, dried over Na₂SO₄, and chromatographed (5 % EtOAc in hexanes) to provide the title compound in 74 % yield.
MS m/e 284 (M+NH₄)⁺.
¹H NMR (CDCl₃, 300 MHz) δ 2.06 (s, 3H), 3.45 (d, 2H, J = 7 Hz), 3.61 (s, 3H), 5.1 (m, 2H), 5.97 (m, 1H), 7.08 (m, 1H), 7.23 (m, 5H), 7.94 (m, 1H).

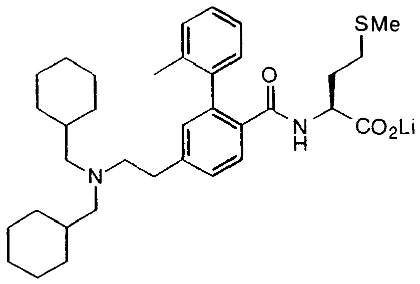

### Example 1022

### N-[4-(N,N-di-(cyclohexylmethyl)aminoethyl)-2-(2-methylphenyl)benzoyl]methionine, lithium salt

N-[4-(N-(cyclohexylmethyl)aminoethyl)-2-(2-methylphenyl)benzoyl methionine methyl ester (300 mg, 0.60 mmol) and cyclohexylcarboxaldehyde (140 mg, 1.21 mmol) were dissolved in 1 % acetic acid in methanol (5 mL) and treated with sodium cyanoborohydride (76 mg, 1.21 mmol). Standard workup followed by flash chromatography (20 % ethyl acetate in hexane) provided 320 mg which was subsequently saponified with LiOH to the title compound.
MS m/e 577 (M-H)-.
¹H NMR (d₆-DMSO, 300 MHz) δ 0.75 (m, 4H), 1.10 (m, 8H), 1.30 (m, 2H), 1.61 (m, 9H), 2.0 (m, 10H), 2.6 (m, 2H), 2.7 (m, 2H), 3.3 (m, 1H), 3.68 (m, 1H), 6.90 (m, 2H), 7.1 (m, 5H), 7.44 (m, 1H).

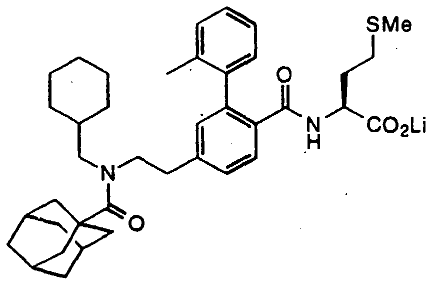

### Example 1024

### N-[4-(N-cyclohexylmethyl-N-1-adamantanoylaminoethyl)-2-(2-methylphenyl)benzoyl]methionine, lithium salt

This compound was prepared in a manner analogous to Example 1023 using 1-adamantanecarbonyl chlroide.
MS m/e 643 (M-H)-.
¹H NMR (d₆-DMSO, 300 MHz) δ 0.87 (m, 8H). 1.15 (m, 4H), 1.6 (m, 14H), 1.9 (m, 12H), 2.85 (m, 1H), 3.18 (m, 2H), 3.6 (m, 2H), 6.91 (m, 1H), 7.02 (m, 1H), 7.2 (m, 5H), 7.48 (m, 1H).

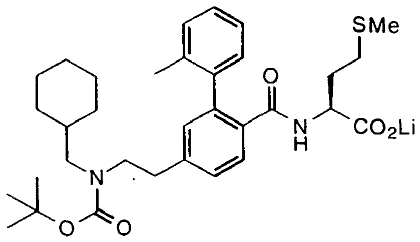

### Example 1025

### N-[4-(N-cyclohexylmethyl-N-t-butoxycarbonylaminoethyl)-2-(2-methylphenyl)benzoyl]methionine, lithium salt

This compound was prepared in a manner analogous to Example 1023 using di-t-butyldicarbonate.
MS m/e 581 (M-H)-.
¹H NMR (d₆-DMSO, 300 MHz) δ 0.83 (m, 2H), 1.15 (m, 4H), 1.38 (s, 9H), 1.6 (m, 9H), 1.95 (m, 6H), 2.18 (m, 2H), 2.8 (m, 4H), 3.7 (m, 1H), 6.9 (m, 1H), 7.0 (m, 1H), 7.2 (m, 5H), 7.45 (m, 1H).

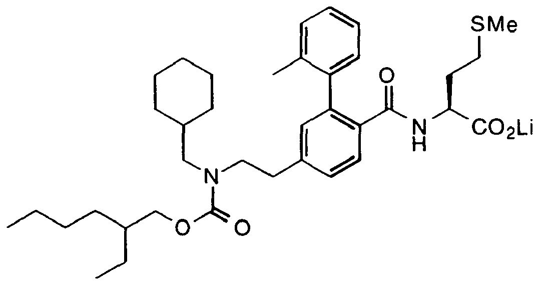

### Example 1026

### N-[4-(N-cyclohexylmethyl-N-2-ethylhexyloxycarbonylaminoethyl)-2-(2-methylphenyl)benzoyl]methionine, lithium salt

This compound was prepared in a manner analogous to Example 1023 using 2-ethylhexyl chloroformate.
MS m/e 637 (M-H)-.
¹H NMR (d₆-DMSO, 300 MHz) δ 0.83 (m, 4H), 1.15 (m, 4H), 1.23 (m, 9H), 1.6 (m, 9H), 1.95 (m, 8H), 2.83 (m, 2H), 3.0 (m, 2H), 3.5 (m, 3H), 3.6 (m, 1H), 3.89 (m, 2H), 4.29 (m, 1H), 6.9 (m, 1H), 7.0 (m, 1H), 7.2 (m, 5H), 7.45 (m, 1H).

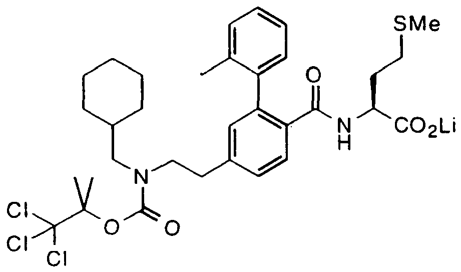

### Example 1027

### N-[4-(N-cyclohexylmethyl-N-2.2.2-trichloroethoxycarbonylaminoethyl)-2-(2-methylphenyl)benzoyl]methionine, lithium salt

This compound was prepared in a manner analogous to Example 1023.
MS m/e 683 (M-H)-.
¹H NMR (d₆-DMSO, 300 MHz) δ 0.84 (m, 2H), 1.17 (m, 4H), 1.6 (m, 5H), 1.9 (m, 14H), 2.9 (m, 3H), 3.03 (m, 1H), 3.5 (m, 3H), 3.6 (m, 1H), 4.28 (m, 1H), 6.9 (m, 1H), 7.0 (m, 2H), 7.2 (m, 5H), 7.45 (m, 1H).

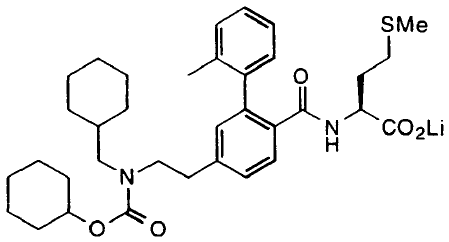

### Example 1028

### N-[4-(N-cyclohexylmethyl-N-cyclohexyloxycarbonylalinoethyl]-2-(2-methylphenyl)benzoyl]methionine, lithium salt

This compound was prepared in a manner analogous to Example 1023.
MS m/e 607 (M-H)-.
¹H NMR (d₆-DMSO, 300 MHz) δ 0.84 (m, 4H), 1.17 (m, 4H), 1.3 (m, 6H), 1.6 (m, 10H), 1.95 (m, 8H), 2.17 (m, 1H), 2.9 (m, 4H), 3.6 (m, 1H), 4.53 (m, 1H), 6.9 (m, 1H), 7.0 (m, 1H), 7.2 (m, 5H), 7.47 (m, 1H).

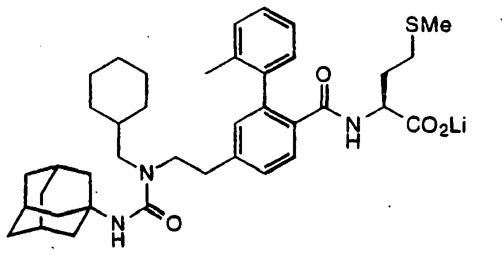

### Example 1032

### N-[4-(N-cyclohexylmethyl-N-adamant-1-aminocarbonylaminoethyl)-2-(2-methylphenyl)benzoyl]methionine, lithium salt

This compound was prepared in a manner analogous to Example 1023 using adamantyl isocyanate.
MS m/e 658 (M-H)-.
¹H NMR (d₆-DMSO, 300 MHz) δ 0.83 (m, 6H), 1.13 (m, 6H), 1.6 (m, 13H), 1.95 (m, 12H), 2.18 (m, 1H), 2.79 (m, 2H), 2.91 (m, 2H), 3.65 (m, 2H), 6.9 (m, 1H), 7.0 (m, 1H), 7.2 (m, 5H), 7.46 (m, 1H).

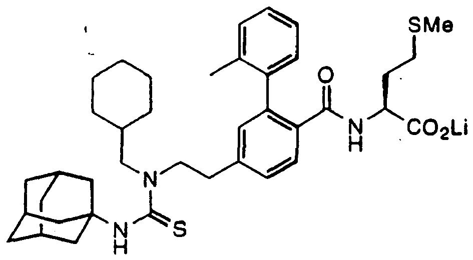

### Example 1033

### N-[4-(N-cyclohexylmethyl-N-adamant-1-aminothiocarbonylaminoethyl)-2-(2-methylphenyl)benzoyl]methionine, lithium salt

This compound was prepared in a manner analogous to Example 1023 using adamantyl isothiocyanate.
MS m/e 674 (M-H)-.
¹H NMR (d₆-DMSO, 300 MHz) δ 0.85 (m, 6H), 1.15 (m, 6H), 1.6 (m, 13H), 2.0 (m, 12H), 2.2 (m, 1H), 2.74 (m, 2H), 2.91 (m, 2H), 3.62 (m, 2H), 6.9-7.5 (m, 8H).

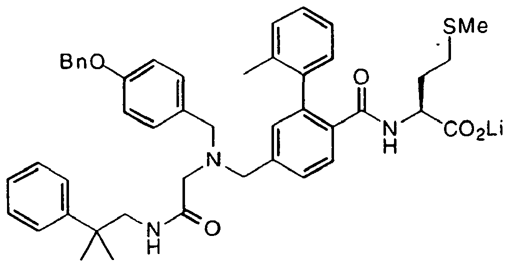

### Example 1048

### N-[4-(N-(4-Benzyloxybenzyl)-N-(N-2-methyl-2-phenylpropylacetamido)aminomethyl)-2-(2-methylphenyl)benzoyl]methionine, lithium salt

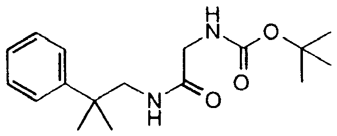

### Example 1048A

### N-(2-Methyl-2-phenylpropyl)-N-tert-butoxycarbonyl-2-aminoacetamide

To a slurry of NaH (10g of a 60% dispersion in mineral oil) in dry THF (300mL) was added benzylcyanide (10g) by means of a dropping funnel. Cautious addition of methyl iodide (13mL) caused rapid gas evolution and an increase in temperature which was moderated with an ice bath. After stirring at ambient temperature for 12h, the reaction was quenched cautiously with water (100mL). The mixture was diluted with ether (500mL) and the layers were separated. The ether layer was washed with water (100mL) containing a small amount of Na₂SO₃ to eliminate the iodine color, then washed with brine (50mL). The organic solution was dried (MgSO₄), filtered and concentrated to afford an oil. This material was added neat to a solution of 1M LiAlH₄ (85mL, THF) in ether (100mL). If necessary, the reduction was initiated after a small amount of starting material was added by warming with a heat gun. The starting material was then added at a rate which maintained a gentle reflux. After addition was complete, the reaction was stirred without heating or cooling for 1h. The reaction was cautiously quenched with vigorous stirring by the addition of water (3.2mL), 15%NaOH (3.2mL), and more water (10mL). The suspension was filtered through celite, which was rinsed with ether. The filtrate was concentrated to give an oil (ca. 20g) which contained mineral oil from the sodium hydride dispersion. A portion of this material (3.3g) was dissolved in DMF (67mL) along with N-(tert-butoxycarbonyl)glycine (3.5g), followed by addition of N-methylmorpholine (3.3mL), 1-hydroxybenzotriazole (3.0g), and 1-ethyl-3-(3-dimethylaminopropyl)carbodiimide (5.0g). After stirring at ambient temperature for 15h, the reaction was poured into ether (500mL), washed with water

(2X100mL), 1M HCl (2X100mL), saturated NaHCO₃ (2X50mL), and brine (10mL). The organic solution was dried (MgSO₄), filtered and concentrated to afford a residue which partly solidified. The residue was triturated with hexane, and filtered to give 4.5g of the title compound. MS(DCI/NH₃) 307 (M+H)⁺.

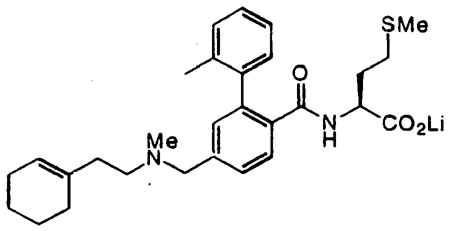

### Example 1056

### N-[4-(N-(2-Cyclohexenylethyl)-N-methylaminomethyl)-2-(2-methylphenyl)benzoyl]methionine, lithium salt

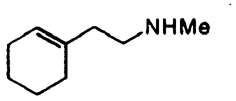

### Example 1056A

### N-Methyl-2-(1-cyclohexenyl)ethylamine

To a solution of 2-(1-cyclohexenyl)ethylamine (4.0g) in 1,4-dioxane (40mL) was added di-tert-butyldicarbonate (7.7g). After gas evolution ceased (=2h) the reaction was concentrated. A portion of the residue (2g) was dissolved in THF (10mL) followed by addition of LiAlH₄ (10mL, 1M THF), which caused an exotherm. After 3h, more LiAlH₄ solution was added (4mL), and the reaction was warmed to reflux. After 1h, the reaction was cooled, and quenched cautiously with vigorous stirring by the addition of water (0.57mL), 1M NaOH (0.6mL), and more water (1.5mL). The suspension was filtered through celite, which was washed with ether. The organic solution was concentrated to give the desired product as a volatile oil (0.8g).
¹H NMR (300 MHz, CDCl₃) δ 1.52-1.67 (m, 4H), 1.89-2.04 (m, 4H), 2.14 (brt, J=7 Hz, 2H), 2.42 (s, 3H), 2.63 (t, J=7 Hz, 2H), 5.45 (m, 1H).

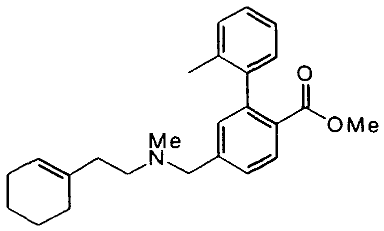

### Example 1056B

### 4-(N-(2-Cyclohexenylethyl)-N-methylaminomethyl)-2-(2-methylphenyl)benzoic acid, Methyl Ester

The title compound was prepared from N-methyl-2-(1-cyclohexenyl)ethylamine according to the procedure in example 608B.
MS (DCI/NH₃) 378 (M+H)⁺.

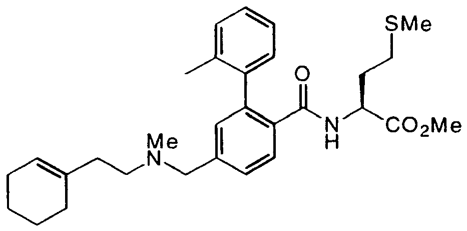

### Example 1056C

### N-[4-(N-(2-Cyclohexenylethyl-N-methylaminomethyl)-2-(2-methylphenyl)benzoyl]methionine, Methyl Ester

The title compound was prepared from 4-(N-(2-cyclohexenylethyl)-N-methylaminomethyl)-2-(2-methylphenyl)benzoic acid methyl ester according to the procedure in examples 608C and D. MS(APCI(+)) 509 (M+H)⁺. MS(APCI(-)) 543 (M+Cl)⁻.

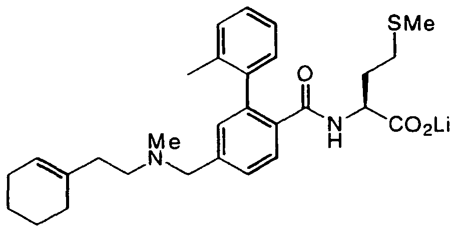

### Example 1056D

### N-[4-(N-(2-Cyclohexenylethyl)-N-methylaminomethyl)-2-(2-methylphenyl)benzol]methionine, lithium salt

N-[4-(N-(2-Cyclohexenylethyl)-N-methylaminomethyl)-2-(2-methylphenyl)benzoyl]methionine methyl ester was converted into the title compound by the procedure in example 608E, and was isolated as a white powder.
¹H NMR (300 MHz, DMSO) δ 1.38-1.75 (m, 4H), 1.80-2.13 (m, 13H), 1.91 (s, 3H), 2.14 (s, 3H), 2.36-2.45 (m, 2H), 3.50 (s, 2H), 3.56-3.67 (brs, 1H), 5.32-5.36 (m, 1H), 6.88-6.92 (m, 1H), 7.05-7.23 (m, 5H), 7.32 (d, J=8.1 Hz, 1H), 7.48 (d, J=8.1 Hz, 1H). MS (APCI(-)) m/e 493 (M-H); Analysis calc'd for C₂₉H₃₇LiN₂O₃S•1.15H₂O: C, 66.81; H, 7.60; N, 5.37; found: C, 66.86; H, 7.34; N, 5.19.

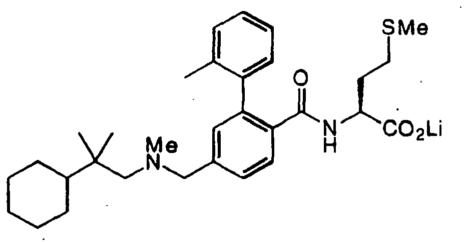

### Example 1069

### N-[4-(N-(2-Cyclohexyl)-2-methylpropyl)-N-methylaminomethyl)-2-(2-methylphenyl)benzoyl]methionine, lithium salt

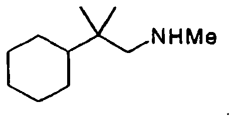

### Example 1069A

### N-Methyl-2-cyclohexyl-2-methylpropylamine

Treatment of 2-phenyl-2-methylpropylamine (example 1048A, 5g) with di-tert-butyldicarbonate according to example 1056A afforded N-tert-butoxycarbonyl-2-phenyl-2-methylpropylamine (10g crude) as a colorless oil. To portion of this material (5g) in methanol (100mL) was added platinum oxide (1g), and the reaction was shaken under hydrogen gas (4atm) for 24h. The reaction was concentrated, diluted with water (100mL), and extracted with chloroform (3X50mL). The organic extracts were washed with brine (20mL), dried (MgSO₄), filtered and concentrated. The residue was purified by silica gel chromatography eluting with 10% EtOAc/hexane to afford a colorless oil (1.0g). This material was reduced with LiAlH₄ according to the procedure described in example 1056A to afford the title compound (0.8g), as a colorless oil.
¹H NMR (300 MHz, CDCl₃) δ 0.83 (s, 6H), 0.87-1.29 (m, 6H), 1.60-1.82 (m, 5H), 2.36 (s, 2H), 2.42 (s, 3H).
MS (APCI(+)) m/e 170 (M+H)⁺.

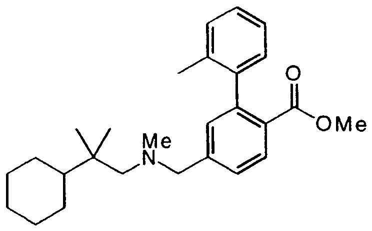

### Example 1069B

### 4-(N-(2-Cyclohexyl-2-methylpropyl)-N-methylaminomethyl)-2-(2-methylphenyl)benzoic acid, Methyl Ester

The title compound was prepared according to the procedure in example 608B, subsitiuting N-methyl-2-cyclohexyl-2-methylpropylamine for N-methylcyclohexylethylamine, and was isolated as a colorless oil. MS(ESI(+)) m/e 408
(M+H)⁺.

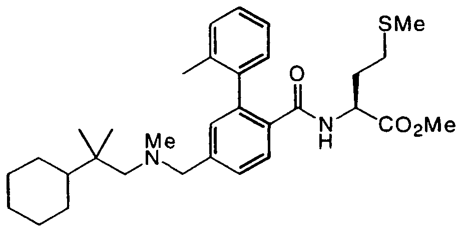

### Example 1069C

### N-[4-(N-(2-Cyclohexyl-2-methylpropyl)-N-methylaminomethyl)-2-(2-methylphenyl)benzoyl]methionine, Methyl Ester

The title compound was prepared from 4-(N-(2-cyclohexyl-2-methylpropyl)-N-methylaminomethyl)-2-(2-methylphenyl)benzoic acid methyl ester according to the procedures described in examples 608C, and D, and was isolated as a colorless oil. MS(ESI(+)) m/e 539 (M+H)⁺. MS(ESI(-)) m/e 537 (M-H)-.

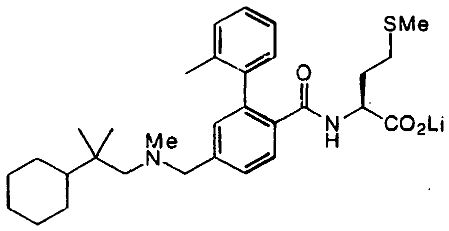

### Example 1069D

### N-[4-(N-(2-Cyclohexyl-2-methylpropyl)-N-methylaminomethyl)-2-(2-methylphenyl)benzoyl]methionine, lithium salt

The title compound was prepared from N-[4-(N-(2-cyclohexyl-2-methylpropyl)-N-methylaminomethyl)-2-(2-methylphenyl)benzoyl]methionine methyl ester according to the procedure in example 608E, and was isolated as a white powder.
¹H NMR (300 MHz, DMSO) δ 0.79 (s, 6H), 0.80-1.27 (m, 5H), 1.50-1.74 (m, 6H), 1.75-2.95 (m, 7H), 1.92 (s, 3H), 2.19 (s, 3H), 2.24 (s, 2H), 3.56 (s, 2H), 3.62-3.72 (m, 1H), 6.92 (d, J=6 Hz, 1H), 7.08-7.25 (m, 5H); 7.36 (d, J=7.8 Hz, 1H), 7.49 (d, J=7.8 Hz, 1H).
MS (ESI(-)) m/e 523 (M-H); Analysis calc'd for C₃₁H₄₃LiN₂O₃S•1.3H₂O: C, 67.70; H, 8.29; N, 5.06; found: C, 67.15; H, 8.08; N, 4.97.
MS m/z 385 (M⁺ - 1, 100).

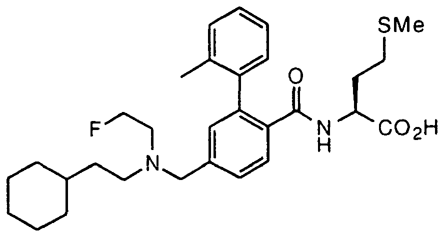

### Example 1094

### N-[4-(N-(2-cyclohexylethyl)-N-2-fluoroethylaminomethyl)-2-(2-methylphenyl)benzoyl]methionine

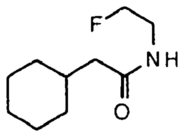

### Example 1094A

### N-(2-Fluoroethyl)-2-cyclohexylacetamide

Following the procedure of example 1178E, 2-fluoroethylamine•HCl (1.00 g, 10.00 mmol) provided 1.58 g (84%) of the title compound.
MS (DCI, NH₃): 188 (MH⁺).

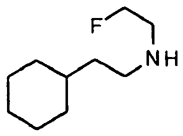

### Example 1094B

### N-(2-Fluoroethyl)-N-2-cyclohexylethylamine

Following the procedure of example 1178F, example 1094A (1.54 g, 8.2 mmol) provided 1.30 g (92%) of the title compound.
MS (DCI, NH₃): 172 (MH⁺).

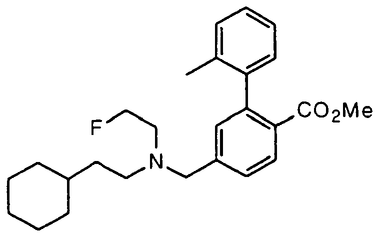

### Example 1094C

### N-[4-(N-(2-cyclohexylethyl)-N-2-fluoroethylaminomethyl)-2-(2-methyl)-phenyl)benzoic acid methyl ester

Following the procedure of example 1178G and substituting potassium phosphate for diisopropylethylamine, and heating at 60°C for 60 hours, example 1094B (188 mg, 1.10 mmol) provided 288 mg (70%) of the title compound.
MS (ESI +): 410 (M + NH₄⁺ -F-).

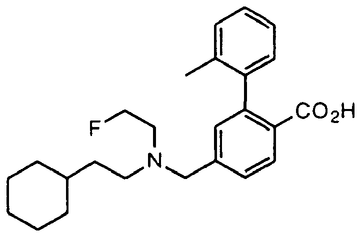

### Example 1094D

### N-[4-(N-(2-cyclohexylethyl)-N-2-fluoroethylaminomethyl)-2-(2-methylphenyl)benzoic acid

Following the procedure of example 1178H, example 1094C (0.28 g, 0.68 mmol) provided 0.25 g (93%) of the title compound.
MS (DCI, NH₃): 398 (MH⁺).

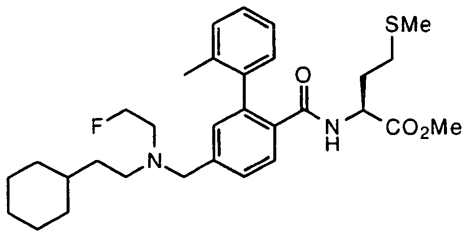

### Example 1094E

### N-[4-(N-(2-cyclohexylethyl)-N-2-fluoroethylaminomethyl)-2-(2-methylphenyl)benzoyllmethionine, methyl ester

Following the procedure of example 1178 I, example 1094D (245 mg, 0.62 mmol) provided 257 mg (77%) of the title compound. MS: (ESI+): 541 (MH)⁺: (ESI-); 539 (M-H).

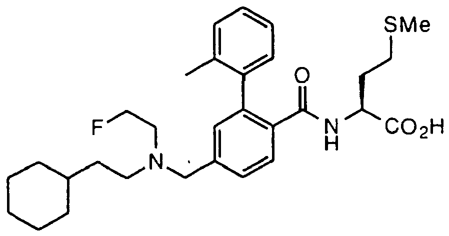

### Example 1094F

### N-[4-(N-(2-cyclohexylethyl)-N-2-fluoroethylaminomethyl)-2-(2-methylphenyl)benzoyl]methionine

Following the procedure of example 1104D, example 1094E (250 mg, 0.46 mmol) provided 240 mg of the title compound.
¹H NMR (δ ,CDCl₃): 7.75 (2H), 7.0-7.4 (4H), 6.4 (1H), 3.8-4.6 (9H), 2.9-3.3 (4H), 0.8-2.3 (21H). MS: (ESI+): 527 (MH)⁺: (ESI-); 525 (M-H). Calc'd for C₃₀H₄₁FN₂O₃S•0.90H₂O: C 66.12 H 7.92 N 5.14; Found: C 66.13 H 7.77 N 4.86.

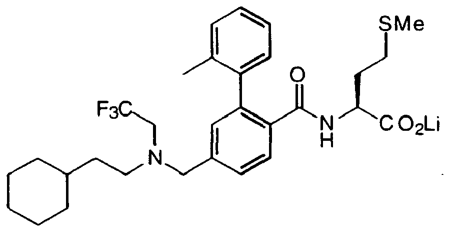

### Example 1103

### N-[4-(N-(2-cyclohexylethyl)-N-2.2.2-trifluoroethylaminomethyl)-2-(2-methylphenyl)benzoyl]methionine, lithium salt

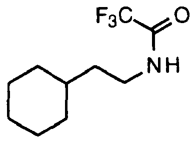

### Example 1103A

### N-trifluoroacetyl-2-cyclohexylethyl amide

Cyclohexylethyamine (1.27 g, 10 mmol) was dissolved in 10 mL of methylene chloride and pyridine (1.8 mL, 15.0 mol) was added and the mixture cooled to -10° C in an ice/acetone bath. The solution was treated with trifluoroacetic anhydride (1.7 mL, 12.0 mmol) in 5 mL of methylene chloride dropwise. After stirring for 2 hours at 0°C the mixture was diluted with 100 mL of ether and extracted with water, 1M aqueous phosphoric acid and satureaed aqueous sodium bicarbonate, dried, filtered and concentrated to give a white solid (2.07g. 92%).
MS (DCI, NH₃): 241 (M+NH₄)⁺.

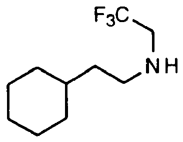

### Example 1103B

### N-2-trifluoroethyl-2-cyclohexylethyl amine

A solution of lithium aluminum hdydride (9 mL of a 1M solution in THF. 9 mmol) was added to a solution of example 1103A (0.67 g, 3.0 mmol) and the mixture was heated to reflux for 2 hours and then cooled to room temperature. The reaction was quenched by the same procedure as example 1178F to provide 0.58 g (92%) of the title compound.
MS (DCI, NH₃): 228 (M+NH₄)⁺.

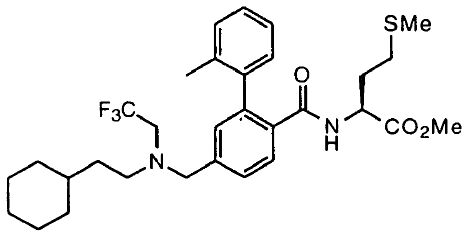

### Example 1103C

### N-[4-(N-(2-cyclohexylethyl)-N-2,2,2-trifluoroethylaminomethyl)-2-(2-methylphenyl)benzoyl]methionine methyl ester

A solution of example 1103B (210 mg, 1.0 mmol) and the aldehyde from example 403G (192 mg, 0.5 mmol) in 3 mL of 1,2 dichoroethane was treated with acetic acid (0.14 mL, 2.5 mmol) and the mixture stirred for 10 minutes. The mixture was treated with sodium triacetoxyborohydride (213 mg, 1.0 mmol) and the mixture stirred overnight. The work-up was the same as that of example 1134E. The crude product was purified by chromatography on silica gel (20 g, 20% ethyl acetate/hexanes) to provide 96 mg (33%) of the title compound.
¹H NMR (300 MHz., CDCl₃): δ 7.91, dd, 1H; 7.42, dd, 1H; 7.18 - 7.36, m, 4H; 7.15, bs, 1H; 5.88, bd, 1H' 4.63, m, 1H; 3.83, s, 2H; 3.65, s, 3H; 3.09, q, 2H; 2.64, t, 2H; 2.18, s, 1.5 H (o-tolyl); 2.07, s, 1.5H (o-tolyl), 2.05, m, 1H; 2.03, s, 1.5H (MeS); 2.01, s, 1.5H (MeS); 1.87, m, 1H; 1.61, bm, 6H; 1.35, m, 2H; 1.20, m 2H; 1.14, m, 2H; 0.85, m, 2H. MS (ESI+): 579 (MH+): (ESI-): 577 (M-H).

Prepared according to the procedure of example 1178J.
¹H NMR (300 MHz., dmso d6): δ 7.52, d, 1H; 7.35, d, 1H; 7.23, m, 3H; 7.12, m, 3H; 6.91, d, 1H; 3.81, s, 2H; 3.66, m, 1H; 3.38, q, 2H; 2.56, t, 2H; 2.06, m, 1H; 2.00, bs, 3H; 1.92, s, 3H; 1.58, m, 7H; 1.00 - 1,38, m, 6H; 0.80, m, 2H.
MS (ESI+): 587; 571; 565 (MH+): (ESI-): 563 (M-H). Calc'd for C₃₀H₃₈LiN₂O₃S•1.75 H₂O; C 59.84; H 6.95; N 4.65; Found: C 59.86; H 6.57; N 4.45.

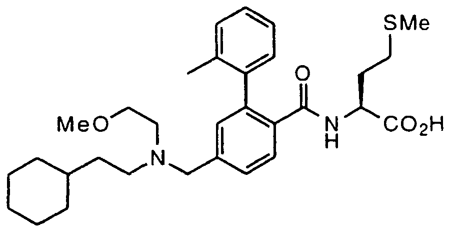

### Example 1104

### N-[4-(N-(2-cyclohexylethyl)-N-2-methoxyethylaminomethyl)-2-(2-methylphenyl)benzoyl]methionine

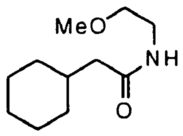

### Example 1104A

### N-(2-methoxyethyl)-2-cyclohexylacetamide

The acid chloride from example 1178E (1.60 g, 10 mmol) in 10 mL of methylene chloride was added dropwise to a cold (0°C) solution of 2-methoxyethylamine (1.3 mL, 15 mmol) and pyridine (1.9 mL, 22 mmol) in 10 mL of methylene chloride and the mixture was stirred overnight. The mixture was diluted with ethyl ether and washed with water, 1M aqueous phosphoric acid , 2M aqueous sodium carbonate and brine, dried, filtered and concentrated to provide 1.70 g (85%) of the title compound as a white solid.
¹H NMR (300 MHz., CDCl₃): δ 5.89, bs, 1H; 3.46, m, 4H; 3.37, s, 3H; 2.05, d, 2H; 1.79, m, 1H; 1.70, bm, 6H; 1.24, m, 2H; 1.17, m, 1H; 0.95, m, 2H.
MS (DCI, NH₃): 200 (MH⁺).

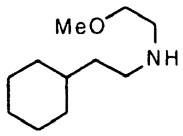

### Example 1104B

### N-(2-methoxyethyl)-N-2-cyclohexylethylamine

Using the procedure of example 1178F, example 1104A (1.70 g, 8.54 mmol) provided the title compound (1.56 g, 100%).
MS (DCI, NH₃): 186 (MH⁺).

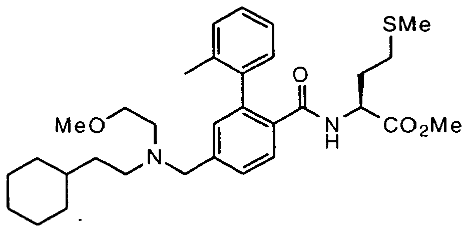

### Example 1104C

### N-[4-(N-(2-cyclohexylethyl)-N-2-methoxyethylaminomethyl)-2-(2-methylphenyl)benzoyllmethionine, methyl ester

Using the procedure of example 1103C, example 1104B (186 mg, 1.0 mmol) and example 403G (192 mg, 0.5 mmol) were combined to provide 78 mg (28%) of the title compound.
¹H NMR (300 MHz., CDCl₃): δ 7.91, dd, 1H; 7.42, dd, 1H; 7.18 - 7.37, m, 4H; 7.17, bs, 1H; 5.89, bd, 1H; 4.64, m, 1H; 3.68, s, 2H; 3.66, s, 3H; 3.45, t, 2H; 3.31, s, 3H; 2.66, t, 2H; 2.50, t, 2H; 2.19, s, 1.5H (o-tolyl); 2.07, s, 1.5H (o-tolyl); 2.05, m, 1H; 2.03, s, 1.5H (SMe); 2.01, s, 1.5H (SMe); 1.85, m, 1H; 1.63, bm, 6H; 1.34, m, 2H; 1.06 - 1.29, m, 4H; 0.88, m, 2H.
MS (ESI+): 555 (MH+): (ESI-): 553 (M-H).

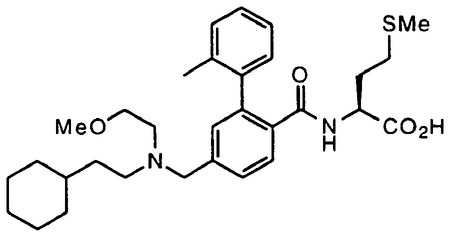

### Example 1104D

### N-[4-(N-(2-cyclohexylethyl)-N-2-methoxyethylaminomethyl)-2-(2-methylphenyl)benzoyl]methionine

A solution of example 1104C (73 mg, 0.13 mmol) in 2 mL of 3:1 THF/methanol was cooled in an ice bath and treated with lithium hydroxide (0.26 mL of a 1M aqueous solution, 0.26 mmol) and the mixture stirred overnight and then concentrated. The solid was diluted with water and the pH adjusted to 4.5 with 1M aqueous phosphoric acid and then extracted with 3 portions of ethyl acetate. The combined organic fractions were washed with brine, dried filtered and concetrated. The residue was lyophilized to provide 70 mg of the title compound.
¹H NMR (300 MHz., CD₃OD): δ 7.74, d, 1H; 7.58, d, 1H; 7.37, m, 1H; 7.10 - 7.31, m, 4H; 4.50, m, 3H; 3.66, t, 2H; 3.37, s, 3H; 3.22, t, 2H; 3.04, m, 2H; 2.22, bs, 1H; 2.10, m, 3H; 1.97, s, 3H; 1.90, m, 2H; 1.53 - 1.77, m, 8H; 1.14 - 1.38, m, 4H; 0.96, m, 2H. MS (ESI+): 541 (MH+); (ESI-): 539 (M-H). Calc'd for C₃₁H₄₄N₂O₄S•0.85 H₂O; C 66.96; H 8.28; N 5.04; Found: C 66.97; H 8.34; N 4.87.

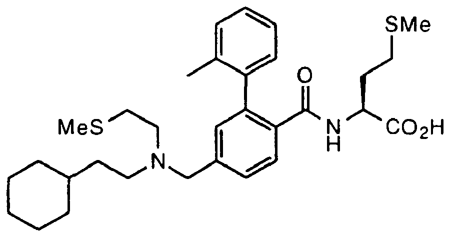

### Example 1105

### N-[4-(N-(2-cyclohexylethyl)-N-2-methylthioethylaminomethyl)-2-(2-methylphenyl)benzoyl]methionine

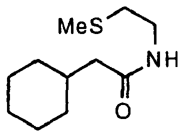

### Example 1105A

### N-(2-methylthioethyl)-2-cyclohexylacetamide

Following the procedure of example 1104A, 2-methylthioethylamine (1.0 g, 11 mmol) was converted to the title compound (1.77 g, 89%).
MS (DCI, NH₃): 216 (MH⁺); 233 (M+NH₄)⁺.

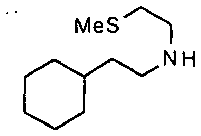

### Example 1105B

### N-(2-methylthioethyl)-2-cyclohexylethylamine

Using the procedure of example 1178F, example 1105A (1.75 g, 8.44 mmol) was converted into the title compound (1.63 g, 100%).
MS (DCI, NH₃): 202 (MH⁺).

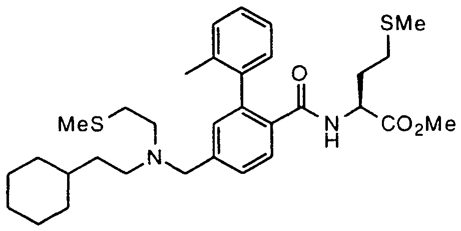

### Example 1105C

### N-[4-(N-(2-cyclohexylethyl)-N-2-methylthioethylaminomethyl)-2-(2-methylphenyl)benzoyl]methionine, methyl ester

Using the procedure of example 1103C, example 1105B (201 mg, 1.0 mmol) and example 403G (192 mg, 0.5 mmol) were combined to provide 151 mg (53%) of the title compound.
¹H NMR (300 MHz., CDCl₃): δ 7.91, dd, 1H; 7.42, dd, 1H; 7.18 - 7.37, m, 4H; 7.17, bs, 1H; 5.89, bd, 1H; 4.63, m, 1H; 3.66, s, 3H; 3.63, s, 2H; 2.68, m, 2H; 2.59, m, 2H; 2.48, t, 2H; 1.99 - 2.21, m, 10H; 1.85, m, 1H; 1.62, bm, 6H; 1.36, m, 2H; 1.06 - 1.30, m, 4H; 0.87, m, 2H.
MS (ESI+): 571 (MH+): (ESI-): 569 (M-H).

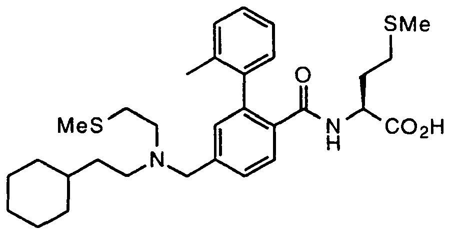

### Example 1105D

N-[4-(N-(2-cyclohexylethyl)-N-2-methylthioethylaminomethyl)-2-(2-methylphenyl)benzoyl]methionine;

A solution of example 1105C (145 mg, 0.25 mmol) in 2 mL of 3:1 THF/methanol was cooled in an ice bath and treated with lithium hydroxide (0.5 mL of a 1M aqueous solution, 0.5 mmol) and the mixture stirred overnight. The solution was concentrated to dryness and diluted with water and the pH adjusted to 4.5 with 1M aqueous phosphoric acid. The solid collected was by filtration and dried in the air to provide 130 mg (93%) of the title compound.
¹H NMR (300 MHz., CD₃OD): δ 7.71, d, 1H; 7.57, d, 1H; 7.35, d, 1H; 7.10 - 7.31, m, 4H; 4.32, m, 1H; 4.17, s, 2H; 3.10, m, 2H; 2.94, m, 2H; 2.76, m, 2H; 2.22, bs, 1H; 2.02 - 2.09, m, 3H; 2.10, s, 3H; 1.99, s, 3H; 1.89, m, 2H; 1.68, m, 6H; 1.56, m, 2H; 1.09 - 1.26, m, 4H; 0.93, m, 2H.
MS (ESI+): 557 (MH+): (ESI-): 555 (M-H). Calc'd for C₃₁H₄₄N₂O₃S₂•0.50 H₂O; C 65.80; H 8.02; N 4.95; Found: C 65.79; H 7.89; N 4.79.

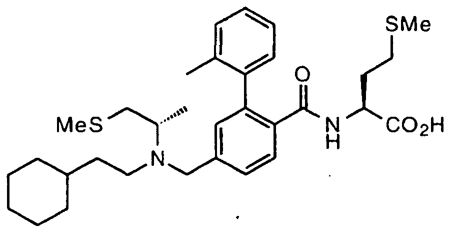

### Example 1106

### N-[4-(N-(2-cyclohexylethyl)-N-1-methyl-2(S)-methylthioethylaminomethyl)-2-(2-methylphenyl)benzoyl]methionine

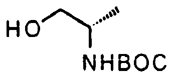

### Example 1106A

### 2(S)-N-t-butoxycarbonylaminopropan-1-ol

A stirred solution of 2(S)-amino-1-propanol (1.0 g, 13.3 mmol) in 20 mL of methylene chloride was treated with di-tertbutyldicarbonate (3.19 g, 14.6 mmol) in 5 mL of methylene chloride and then the solution was treated with 10 mL of 2M aqueous sodium carbonate and stirred for 2 hours. The biphasic mixture was diluted with water and the layers were separated. The aqueous layer was extracted with methylene chloride and the combined organic layers were dried, filtered and concentrated to provide 2.35 g (105%) of the title compound.
¹H NMR (300 MHz., CDCl₃)_{:} δ 4.59, bs, 1H; 3.77, m, 1H; 3.64, dd, 1H; 3.52, dd, 1H; 2.42, bs, 1H; 1.44, s, 9H; 1.14, d, 3H.
MS (DCI, NH₃): 176 (MH)⁺; 193 (M+NH₄)⁺.

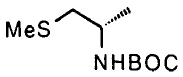

### Example 1106B

### 1-Methylthio-2(S)-N-t-butoxycarbonylaminopropane

A stirred solution of example 1106A (350 mg, 2.0 mmol) in 6 mL of methylene chloride was cooled in an ice/acetone bath and sequentially treated with triethylamine (0.34 mL, 2.4 mmol) and methanesulfonyl chloride (0.17 mL, 2.2 mmol) and the mixture stirred for 2 hours and then diluted with ether, extracted with water, 1M aqueousphosphoric acid, brine, dried filterd and concentrated to provide a yellow oil that was used directly. The mesylate was dissolved in 2 mL of DMF and added to a mixture of sodium thiomethoxide (280 mg, 4.0 mmol) and 5 mL of DMF and the mixture was stirred for 2 hours. The reaction was quenched by the addition of water and the mixture diluted with water and ethyl acetate. The layers were separated and the mixture was extracted with 2 additional portions of ethyl acetate and the combined organic layers washed with water and brine, dried, filtered and concentrated to provide 328 mg (80% overall) of the title compound.
¹H NMR (300 MHz., CDCl₃): δ 3.86, bs, 1H; 2.65, dd, 1H; 2.56, dd, 1H; 2.14, s, 3H; 1.45, s, 9H; 1.22, d, 3H.
MS (DCI, NH₃): 206 (MH)⁺; 223 (M+NH₄)⁺.

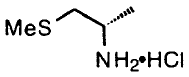

### Example 1106C

### 1-Methylthio-2(S)-aminopropane hydrochloride salt

Example 1106B (320 mg, 1.56 mmol) was dissolved in 2 mL of 4N HCl/dioxane and stirred for 1 Hour. The mixture was diluted with ether and filtered to provide 103 mg (53%) of the title compound as a white solid.
¹H NMR (300 MHz., CDCl₃): δ 8.56, bs, 3H; 3.51, m, 1H; 2.89, dd, 1H; 2.78, dd, 1H; 2.17, s, 3H; 1.54, d, 3H.
MS (DCI, NH₃): 123 (M+NH₄)⁺.

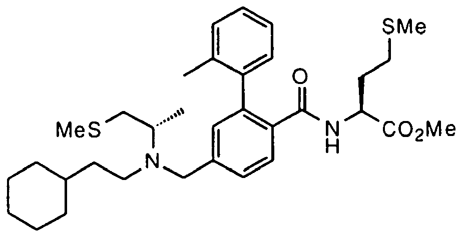

### Example 1106D

### N-[4-(N-(2-cyclohexylethyl)-N-1-methyl-2(S)-methylthioethylaminomethyl)-2-(2-methylphenyl)benzoyl]methionine, methyl ester

Part 1. Following the general procedure of example 403H, example 1106C (98 mg, 0.69 mmol), example 403G (243 mg, 0.63 mmol), diisopropylethylamine (0.12 mL, 0.69 mmol) and acetic acid (0.18 mL, 3.14 mmol) were stirred in 4 mL of 1,2-dichloroethane for 2 hours and then treated with sodium triacetoxyborohydride (263 mg, 1.26 mmol). This procedure yielded 332 mg of material that was used in the next step.

Part 2. The amine prepared in part 1 was treated with 2-cyclohexylacetaldehyde (159 mg, 1.26 mmol), acetic acid (0.36 mL, 6.3 mmol) and sitrred for 2 hours. This solution was treated with sodium triacetoxyborohydride (263 mg, 1.26 mmol) and the mixture stirred overnight. The mixture was quenched and worked-up as described in example 403H. The residue obtained was purified by cloumn chromatography on silica gel (20 g, 20% ethyl acetate/hexanes) to provide 225 mg (61 % overall) of the title compound.
¹H NMR (300 MHz., CDCl₃): δ 7.89, dd, 1H; 7.47, d, 1H; 7.15 - 7.37, m, 5H; 5.87, bd, 1H; 4.63, m, 1H; 3.67, d, 1H; 3.65, s, 3H; 3.55, d, 1H; 2.96, m, 1H; 2.75, dd, 1H; 2.44, m, 2H; 2.37, dd, 1H; 1.99 - 2.22, m, 10H; 1.84, m, 1H; 1.60, m, 6H; 1.09 - 1.33, m, 6H; 1.08, d, 3H; 0.72 - 1.00, m, 2H.
MS (ESI+): 585 (MH+): (ESI-): 583 (M-H).

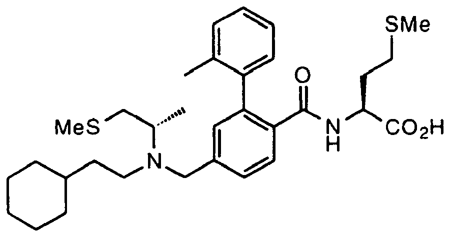

### Example 1106

### N-[4-(N-(2-cyclohexylethyl)-N-1-methyl-2(S)-methylthioethylaminomethyl)-2-(2-methylphenyl)benzoyl]methionine

Following the procedure of example 1105D, example I 106D (210 mg, 0.36 mmol) provided 110 mg (53%) of the title compound.
¹H NMR (300 MHz., CD₃OD): δ7.69, d, 1H; 7.56, bd, 1H; 7.37, bd, 1H; 7.09 - 7.32, m, 4H; 4.33, m, 1H; 4.16. m, 1H; 4.00, m, 1H; 3.32, dt, 1H; 2.89, m, 3H; 2.64, m, 1H; 2.23, bs, 1H; 2.06, m, 2H; 2.04, s, 3H; 1.98, s, 3H; 1.89, m, 2H; 1.65, m, 6H: 1.44, m, 2H; 1.32, d, 3H; 1.28, m, 3H; 0.88, m, 2H.
MS (ESI+): 571 (MH+): (ESI-): 569 (M-H). Calc'd for C₃₂H₄₆N₂O₃S₂; C 67.33; H 8.12; N 4.91; Found: C 67.12; H 8.10; N 4.70.

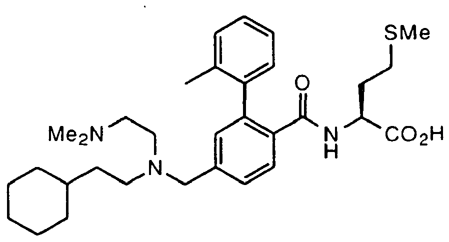

### Example 1107

### N-[4-(N-(2-cyclohexylethyl)-N-2-N,N-dimethylaminomethyl-2-(2-methylphenyl)benzoyl]methionine

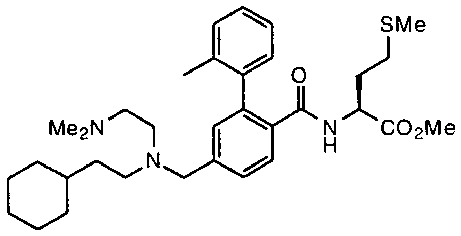

### Example 1107A

### N-[4-(N-(2-cyclohexylethyl)-N-2-N,N-dimethylaminomethyl)-2-(2-methylphenyl)benzoyl]methionine, methyl ester

Part 1. Following the procedure of example 1106D, part 1, example 403G (550 mg, 1.43 mmol) and 2-N,N-dimethylaminoethylamine (0.31 mL, 2.86 mmol) and acetic acid (0.82 mL, 14.3 mmol) gave the coressponding secondary amine (673 mg).

Part 2. Following the procedure of example 1106D part 2, the amine produced in example 1107A, part 1 (660 mg, 1.44 mmol) and 2-cyclohexyacetaldehyde (364 mg, 2.88 mmol) gave a material that was purified by column chromatography on silica gel (25 g, ethyl acetate then 90/10/0.1 ethyl acetate/methanol/conc. aq. ammonia) providing 498 mg (60% overall) of the title compound.
¹H NMR (300 MHz., CDCl₃): δ 790, dd, 1H; 7.41, dd, 1H; 7.18 - 7.34, m, 4H; 7.16, bs, 1H; 5.88, bs, 1H; 4.62, m, 1H; 3.65, s, 3H; 3.63, s, 2H; 2.57, m, 2H: 2.47, m, 2H; 2.39, m, 2H; 2.21, s, 6H; 1.99, 2.28, m, 7H; 1.86, m, 1H; 1.63, bm, 6H: 1.35, m, 2H; 1.20 m, 2H; 1.14. m, 2H; 0.85, m, 2H.
MS (ESI+): 568 (MH+): (ESI-): 566 (M-H).

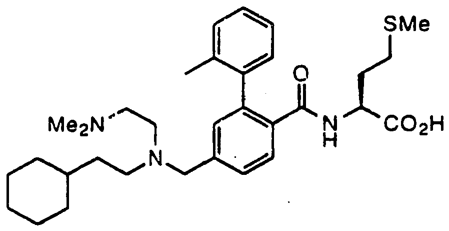

### Example 1107B

### N-[4-(N-(2-cyclohexylethyl)-N-2-N,N-dimethylaminomethyl)-2-(2-methylphenyl)benzoylmethionine

Following the procedure of example 1104D, example 1107A (485 mg, 0.85 mmol) provided 382 mg (81%) of the title compound as a white lyophilate.
¹H NMR (300 MHz., CD₃OD): δ 7.66, d, 1H; 7.46, d, 1H; 7.05 - 7.33, m, 5H; 4.35, m, 1H; 3.74, s, 2H; 3.17, t, 1H; 2.82, t, 2H; 2.75, s, 6H; 2.60, m, 2H; .24, bs, 1H; 1.94 - 2.12, m, 6H; 1.85, m, 2H; 1.67, m, 6H; 1.45, m, 2H; 1.21, m, 4H; 0.92, m, 2H.
MS (ESI+): 554 (MH+): (ESI-): 552 (M-H). Calc'd for C₃₂H₄₇N₃O₃S·1.00 H₂O; C 67.22; H 8.64; N 7.35; Found: C 67.23; H 8.43; N 7.26.

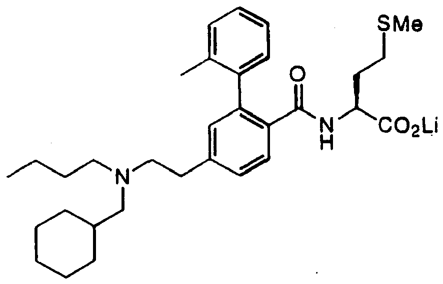

### Example 1140

### N-[4-(N-cyclohexylmethyl-N-butylaminoethyl)-2-(2-methylphenyl)benzoyl]methionine lithium salt

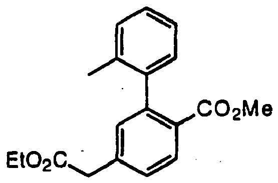

### Example 1140A

### Methyl 4-(Ethoxycarbonylmethyl)-2-(2-methylphenyl)benzoate

A solution of intermediate 1178D (397 g, 1.24 mmol), palladium(II) acetate (22 mg), 1,3-bis(diphenylphosphino)propane (42 mg), N,N-diisopropylethylamine (0.5 mL) in ethanol ( 1 mL) and DMF (5 mL) was stirred at 80 °C under carbon monoxide balloon for 4 hours. The reaction mixture was then partitioned between ethyl acetate (80 mL) and water (20 mL). The organic layer was washed with water (2 X 20 mL), brine (20 mL), dried over anhydrous magnesium sulfate, filtered and concentrated. The residue was purified by column chromatography with 5% ethyl acetate in hexane to give the title compound (233 mg, 58%). ¹HNMR (300 MHz, CDCl₃) δ 7.94 (d, 1 H), 7.35 (dd, 1 H), 7.30-7.17 (m, 3 H). 7.16 (d, 1 H), 7.07 (br d, 1 H), 4.16 (q, 2 H), 3.67 (s, 2 H), 3.61 (s, 3 H), 2.06 (s, 3 H), 1.25 (t, 3 H). MS(CI/NH₃) m/z: 330 (M+NH₄)⁺.

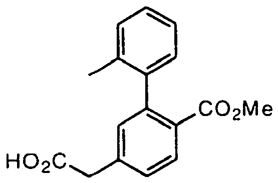

### Example 1140B

### Methyl 4-(Carboxymethyl)-2-(2-methylphenyl)benzoate

To the solution of intermediate 1140A (213 mg, 0.682 mmol) in methanol (3 mL) was added NaOH (0.979 M in water, 0.697 mL). After 2 hours, the reaction mixture was acidified with HCl (1.0 M, 1 mL), and was then partitioned between ethyl acetate (80 mL) and water (20 mL). The organic layer was washed with water (2 X 20 mL), brine (20 mL), dried over anhydrous magnesium sulfate, filtered and concentrated. The residue was used witout further purification.

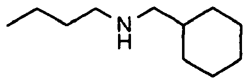

### Example 1140C

### N-Butylcyclohexymethylamine

The procedures descriped in the Example 1178E and 1178F were used here to convert cyclohexylacetyl chloride (1.47 g, 10.0 mmol) and butylamine to the title amine in 85% yield. The amine was not purified before it was used.

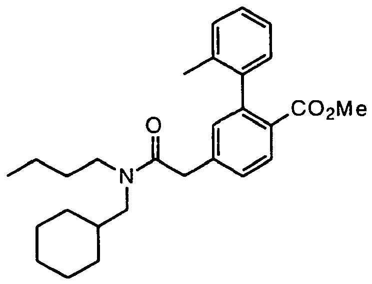

### Example 1140D

### Methyl 4-(N-Cyclohexylmethyl-N-butylaminocarbonylmethyl)-2-(2-methylphenyl)benzoate

The procedure described in example 1144C was used here to combine intermediate 1140B (311 mg, 1.10 mmol) and intermediate 1140C (205 mg) to give the title compound (247 mg, 52%). ¹HNMR (300 MHz, CDCl₃) δ 7.94 (d, 1 H), 7.33 (M, 1 H), 7.25-7.15 (m, 3 H), 7.13,7.11 (2 d's, 1 H), 7.05 (m, 1 H), 3.76,3.75 (2 s's, 2 H), 3.60 (s, 3 H), 3.35-3.05 (m, 4H), 2.05,2.04 (2 s's, 3 H), 1.80-1.10 (m, 15 H), 0.91,0.89 (2 t's, 3 H). MS(CI/NH₃) m/z: 436 (M+H)⁺.

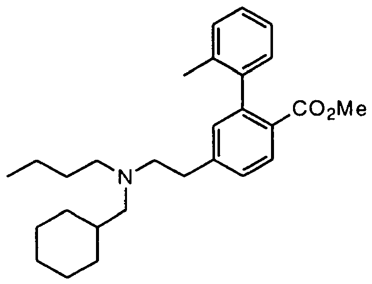

### Example 1140E

### Methyl 4(N-Cyclohexylmethyl-N-butylaminoethyl)-2-(2-methylphenyl)benzoate

A solution of intermediate 1140D (118 mg, 0.271 mmol) and borane (1.0 M in THF, 0.54 mL) in THF was reluxed for 15 hours. Methanol (0.5 mL) was added dropwisly to the reaction, followed by concentrated HCl (0.5 mL), and the mixture was heated at 60 °C for 1 hour. The it was cooled to room temperature, The reaction mixture was adjusted to pH about 12-14 with sodium carbonate (2.0 M in water). The reaction mixture was then partitioned between ethyl acetate (50 mL) and water (5 mL). The organic layer was washed with water (10 mL), brine (20 mL), dried over anhydrous magnesium sulfate, filtered and concentrated to give the intermediate amine 1140E. The amine was used without further purification. ¹HNMR (300 MHz, CDCl₃) δ 7.90 (d, 1 H), 7.28-7.17 (m, 4 H), 7.05 (m, 2 H), 3.60 (s, 3 H), 2.75 (m, 2 H), 2.66 (m, 2 H), 2.40 (t, 2 H), 2.19 (d, 2 H), 2.06 (s, 3 H), 1.80-1.10 (m, 15 H), 0.88 (t, 3 H). MS(CI/NH₃) m/z: 422 (M+H)⁺.

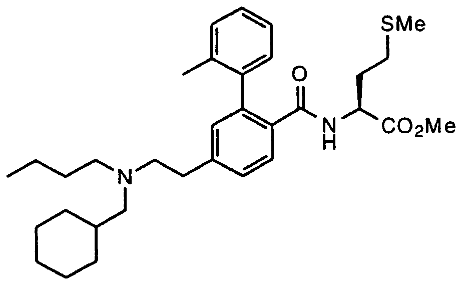

### Examples 1140F

### N-[4-(N-Cyclohexylmethyl-N-butylaminoethyl)-2-(2-methylphenyl)benzoy]imethionine Methyl Ester

The procedures descriped in the Example 403E and 403F were used here to convert the above intermediate amine 1140E to the title methyl ester (113 mg, 76%, 3 steps from 1140D). ¹HNMR (300 MHz, CDCl₃) δ 7.90 (2 d's, 1 H), 7.34-7.18 (m, 5 H), 7.01 (s, I H), 5.87 (br d, I H), 4.62 (m, 1 H), 3.65 (s, 3 H), 2.75 (m, 2 H), 2.66 (m, 2 H), 2.4 (t, 2 H), 2.20 (d, 2 H), 2.19-1.98 (m, 8 H), 1.87 (m, 1 H), 1.80-1.10 (m, 16 H), 0.88 (t, 3 H). MS(CI/NH₃) m/z: 553 (M+H)⁺.

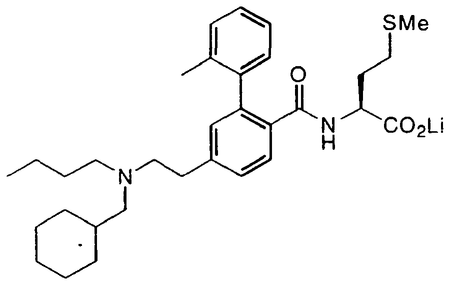

### Example 1140G

### N-[4-(N-cyclohexylmethyl-N-butylaminoethyl)-2-(2-methylphenyl)benzoyl]methionine lithium salt

The procedure descriped in the Example 4031 was used here to convert the intermediate 1140F (107 mg) to the title lithium salt (91 mg, 87%). ¹H NMR (300 MHz, dmso-d₆) δ 7.51 (d, 1 H), 7.33-7.13 (m, 5 H), 7.05 (br s, 1 H), 6.95 (m, 1 H), 3.71 (m, 1 H), 2.76 (m, 2 H), 2.67 (m, 2 H), 2.42 (t, 2 H), 2.21 (d, 2 H), 2.10-1.82 (m, 8 H), 1.80-1.10 (m, 17 H), 0.88 (t, 3 H). MS(ESI-) m/z: 537 (M-H)⁻.

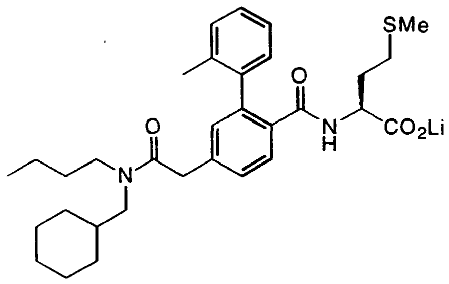

### Example 1141

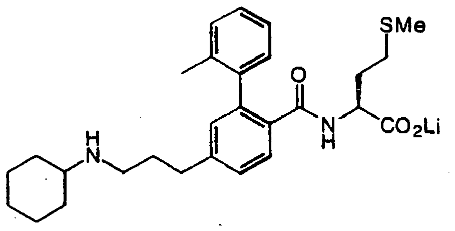

### Example 1144

### N-[4-(N-Cyclohexylpropyl)-2-(2-methylphenyl)benzoyl]methionine lithium salt

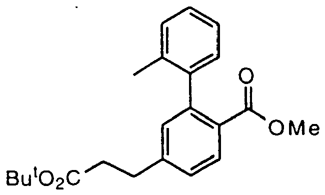

### Example 1144A

### Methyl 4-(tert-Butoxycarbonylethyl)-2-(2-methylphenyl)benzoate

To a solution of (t-butoxycarbonylmethyl)triphenylphosphonium bromide (10.98 g, 24.0 mmol) in THF (150 mL) at 0 °C was added potassium t-butoxide (1.0 M in THF, 24 mL) over 5 min. After 2 h, the aldehyde from example 1171A (20 mmol) in THF (10 mL) was added slowly over 5 min., and the reaction was further stirred for 30 min. The reaction mixture was diluted with hexane (200 mL), and the resulting muddy mixture was filtered through silica gel (200 g), rinsed with ether, and concentrated to give an intermediate olefin. ¹H NMR (300 MHz, CDCl₃) δ 7.97 (d, 1 H), 7.59 (d, 1 H), 7.54 (dd, 1 H), 7.37 (d, I H), 7.30-7.27 (m, 3 H), 7.06 (d, 1 H), 6.44 (d, 1 H), 3.61 (s, 3 H), 2.06 (s, 3 H), 1.52 (s, 9 H). MS(CI/NH₃) m/z: 353 (M+H)⁺, 370 (M+NH₄)⁺.

That intermediate was mixed with palladium on carbon (10%, 2.0 g) in ethanol (30 mL), and was stirred under a hydrogen balloon overnight. The mixture was then filtered through Celite^{™} (5 g), and the filtrate was concentrated. The residue was then redesolved in ether (100 mL) and the solution was filtered through silica gel (30 g). Concentration of the filtrate afforded the title compound (7.27 g, 99% for 2 steps). ¹H NMR (300 MHz, CDCl₃) δ 7.91 (d, 1H), 7.28-7.15 (m, 4 H), 7.07-7.03 (m, 2 H), 3.60 (s, 3 H), 2.97 (t, 2 H), 2.57 (t, 2 H), 2.05 (s, 3 H), 1.40 (s, 9 H). MS(CI/NH₃) m/z: 355 (M+H)⁺, 372 (M+NH₄)⁺.

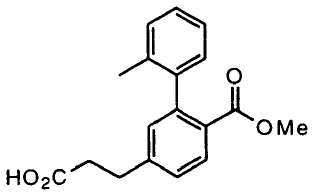

### Example 1144B

### Methyl 4-(2-Carboxyethyl)-2-(2-methylphenyl)benzoate

A solution of intermediate 1144A (5.00 g) in trifluoroacetic acid (20 mL) and methyl sulfide (3 mL) was stirred at room temperature for 7 hours. Sovlent was then evaporated to give an off-white solid, which was used without further purification.

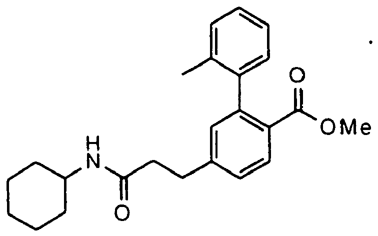

### Example 1144C

### Methyl 4-(2-Cyclohexylcarbomoylethyl)-2-(2-methylphenyl)benzoate

To a solution of intermediate 1144B (150 mg, 0.50 mmol), oxallyl chloride (2.0 M in DCM, 0.5 mL) in DCM (2 mL) was added a small drop of DMF. After 2 hours at room temperature , the reaction was concentrated to drynees, and redeolved in DCM (3 mL). To it was added cyclohexylamine (99 mg, 1 mmol) and triethylamine (100 mg, 1 mmol). After 15 min., HCl (1.0 M in ether, 2.0 mL) was added to the reaction mixture, and it was filtered through silica gel (5 g). The residue after concentration of the filtrate was purified by column chromatography with 20% ethyl acetate in hexane to give the title compound (152 mg, 80%).
¹H NMR (300 MHz, CDCl₃) δ 7.90 (d, 1 H), 7.28-7.15 (m, 4 H), 7.07-7.02 (m, 2 H), .5.16 (m, 1 H), 3.72 (m, 1 H), 3.60 (s, 3H), 3.02 (t, 2 H), 2.45 (t, 2 H), 2.05 (s, 3 H), 1.85 (m, 2 H), 1.70-1.55 (m, 3 H), 1.40-0.95 (m, 6 H). MS(CI/NH₃) m/z: 380 (M+H)⁺, 397 (M+NH₄)⁺.

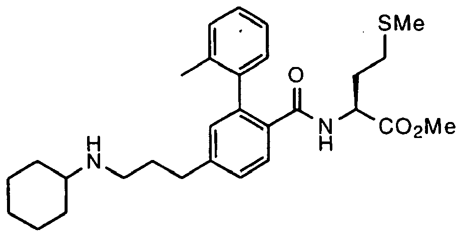

### Example 1144D

### N-[4-(N-Cyclohexylpropyl)-2-(2-methylphenyl)benzoyl]methionine

A solution of intermediate 1144C (150 mg, 0.40 mmol) and borane (1.0 M in THF, 1.0 mL) in THF (1 mL) was reluxed for 15 hours. Methanol (0.5 mL) was added dropwisly to the reaction, followed by concentrated HCl (0.5 mL), and the mixture was heated at 60 °C for 1 hour. The reaction mixture was cooled to room temperature, and was adjusted to pH about 12-14 with sodium carbonate (2.0 M in water). The reaction mixture was then partitioned between ethyl acetate (50 mL) and water (5 mL). The organic layer was washed with water (10 mL), brine (20 mL), dried over anhydrous magnesium sulfate, filtered and concentrated to give the intermediate amine. The amine was used without further purification. MS(CI/NH₃) m/z: 366 (M+H)⁺.

The procedures descriped in the Example 403E and 403F were used here to convert the above intermediate amine to the title methyl ester (58%, 3 steps).

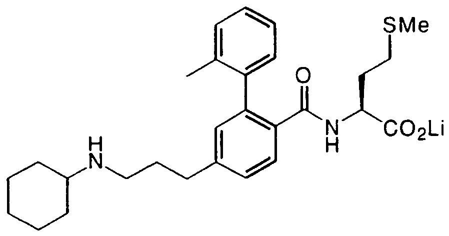

### Example 1144E

### N-[4-(N-Cyclohexylpropyl)-2-(2-methylphenyl)benzoyl]methionine lithium salt

The procedure descriped in the Example 4031 was used here to convert the intermediate 1 144D (121 mg) to the title lithium salt (107 mg, 100%). ¹H NMR (300 MHz, dmso-d₆) δ 7.45 (d, 1 H), 7.27-7.08 (m, 4 H), 7.02-6.93 (m, 2 H), 6.90 (m, 1 H), 3.80 (m, 1 H), 3.65 (m, 1 H), 3.30 (m, 2 H), 2.64 (t, 2 H), 2.20-1.80 (m, 10 H), 1.80-1.45 (m, 7 H), 1.30-0.88 (m, 6 H). MS(ESI-) m/z: 481 (M-H)⁻.

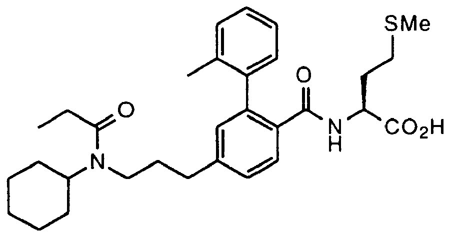

### Example 1145

### N-[4-(N-Cyclohexyl-N-propanoylaminopropyl)-2-(2-methylphenyl)benzoyl]methionine

To s stirred mixture of 1144E (70 mg, 0.14 mmol) in THF (1 mL) and saturated aqueous sodium bicarbonate (1 mL) was added propionyl chloride (0.10 mL). After 10 min, the reaction mixture was adjusted to pH 4-5, and it was then partitioned between ethyl acetate (50 mL) and water (5 mL). The organic layer was washed with water (10 mL), brine (10 mL), dried over anhydrous magnesium sulfate, filtered and concentrated. The residue was heated at 60 °C under high vacuum for 5 hours to give the title compound (59 mg, 78%). ¹H NMR (300 MHz, dmso-d₆) δ 7.47 (m, 1 H), 7.32-6.97 (m, 7 H), 4.25 (m, 1 H), 3.57 (m, 1 H), 3.35 (m, 2 H), 2.80-2.60 (m, 2 H), 2.30-1.85 (m, 12 H), 1.85-1.45 (m, 7 H), 1.30-0.88 (m, 9 H). MS(ESI-) m/z: 537 (M-H)⁻.

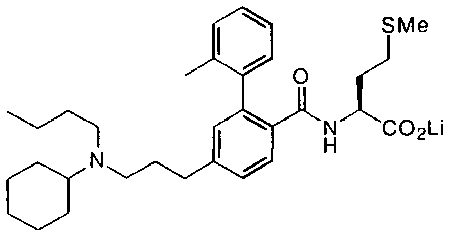

### Example 1146

### N-[4-(N-Cyclohexyl-N-butylaminopropyl)-2-(2-methylphenyl)benzoyl]methionine lithium salt

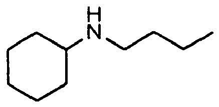

### Example 1146A

### N-Butylcyclohexaylamine

The procedures descriped in the Example 1178E and 1178F were used here to convert butyric chloride and cyclohexylamine to the title amine in 86% yield. ¹H NMR (300 MHz, CDCl₃) δ 2.62 (t, 2 H), 2.41 (m, 1 H), 1.95-1.00 (m, 15 H), 0.92 (t, 3 H). MS(CI/NH₃) m/z: 156 (M+H)⁺.

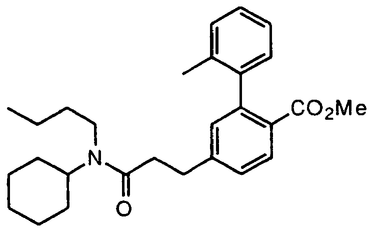

### Example 1146B

### Methyl N-[4-(N-Cyclohexyl-N-butylaminopropyl)-2-(2-methylphenyl)benzoate

The procedure descriped in the Example 1144C was used here to convert the intermediate 1144B (298 mg) and N-butylcyclohexylamine (intermediate 1146A, 310 mg, 2.0 mmol) to the title methyl ester (233 mg, 54%). ¹H NMR (300 MHz, CDCl₃) δ 7.90 (2 d's, 1 H), 7.30-7.15 (m, 4 H), 7.07 (m, 2 H), 4.25 (m, 1 H), 3.60 (s, 3 H), 3.18 (m, 1 H). 3.05 (m, 3 H), 2.62 (m, 2 H), 2.06 (2s's, 3 H), 1.85-1.05 (m, 14 H). 0.90 (2 t's, 3 H). MS(CI/NH₃) m/z: 436 (M+H)⁺.

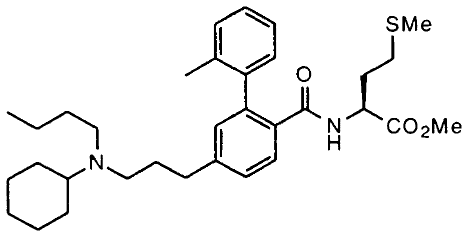

### Example 1146C

### N-[4-(N-Cyclohexyl-N-butylaminopropyl)-2-(2-methylphenyl)benzoyl]methionine Methyl Ester

The procedure descriped in the Example 1144C was used here to convert the intermediate 1146B (230 mg) to the title methyl ester (184 mg, 63%). ¹H NMR (300 MHz, CDCl₃) δ 7.90 (2 d's, 1 H), ), 7.35-7.19 (m, 4 H), 7.03 (m, 1 H), 5.89 (m, 1 H), 4.62 (m, 1 H), 3.66 (s, 3 H), 3.05 (m, 1 H), 2.66 (t, 2 H), 2.46 (t, 2 H), 2.41 (t, 2 H), 2.20-2.00 (4 s's, 6 H), 2.05 (m, 2 H), 1.90-1.00 (m, 18 H), 0.90 (t, 3 H). MS(CI/NH₃) m/z: 553 (M+H)⁺.

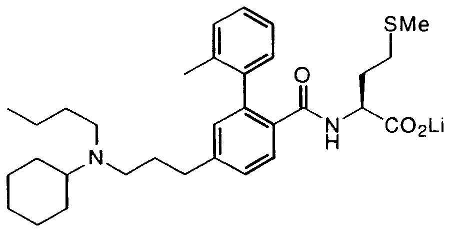

### Example 1146D

### N-[4-(N-Cyclohexyl-N-butylaminopropyl)-2-(2-methylphenyl)benzoyl]methionine lithium salt

The procedure descriped in the Example 4031 was used here to convert the intermediate 1146C (179 mg) to the title lithium salt (153 mg, 81 %). ¹H NMR (300 MHz, dmso-d₆) δ 7.46 (m, 1 H), 7.35-7.08 (m, 4 H), 7.07-6.90 (m, 2 H), 3.70 (m, 1 H), 3.05 (m, 1 H), 2.64 (t, 2 H), 2.37 (m, 4 H), 2.20-1.90 (m, 8 H), 1.90-0.95 (m, 18 H), 0.85 (t, 3 H). MS(ESI-) m/z: 537 (M-H)⁻.

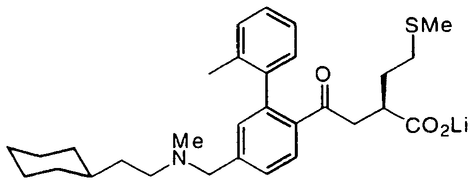

### Example 1158

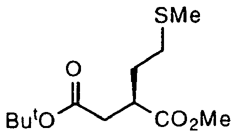

### Example 1158A

### Methyl 2-(tert-butoxycarbonylmethyl)-4-methylthiobutyrate

To a -78 °C solution of methyl 4-methylthiobutyrate (1.48 g, 10.0 mmol) in THF (20 mL) was added sodium bis(trimethylsilyl)amide (1.0 M in THF, 11 mL). After 30 min, tert-butyl bromoacetate (2.34 g, 12.0 mmol) was added to the reaction, and the reaction mixture was gradually warmed to the room temperature over 6 hours. The reaction mixture was then partitioned between ethyl acetate (80 mL) and water (20 mL). The organic layer was washed with water (2 X 20 mL), brine (20 mL), dried over anhydrous magnesium sulfate, filtered and concentrated. The residue was purified by column chromatography with 5% ethyl acetate in hexane to give the title compound (1.21 g, 46%). ¹HNMR (300 MHz, CDCl₃) δ 3.75 (s, 3 H), 2.71 (t, 2 H), 2.51 (t, 2 H), 2.32 (m, 1 H), 2.06 (s, 1 H), 1.89 (t, 1 H), 1.41 (s, 9 H). MS(CI/NH₃) m/z: 263 (M+H)⁺.

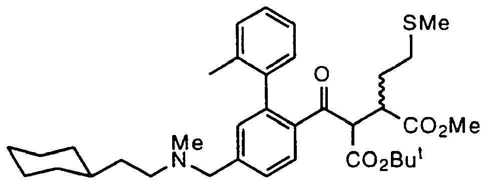

### Example 1158B

To a solution of the acid from example 608C (530 mg, 1.32 mmol) in DCM (2 mL) was added oxallyl chloride (2.0 M in DCM, 1.5 mL), followed by a small drop of DMF. After 2 hours at room temperature , the solvent was removed, and the residue was further dried under high vacuum (1 mmHg) for 1 hour. The solid (acid chloride) was redesolved in THF (5 mL).

To a -78 °C solution of 1158A (1.21 g, 4.61 mmol) in THF (10 mL) in a separate flask was added sodium bis(trimethylsilyl)amide (1.0 M in THF, 5.28 mL). After 30 min.. the acid chloride solution was added slowly to the reaction mixture via a cannula. After I hour, the reaction mixture was quenched with saturated aqueous ammonium chloride (3 mL) at - 78°C. After it reached the room temperature, the reaction mixture was then partitioned between ethyl acetate (80 mL) and water (20 mL). The organic layer was washed with sodium bicarbonate (saturated in water, 10 mL), water (2 X 10 mL), brine (20 mL), dried over anhydrous magnesium sulfate, filtered and concentrated. The residue was purified by column chromatography with 30% ethyl acetate in hexane to give the title compound (430 mg, 53%). ¹HNMR is messy because of 4 diastereomers exist. MS(CI/NH₃) m/z: 610 (M+H)⁺.

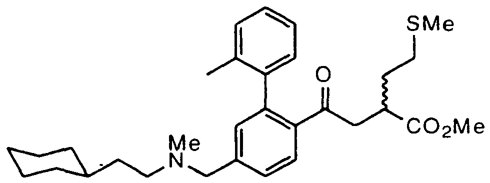

### Example 1158C

### Methyl 3-N-[4-(N-Cyclohexyl-N-butylaminopropyl)-2-(2-methylphenyl)benzoylmethyl]-4-methylthiobutyrate

A solution of 1158B (420 mg, 0.69 mmol) in HCl (4.0 M in 1,4-dioxane, 5 mL) was heated at 80 °C for 2 hours. Solvent was evaporated, and the residue was redesolved in ethyl acetate (100 mL). The mixture was then washed with sodium bicarbonate (saturated in water, 20 mL), water (20 mL), brine (20 mL), dried over anhydrous magnesium sulfate, filtered and concentrated. The residue was purified by column chromatography with 30% ethyl acetate in hexane to give the title compound (121 mg, 34%). ¹HNMR (300 MHz, CDCl₃) δ 7.62 (d, 1 H), 7.40 (br d, 1 H), 7.31-7.12 (m, 4 H), 7.07 (br d, 1 H), 3.62 (s, 3 H), 3.54 (br s, 2 H), 2.85 (m, 1 H), 2.71 (m, 1 H), 2.40 (m, 2 H), 2.35-2.00 (m, 12 H), 1.80-0.80 (m, 15 H). MS(CI/NH₃) m/z: 510 (M+H)⁺.

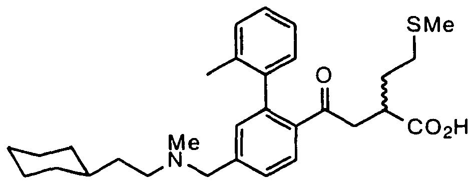

### Example 1158D

### 3-[4-(N-Cyclohexyl-N-methylaminopropyl)-2-(2-methylphenyl)benzoylmethyl]-4-methylthiobutyric acid

The intermediate 1158C (112 mg) in MeOH (2 ML) and lithium hydroxide (1.0 M in water, 0.7 mL) was heated at 50 °C for 5 hours. The reaction mixture was then adjusted to pH 4-5 with KH₂PO₄ (saturated in water), and extracted with ethyl acetate (3 X 20 mL). The combined extracts were washed with brine, dried over anhydrous magnesium sulfate, filtered and concentrated to give the title compound (110 mg, 100%). ¹H NMR (300 MHz, dmso-d₆) δ 7.77 (m, 1 H), 7.61 (br d, 1 H), 7.40 (m, 1 H), 7.35-7.15 (m, 3 H), 7.07 (m, 1 H), 4.15 (br loop, 2 H), 2.88 (m, 2 H), 2.69 (m, I H), 2.28 (m, 2 H), 2.22-1.96 (m, 11 H), 1.72-0.80 (m, 15 H). MS(ESI-) m/z: 494 (M-H)⁻.

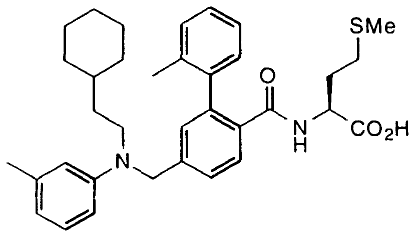

### Example 1171

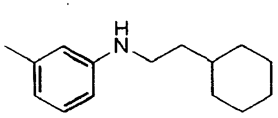

### Example 1171A

### N-(2-Cyclohexylethyl)-N-(3-methylphenyl)amine

To a stirred solution at ambient temperature of cyclohexylacetic acid (500 mg, 3.52 mmol) and 3-methylaniline (0.45 mL, 4.22 mmol) in DMF (10 mL) was added 1-ethyl-3-(3-dimethylaminopropyl)carbodiimide (809 mg, 4.22 mmol). Reaction stirred overnight at ambient tenperature. Reaction diluted with EtOAc and washed with water, 1.0M NaHCO₃ (2x), 1N H₃PO₄ (2x), and brine. Organic layer dried with Na₂SO₄, filtered, and concentrated in vacuo. To a solution at ambient temperature under N₂ of this residue in anhydrous THF (3 mL) was added a 1.0M lithium aluminum hydride solution (7.00 mL, 7 mmol) in THF. Reaction refluxed overnight. Reaction cooled to 0°C and quenched with successive addition of water (0.27 mL), 15% aqueous NaOH (0.27 mL), and water (0.80 mL). Mixture stirred 30 minutes at ambient temperature, and solids filtered off through celite and washed with EtOAc. Filtrate dried with Na₂SO₄, filtered, and concentrated in vacuo to produce a colorless oil. m/e (DCI/NH₃) 218 (MH⁺)

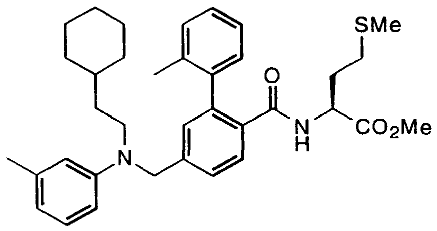

### Example 1171B

### N-[4-N-(2-Cyclohexyl-N-(3-methylphenyl)aminomethyl-2-(2-methylphenyl)benzoyl]methionine methyl ester

The desired ester was prepared using the method described in Example 403H starting with the compounds described in Example 403G and Example 1171A. m/e (ESI) 585 (MH⁻)

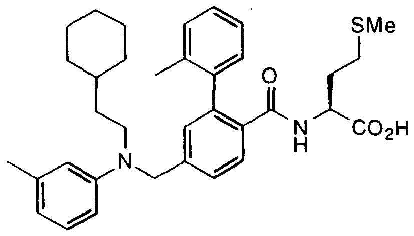

### Example 1171C

### N-[4-N-(2-Cyclohexyethyl)-N-(3-methylphenyl)aminomethyl-2-(2-methylphenyl)benzoyl]methionine

The desired compound was prepared according to the method of Example 4031 starting with compound prepared in Example 1171B. ¹H (300MHz, CDCl₃, δ) 7.92 (1H, t, J=9Hz), 7.40-7.00 (8H, m), 6.47 (2H, m), 5.86 (1H, d, J=8Hz), 4.51 (4H, m), 3.39 (2H, m), 2.25 (3H, s), 2.15-1.80 (8H, m), 1.70 (5H, m), 1.50 (3H, m), 1.40-1.05 (4H, m), 0.96 (2H, m). m/e (ESI) 571 (MH⁻) Anal.calc. for C₃₅H₄₄N₂O₃S·1.00 H₂O C 71.15, H 7.85, N 4.74 Found C 70.91, H 7.89, N 4.46

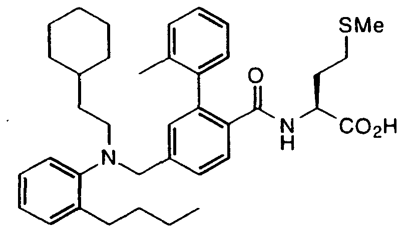

### Example 1172

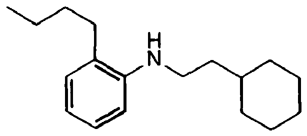

### Example 1172A

### N-(2-Butylphenyl)-N-(2-cyclohexylethyl)amine

The desired amine was prepared using the method described in Example 1171A starting with cyclohexylacetic acid and 2-butylaniline. m/e (DCI/NH₃) 260 (MH⁺)

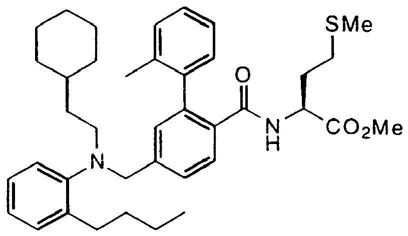

### Example 1172B

### N-[4-N-(2-Butylphenyl)-N-(2-cyclohexylethyl)aminomethyl-2-(2-methylphenyl)benzoyl]methionine methyl ester

The desired ester was prepared using the method described in Example 403H starting with the compounds described in Example 403G and Example 1172A. m/e (ESI) 627 (MH⁻)

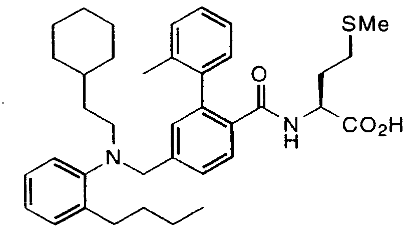

### Example 11172C

### N-[4-N-(2-Butylphenyl)-N-(2-cyclohexylethyl)aminomethyl-2-(2-methylphenyl)benzoyl]methionine

The desired compound was prepared according to the method of Example 4031 starting with compound prepared in Example 1172B.¹H (300MHz, CDCl₃, δ) 7.94 (1H, t, J=9Hz), 7.41 (1H, bd, J=8HZ), 7.40-7.00 (9H, m), 5.85 (1H. dd, J=8&2Hz), 4.55 (1H, m), 4.07 (2H, s), 2.91 (2H, m), 2.68 (2H, m), 2.20-1.80 (9H, m), 1.70-1.40 (8H, m), 1.40-1.00 (8H, m), 0.86 (3H, t, J=8Hz), 0.79 (2H, m). m/e (ESI) 613 (MH⁻) Anal.calc. for C₃₈H₅₀N₂O₃S·0.25 H₂O C 73.69, H 8.22, N 4.52 Found C 73.74, H 8.17, N 4.30

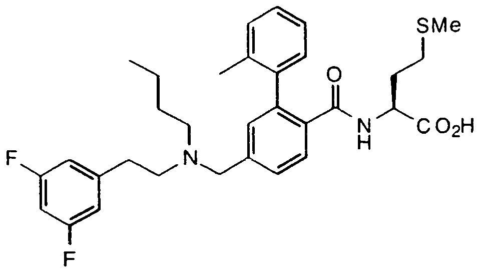

### Example 1173

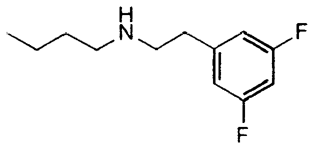

### Example 1173A

### N-(2-Butylphenyl)-N-(2-(3,5-difluorolphenylethyl)amine

The desired amine was prepared using the method described in Example 1171A starting with 3,5-difluorophenylacetic acid and butylamine. m/e (DCI/NH₃) 214 (MH⁺)

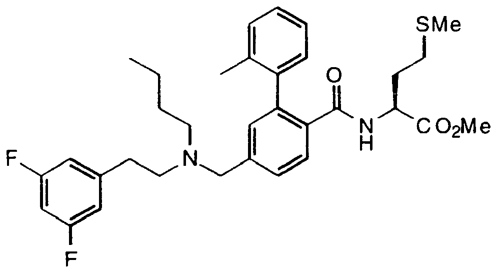

### Example 1173B

### N-[4-N-Butyl-N-(2-(3,5-difluoro)phenylethyl)aminomethyl-2-(2-methyl)phenyl)benzoyl]methionine methyl ester

The desired ester was prepared using the method described in Example 403H starting with the compounds described in Example 403G and Example 1173A. m/e (ESI) 581 (MH⁻)

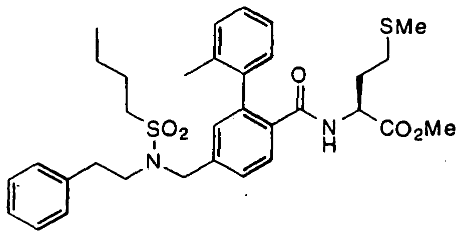

### Example 1174D

### N-[4-N-Butanesulfonyl-N-(2-phenylethyl)aminomethyl-2-(2-methylphenyl)benzoyl]methionine methyl ester

The desired compound was prepared using the method described in Example 403F starting with the product from Example 1174C. m/e (ESI) 480 (MH⁻)

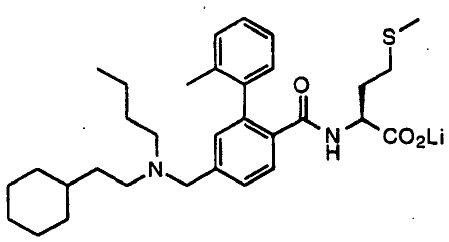

### Example 1178

### N-[4-(N-(-2-cyclohexylethyl)-N-butylaminomethyl)-2-(2-methylphenyl)benzoyl]methionine,

### Lithium Salt

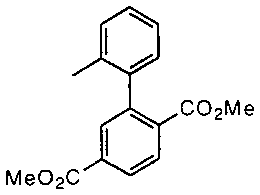

### Example 1178A

### Dimethyl-(2-methylphenyl)terephthalate

A mixture of dimethyliodoterephthalate (278 g, 0.87 mol), 2-methylphenylboronic acid (141 g, 1.04 mol) palladium (II) acetate (1.95 g, 0.0087 mol) and triphenylphosphine (9.1 g, 0.035 mol) in 2.2 L of toluene and 2.2 L of 2M sodium carbonate was degassed with nitrogen and heated to 80°C for 1.5 hours and cooled to ambient temperature. The layers were separated and the organic layer filtered through a plug of silica gel (600g) prewetted with methyl t-butylether (MTBE, 1.2 L). The frit was washed with 5 L of MTBE. The mixture was then concentrated to provide 237 g (96%) of the title compound. ¹H NMR (CDCl₃) δ 8.09, dd, 1H; 8.02, d, 1H; 7.95, d, 1H; 7.20 - 7.34, m, 3H; 7.10, bd, 1H, 3.96, s, 3H; 3.64, s, 3H; 2.08, s, 3H. MS (DCI/NH₃) 302 (M + NH₄)⁺.

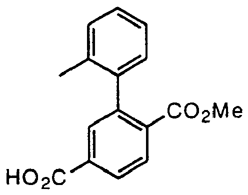

### Example 1178B

### 2-(2-methylphenyl)-4-carboxybenzoic acid, methyl ester

A solution of example 1178A (194 g, 0.68 mol) in 2:1 THF/methanol (∼0.3M) was cooled to 0°C and lithium hydroxide (0.38 L of a 2.2 M aqueous solution, 0.82 mol) was added such that the reaction temperature remained below 10°C. The cooling bath was removed and the mixture allowed to warm to 11°C overnight and then warmed to - 20°C over 4 hours. The mixture was concentrated to a volume of - 1.2 L and then diluted to 5.6 L with water. The mixture was extracted with hexanes and the aqueous layer filtered through celite (∼200 g) and the celite pad washed with water. The mixture was diluted with ethyl acetate (6 L) and the pH of the aqueous phase adjusted to 5.5 by the addition of 3M aqueous HCl (-250 mL). The organic phase was removed and concentrated to provide 171 g (93%) of the title compound. The material was ∼ 87% pure. An analytical sample was obtained by recrystallization from aqueous ethanol. ¹H NMR (CDCl₃) δ 8.14, dd, I H; 8.03, d, 1H; 8.01, d, 1H; 7.28 - 7.42, m, 3H; 7.09, bd, 1H; 3.64, s, 3H; 2.08, s, 3H. MS (DCI/NH₃): 271 (MH)⁺; 288 (M + NH₄)⁺.

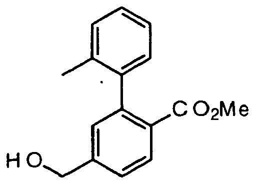

### Example 1178C

### 4-hydroxymethyl-2-(2-methylphenyl)benzoic, methyl ester

A solution of example 1178B (4.67g, 17.3 mmol) in 35 mL of THF was cooled in an ice bath and treated with borane (0.88M in THF, 39 mL, 34.6 mmol) such that the internal temperature remained below 10°C. The cooling bath was removed and the solution stirred for 3 hours and then cooled in an ice bath. The reaction was quenched by the careful addition of 8 mL of water (vigorous evolution of hydrogen gas) keeping the temperature below 10°C. An additional 8 mL of water was added and the mixture partitioned between ethyl acetate and 2N sodium hydroxide. The layers were separated and the organic layer was extracted with water, dried, filtered and concentrated. The residue was purified by column chromatography on silica gel to provide 3.90 g (88%) of the title compound. ¹H NMR (CDCl₃) δ 7.98, d, 1H; 743, dd, 1H; 7.16 - 7.28, m, 4H; 7.07, bd, 1H; 4.77, s, 2H; 3.62, s, 3H; 2.05, s, 3H; 1.78, bs, 1H. MS (DCI/NH₃): 257 (MH)⁺; 274 (M + NH₄)⁺.

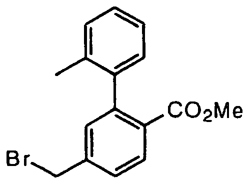

### Example 1178D

### 4-bromomethyl-2-(2-methylphenyl)benzoic, methyl ester

A solution of 36 g (140 mmol) of example 1178C and 13.4 g (154 mmol) lithium bromide in DMF (150 mL) was chilled in an ice-water bath, then 40.3 g (14.0 mL, 149 mmol) phosphorous tribromide was added, followed by more DMF (50 mL). After 15 minutes the reaction was partitioned between water (1200 mL) and Et₂O (600 mL). The aqueous layer was extracted with Et₂O (2 x 150 mL), then the combined Et₂O layers were washed with brine, and dried over Na₂SO₄. After filtration and concentration, recovered 44.5 g (97.5%) slightly cloudy, almost colorless oil that was 2% DMF by weight (determined by NMR). ¹H NMR (CDCl₃) δ 7.84 (d, 1H), 7.44 (dd, 1H), 7.24 (m, 4H), 7.07 (br d, 1H), 4.50 (s, 2H), 3.62 (s, 3H), 2.07 (s, 3H). MS (DCI/NH3) 336/338 (M+H+NH3)⁺.

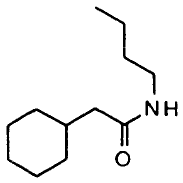

### Example 1178E

### N-butyl-N-2-cyclohexylacetamide

2-Cyclohexylacetic acid (42.66 g, 0.30 mol) was dissolved in 85 mL of thionyl chloride and the mixture heated to reflux for 2 hours. After cooling to room temperature, the yellow solution was concentrated. Toluene was added and the solution was concentrated again and the acid chloride used directly. The acid chloride was diluted with 100 mL of methylene chloride and this solution added to a biphasic mixture of butylamine (60 mL, 0.60 mol) in 100 mL of methylene chloride and 2M aqueous potassium carbonate (150 mL) and the mixture was stirred overnight at ambient temperature. An additional 30 mL of butylamine was added and stirring continued for 2 hours and then the mixture was poured into a separatory funnel. The layers were separated and the aqueous phase was extracted with 1 portion of methylene chloride and the combined organic extracts were dried, filtered and concentrated to an off white solid. This material was suspended in 400 mL of 1:1 ether/hexanes and filtered. The solid was washed with 2 additional portions of 1:1 ether/hexanes. The filtrates were extracted with 3 portions of aqueous HCl, dried, filtered and concentrated to a volume of - 200 mL. The solid that formed was collecterd by filtration and combined with the previous solid material and dried under vacuum to give the title compound (49.50 g, 88%). ¹H nmr (300 MHz., CDCl₃): δ 5.35, bs, 1H; 3.24, q, 2H; 2.02, d, 2H; 1.70, bm, 6H; 1.47, m, 2H; envelope 1.06 - 1.42, 5H; 0.91, m, 5H. MS (DCI-NH₃): 198 (MH⁺); 215 (M+NH₄⁺).

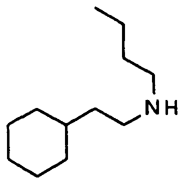

### Example 1178F

### N-butyl-N-2-cyclohexylethylamine

A stirred suspension of lithium aluminum hydride (23.74 g, 0.63 mol) in THF (400 mL) was cooled in an ice bath and treated with a solution of example 1178E (49.50 g, 0.26 mol) in THF (300 mL). The ice bath was removed and the mixture heated to gentle reflux for 20 hours. The solution was cooled in an ice bath and quenched by the careful addition of 24 mL of water in 100 mL of THF, followed by 24 mL of 15% aqueous sodium hydroxide, followed by an additional 72 mL of water. The thick slurry was vigorously stirred for 15 minutes at which time 600 mL of methylene chloride and excess sodium sulfate were sequentially added. The mixture was stirred for I hour and then filtered through celite. The celite pad was washed well with methylene chloride and the filtrate concentrated to give the title compound (47.80 g, 100%) which was sufficiently pure for the next step. ¹H nmr (300 MHz., CDCl₃): δ 2.61, m, 4H; 1.69, m, 5H; envelope 1.05 - 1.53, 11H; 0.91, m, 5H. MS (DCI-NH₃): 184 (MH⁺).

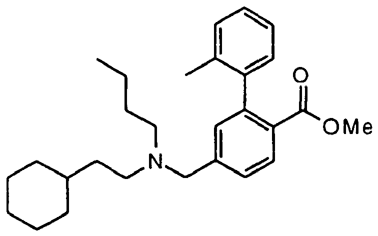

### Example 1178G

### 4-(N-(-2-cyclohexylethyl)-N-butylaminomethyl)-2-(2-methylphenyl)benzoic acid, methyl ester

A solution of example 1178D (22.2 g, 0.070mol) and diisopropylethylamine (15.7 mL, 0.090 mol) in 100 mL of acetonitrile was treated with N-butyl-N-2-cyclohexylethylamine (15.3 g, 0.084 mol). The cloudy mixture was stirred for two hours and then briefly warmed to ∼45C. After cooling to ambient temperature, the mixture was concentrated to remove the acetonitrile and then diluted with 400 mL of water. The pH of the mixture was brought to >10 with solid potassium phosphate and extracted with 3 portions of ethyl ether. The combined ether extracts were extracted with 1 portion of water and two portions of brine, dried, filtered and concentrated. The residue obtained (34.4 g, 117%) was used directly. An analytical sample was obtained by column chromatography on silica gel (3% ethyl acetate/hexanes) to provide pure material. H nmr (300 MHz., CDCl₃): δ 7.92, d, 1H; 7.48, dd, 1H; 7.16 - 7.28, m, 4H; 7.07, bd, 1H; 3.62, s, 3H; 3.57, s, 2H; 2.41, quartet, 4H; 2.06, s, 3H; 1.62, bm, 5H; envelope 1.05 - 1.48, 10H; 0.85, bm, 5H. MS (ESI+): 422 (MH⁺): (ESI-): 420 (M-H).

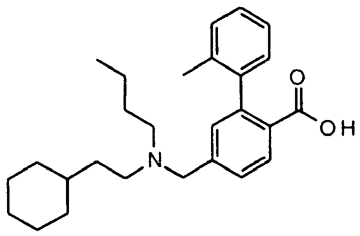

### Example 1178H

### N-[4-(N-(-2-cyclohexylethyl)-N-butylaminomethyl)-2-(2-methylphenyl)benzoic acid

A solution of 1178G (34.35 g, 0.081 mol) in 210 mL of ethanol was treated with aqueous sodium hydroxide (4N, 70 mL, 0.28 mol) and the mixture heated to reflux until judged complete by tlc analysis. After cooling to room temperature, the mixture was concentrated to remove the ethanol. The resulting solid was partially dissolved by adding water and the mixture extracted with ethyl ether. The ether layer was then washed with water and then with 1M aqueous phosphoric acid which resulted in an oily precipitate. The precipitate was dissolved by extracting with 3 portions of ethyl acetate and the combined ethyl acetate layer were washed with water, 0.5M aqueous phosphoric acid, brine and then dried, filtered and concentrated to give 24.5 g, (86% yield for the two steps) as a cream colored solid. ¹H nmr (300 MHz., CD₃OD): δ 7.96, d, 1H; 7.64, dd, 1H; 7.37, d, 1H; 7.22, m, 2H; 7.18, m, 1H; 7.07, d, 1H; 4.41, bs, 2H; 3.12, m, 4H; 2.10, s, 3H; 1.18, bm, 9H; 1.37, sextet, 2H; 1.23, m, 3H; 0.96, t, 3H; 0.94, m, 2H. MS (ESI+): 408 (MH⁺): (ESI-): 406 (M-H).

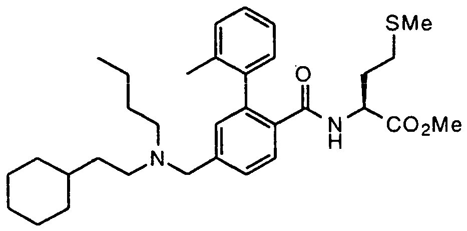

### Example 11781

### N-[4-(N-Butyl-N-(2-cyclohexylethyl)aminomethyl)-2-(2-methylphenyl)benzoyl]methionine methyl ester

Partitioned 13.2 g (66.1 mmol) L-methionine methyl ester, hydrochloride salt between saturated aqueous NaHCO₃ (80 mL) and CH₂Cl₂ (75 mL). Added the organic layer to the following solution: 24.5 g (60.2 mmol) acid from Example 1178H, 10.0 g (65.3 mmol) HOBT·H₂O, and 12.6 g (65.7 mmol) EDCI·HCl in DMF (150 mL). After stirring at RT overnight partitioned the reaction between saturated aqueous NaHCO₃ (500 mL) and EtOAc (1200 mL). The organic layer was washed with water and brine, then dried over Na₂SO₄. After filtration and concentration, recovered 30 g orange oil that was purified by chromatography using hex/EtOAc 3/1. Recovered 22.9 g (69%) of the title compound. ¹H NMR (CDCl₃) δ 7.90 (m, 1H), 7.40 (d, 1H), 7.30, 7.20, 7.16 (all m, total 5H), 5.88 (br d, 1H), 4.62 (m, 1H), 3.66 (s, 3H), 3.57 (s, 2H), 2.41 (m, 4H), 2.18, 2.13, 2.04 (s, m, m, total 9H), 1.85 (m, 1H), 1.62 (m, 5H), 1.50-1.10 (envelope, 10H), 0.87 (m, 5H). MS (APCI) 553 (M+H)+.

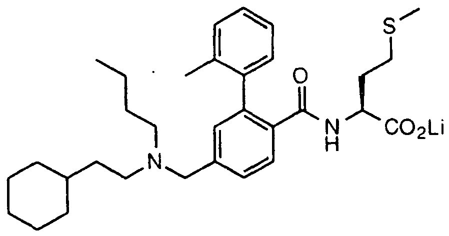

### Example 1178J

### N-[4-(N-(-2-cyclohexylethyl)-N-butylaminomethyl)-2-(2-methylphenyl)benzol]methionine, Lithium Salt

A solution of example 11781 (22.9 g, 0.041 mol), in 200 mL of 3:1 THF methanol was cooled in an ice bath and then tretaed with aqueous lithium hydroxide (1M, 83 mL, 0.083 mol) dropwise. The ice bath was removed and the mixture was stirred for 20 hours. The solution was concentrated to remove the organics and the resulting thick slurry diluted with water until a clear solution resulted (-1.2 L). The pH of the solution was carefully adjusted to pH∼5 with 1M aqueous phosphoric acid and stirred for 1 hour. The solid was collected by filtration and dried under vacuum over phosphorous pentoxide to provide 19.93 g of a cream colored solid. This material was dissolved in 200 mL of THF and treated with a solution of 1.55 g (0.037 mol) of lithium hydroxide in 75 mL of water. The mixture was stirred for 15 minutes and the THF removed under vacuum on a rotary evaporator. The mixture was diluted with 500 mL of water and lyophilized to give 20.10 g (89% overall) of the title compound. ¹H nmr (300 MHz., CD₃OD): δ 7.64, m, 1H; 7.41, d, 1H; 7.05 - 7.32, m, 5H; 4.25, m, 1H; 3.69, s, 2H; 2.52, m, 4H; 2.51, s, 1.5H (1/2 o-tolyl); 2.06, s, 1.5 H (1/2 o-tolyl); 1.98, s, 3H; 1.97, m, 1H; 1.73, m, 2H; 1.64, bm, 6H; envelope 1.04 - 1.56, 10H; 0.90, m, 5H. MS (ESI+): 539 (MH⁺): (ESI-): 537 (M-H). Calc'd for C₃₂H₄₅N₂O₃SLi·0.60 H₂O; C 69.19; H 8.38; N 5.04; Found: C 69.25; H 8.50; N 4.99.

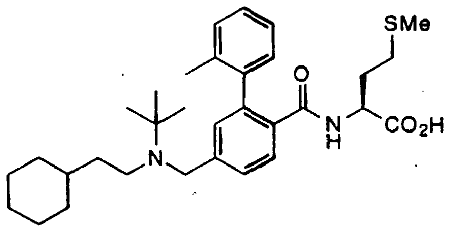

### Example 1181

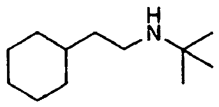

### Example 1181A

### N-t-Butyl-N-(2-cyclohexylethyl)amine

The desired amine was prepared using the method described in Example 1171A starting with cyclohexylacetic acid and t-butylamine. m/e (DCI/NH₃) 184 (MH⁺)

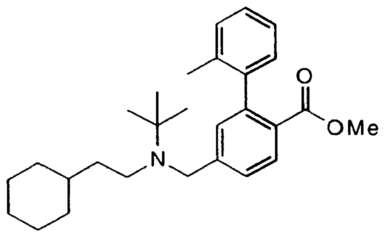

### Example 1181B

### 4-(N-t-Butyl-N-(2-cyclohexylethyl)aminomethyl)-2-(2-methylphenyl)benzoic acid methyl ester

The desired compound was prepared using the method described in Example 1178G starting with N-t-butyl-N-(2-cyclohexylethyl)amine, prepared as in Example 1181A, and 4-bromomethyl-2-(2-methylphenyl)benzoic acid methyl ester, prepared as in Example 1178A-D. m/e (ESI) 422 (MH⁺)

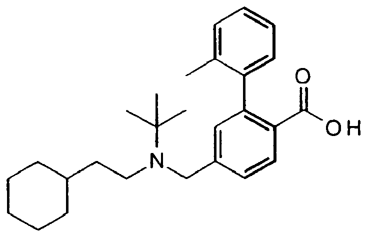

### Example 1181C

### 4-(N-t-Butyl-N-(2-cyclohexylethyl)aminomethyl)-2-(2-methylphenyl)benzoic acid

The desired acid was prepared using the method described in Example 403E starting with the compound prepared in Example 1181B.

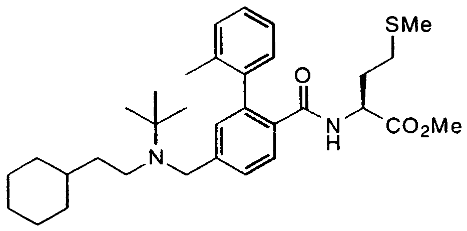

### Example 1181D

### N-[4-N-t-Butyl-N-(2-cyclohexylethyl)aminomethyl-2-(2-methylphenyl)benzoyl]methionine methyl ester

The desired product was prepared using the method described in Example 403F starting with the compound prepared inExample 1181C. m/e (ESI) 553 (MH⁺)

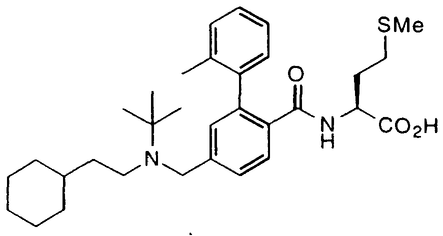

### Example 1181E

### N-[4-N-t-Butyl-N-(2-cyclohexylethyl)aminomethyl-2-(2-methylphenyl)benzoyl]methionine

The desired compound was prepared according to the method of Example 4031 starting with compound prepared in Example 1181D.¹H (300MHz, CDCl₃, δ) 7.78 (1H, m), 7.67 (1H, m), 7.40-7.00 (5H, m), 6.21 (1H, m), 4.38 (1H, m), 4.13 (2H, m), 2.93 (2H, m), 2.20-2.00 (7H, m), 2.00 (3H, s), 1.60 (4H, m), 1.43 (12H, bs), 1.40-0.90 (4H, m), 0.75 (2H, m). m/e (ESI) 537 (MH⁺) Anal.calc. for C₃₂H₄₆N₂O₃S·0.75 H₂O C 69.59, H 8.67, N 5.07 Found C 69.78, H 8.65, N 4.89

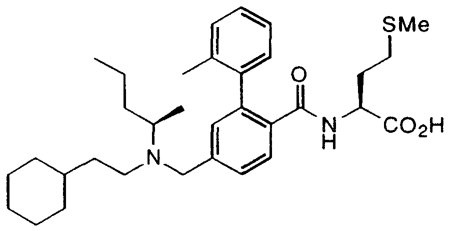

### Example 1182

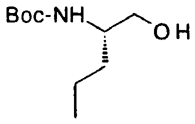

### Example 1182A

### (2S)-t-Butoxycarbonylaminopentan-1-ol

The desired product was prepared using the methods described in Example 1183A starting with L-norvaline.

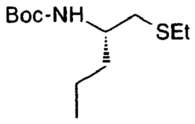

### Example 1182B

### (2S)-t-Butoxycarbonylamino-1-ethylthiopentane

The desired product was prepared using the methods described in Example 403B and 403C starting with the compound prepared in Example 1182A.

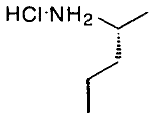

### Example 1182C

### (2R)-Aminopentane hydrochloride salt

The desired product was prepared using the methods described in Example 1183C starting with the compound prepared in Example 1182B.

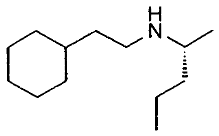

### Example 1182D

### N-(2-Cyclohexylethyl)-N-(pent-2-yl)amine

The desired amine was prepared using the method described in Example 1171A, except triethylamine was added, starting with cyclohexylacetic acid and the compound from Example 1182C. m/e (DCI) 198 (MH⁺)

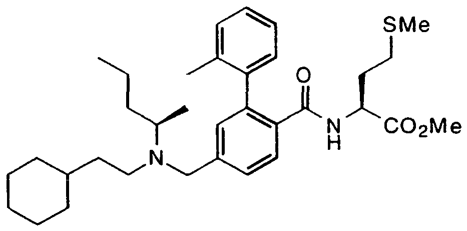

### Example 1182E

### N-[4-N-(2-Cyclohexylethyl)-N-(pent-2-yl)aminomethyl-2-(2-methylphenyl)benzoyl]methionine methyl ester

The desired product was prepared using the method described in Example 403H starting with the compound prepared in Example 1182D and N-[4-formyl-2-(2-methylphenyl)benzoyl]methionine methyl ester, prepared as in Example 403G. m/e (ESI) 567 (MH⁺)

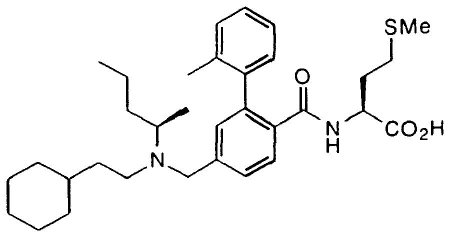

### Example 1182F

### N-[4-N-(2-Cyclohexylethlyl)-N-(pent-2-yl)aminomethyl-2-2-methylphenyl)benzoyl]methionine

The desired compound was prepared according to the method of Example 4031 starting with the compound prepared in Example 1182E.¹H (300MHz, CDCl₃, δ) 7.74 (1H, m), 7.62 (1H, m), 7.40-7.00 (5H, m), 6.46 (1H, m), 4.37 (1H, m), 3.94 (2H, m), 3.37 (1H, m), 2.90 (2H, m), 2.20-1.80 (8H, m), 1.80-1.60 (6H, m), 1.55-1.25 (6H, m), 1.25-1.00 (8H, m), 0.91 (3H, t, J=8Hz), 0.82 (2H, m). m/e (ESI) 551 (MH⁻) Anal.calc. for C33H₄₈N₂O₃S·050 H₂O C 70.55, H 8.79, N 4.99 Found C 70.55, H 8.71, N 4.87

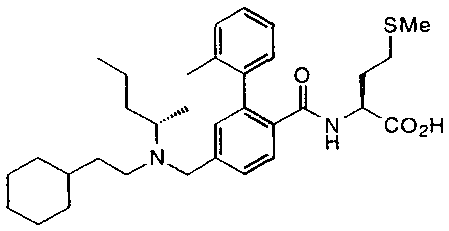

### Example 1183

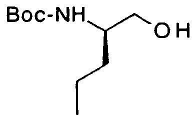

### Example 1183A

### (2R)-t-Butoxycarbonylaminopentan-1-ol

To a stirred solution at ambient temperature of D-norvaline (5.00 g, 42.7 mmol) in THF (100 mL) was added an aqueous 4N NaOH solution (21 mL, 84 mmol), di-t-butyl dicarbonate (11.2 g, 51.2 mmol), and tetrabutylammonium bromide (1.0 g). Two-phase solution stirred overnight at ambient temperature. Reaction neutralized with aqueous 3N HCl to pH 6 and extracted with CHCl₃ (3x). Extracts dried with Na₂SO₄, filtered, and concentrated in vacuo to produce a colorless oil. To a stirred solution at 0°C under N₂ of the crude residue in anhydrous THF (80 mL) was added dropwise via addition funnel a 1.0M borane-THF complex (100 mL, 100 mmol) in THF. After stirring overnight at ambient temperature, reaction cooled back to 0°C and quenched with an aqueous 4N NaOH solution (50 mL). Mixture stirred one hour at ambient temperature, and then, extracted with CH₂Cl₂ (3x). Extracts dried with Na₂SO₄, filtered, and concentrated in vacuo. Residue purified by flash chromatography on silica gel eluting with 30% EtOAc/Hexanes to afford the alcohol as a pale yellow oil (3.87 g, 45%). m/e (DCI) 204 (MH⁺)

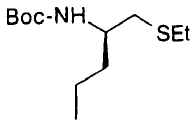

### Example 1183B

### (2R)-t-Butoxycarbonylamino-1-ethylthiopentane

The desired product was prepared using the methods described in Example 403B and 403C starting with the compound prepared in Example 1183A. m/e (DCI) 248 (MH⁺)

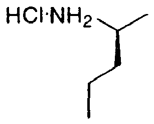

### Example 1183C

### (2S)-Aminopentane hydrochloride salt

To a stirred solution at ambient temperature of (2R)-t-butoxycarbonylamino-1-ethylthiopentane (655 mg, 2.65 mmol), prepared as in Example 1183B, in EtOH (5 mL) was added a 50% slurry of Raney Nickel (2.65 g) in water. Mixture stirred vigorously at 80°C for 2 days. Reaction filtered through celite, and celite and catalyst washed with EOAc. Filtrate concentrated in vacuo to produce a colorless liquid. Residue taken up in a solution of 4N HCl in dioxane (5 mL), and reaction stirred overnight at ambient temperature. Ether added until a solid precipitated. Solid filtered off, washed with ether, and dried to produce the desired compound as a white solid (167 mg, 59%).

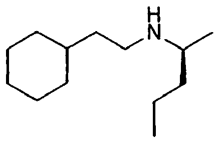

### Example 1183D

### N-(2-Cyclohexylethyl)-N-(pent-2-yl)amine

The desired amine was prepared using the method described in Example 1171A. except triethylamine was added, starting with cyclohexylacetic acid and the compound from Example 1183C. ¹H NMR (CDCl₃, 300 MHz) δ 2.70-2.50 (m, 4H), 1.80-1.60 (m, 6H), 1.50-1.00 (m, 8H), 1.04 (d, 3H, J=8Hz), 1.00-0.80 (m, 5H)

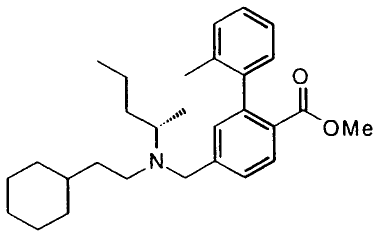

### Example 1183E

### N-[-N-(2-Cyclohexylethyl)-N-(pent-2-yl)aminomethyl-2-(2-methyl)benzoic acid methyl ester

The desired compound was prepared using the method described in Example 1178G starting with N-(2-cyclohexylethyl)-N-(1-methylbutyl)amine, prepared as in Example 1183D, and 4-bromomethyl-2-(2-methylphenyl)benzoic acid methyl ester, prepared as in Example 1178A-D. m/e (ESI) 436 (MH⁺)

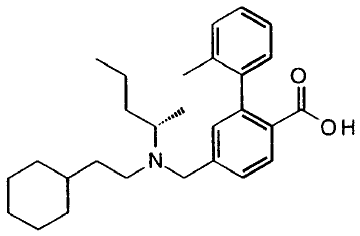

### Example 1183F

### N-[4-N-(2-Cyclohexylethyl)-N-(pent-2-yl)aminomethyl-2-(2-methylphenyl)benzoic acid

The desired acid was prepared using the method described in Example 403E starting with the compound prepared in Example 1183E.

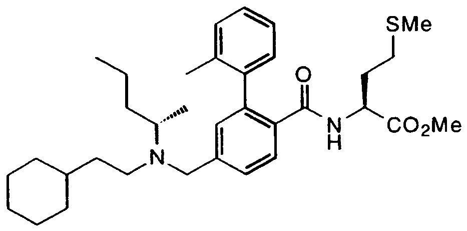

### Example 1183G

### N-[4-N-(2-Cyclohexylethyl)-N-(pent-2-yl)methyl-2-(2-methylphenyl)benzoyl]methionine methyl ester

The desired product was prepared using the method described in Example 403F starting with the compound prepared in Example 1183F. m/e (ESI) 567 (MH⁺)

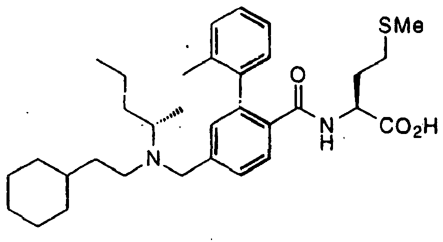

### Example 1183H

### N-[4-N-(2-Cyclohexylethyl)-N-(pent-2-yl)aminomethyl-2-(2-methylphenyl)benzoyl]methionine

The desired compound was prepared according to the method of Example 4031 starting with the compound from Example 1183G.¹H (300MHz, CDCl₃, δ 7.69 (2H, m), 7.40-7.00 (5H, m), 6.46 (1H, m), 4.38 (1H, m), 4.05 (2H, m), 3.41 (1H, m), 2.90 (2H, m), 2.20-1.75 (9H, m), 1.75-1.50 (7H, m), 1.50-1.00 (12H, m), 0.90 (5H, m). m/e (ESI) 551 (MH⁻) Anal.calc. for C₃₃H₄₈N₂O₃S·0.50 H₂O C 70.55, H 8.79, N 4.99 Found C 70.65, H 8.63, N 4.93

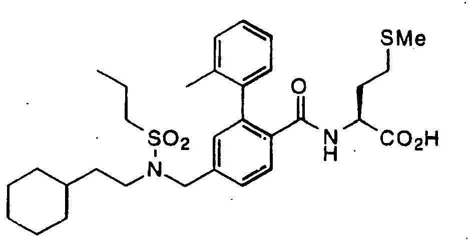

### Example 1187

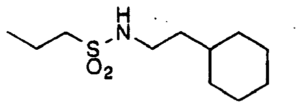

### Example 1187A

### N-(2-Cyclohexylethyl)-N-propanesulfonylamine

The desired product was prepared using the method described in Example 1174A starting with cyclohexylethylamine and 1-propanesulfonyl chloride.

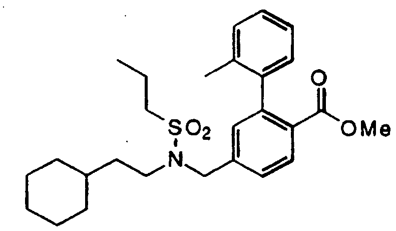

### Example 1187B

### 4-(N-(2-Cyclohexylethyl)-N-propanesulfonylaminomethyl)-2-(2-methylphenyl)benzoic acid methyl ester

The desired product was prepared using the method described in Example 1174B starting with N-(2-cyclohexylethyl)-N-propanesulfonylamine, prepared as in Example 1187A, and 4-bromomethyl-2-(2-methylphenyl)benzoic acid methyl ester, prepared as in Example 1178A-D, m/e (ESI) 472 (MH⁺)

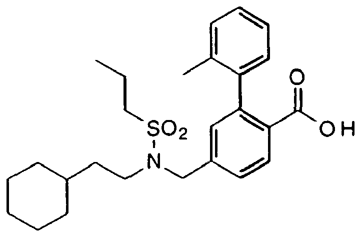

### Example 1187C

### 4-(N-(2-Cyclohexylethyl)-N-propanesulfonylamino-2-(2-methylphenyl)benzoic acid

The desired acid was prepared using the method described in Example 403E starting with the product from Example 1187B.

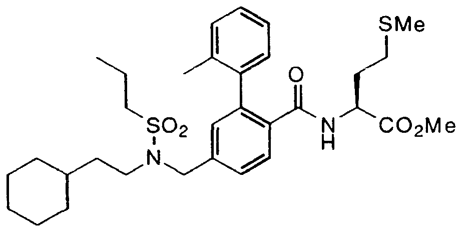

### Example 1187D

### N-[4-N-(2-Cyclohexylethyl)-N-propanesulfonylaminomethyl-2-(2-ethylphenyl)benzoyl]methionine methyl ester

The desired compound was prepared using the method described in Example 403F starting with the product from Example 1187C. m/e (ESI) 601 (MH⁻)

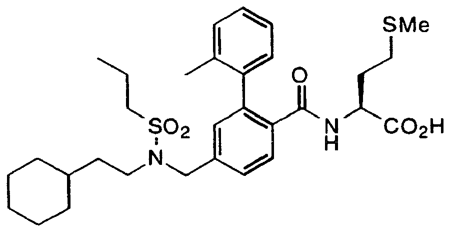

### Example 1187E

### N-[4-N-(2-Cyclohexylethyl)-N-propanesulfonylaminomethyl-2-(2-methylphenyl)benzoyl]methionine

The desired compound was prepared according to the method of Example 4031 starting with the compound prepared in Example 1187D.¹H (300MHz, CDCl₃, δ) 8.00 (1H, dd, J=8&7Hz), 7.43 (1H, dd, J=7&2Hz), 7.40-7.10 (5H, m), 5.90 (1H, m), 4.58 (1H, m), 4.42 (2H, s), 3.20 (2H, m), 2.94 (2H, m), 2.20-2.00 (7H, m), 2.00-1.80 (4H, m), 1.60 (6H, m), 1.38 (2H, m), 1.15 (4H, m), 1.05 (3H, t, J=8Hz), 0.86 (2H, m). m/e (ESI) 587 (MH⁻) Anal.calc. for C₃₁H₄₄N₂O₅S2·0.25 H₂O C 62.75, H 7.56, N 4.72 Found C 62.75, H 7.56, N 4.49

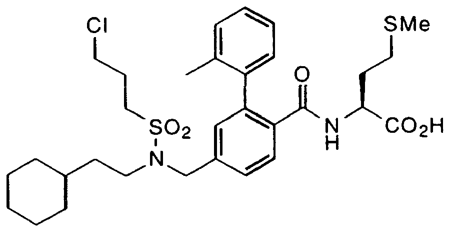

### Example 1188

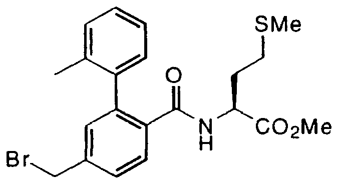

### Example 1188A

### -[Bromomethyl-2-(2-methylphenyl)benzoyl]methionine methyl ester

To a stirred solution at -10°C under N₂ of N-[4-hydroxymethyl-2-(2-methylphenyl)benzoyl]methionine methyl ester (200 mg, 0.517 mmol), prepared as in Example 403F, and carbon tetrabromide (189 mg, 0.568 mmol) in CH₂Cl₂ (5 mL) was added triphenylphosphine (163 mg, 0.620 mmol). Reaction stirred one hour at -10°C, and then, solvents concentrated in vacuo to produce a colorless glass. The residue could not be stored, and so, was used directly in the reaction in Example 1188B.

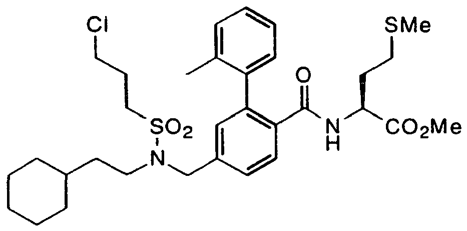

### Example 1188B

### N-[4-N-(3-Chloropropanesulfonyl)-N-(2-cyclohexylethyl)aminomethyl-2-(2-methylphenyl)benzoy_llmethionine methyl ester

The desired compound was prepared using the method described in Example 174B (except reaction run at -40°C) starting with the product from Example 188A and N-(3-chloropropanesulfonyl)-N-(2-cyclohexylethyl)amine, prepared as in Example 1189A using the method described in Example 1174A. m/e (ESI) 635 (MH⁻)

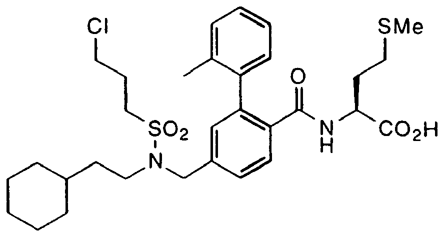

### Example 1188C

### N-[4-N-(3-Chloropropanesulfonyl)-N-(2-cyclohexylethyl)aminomethyl-2-(2-methylphenyl)benzoyl]methionine

The desired compound was prepared according to the method of Example 4031 starting with the compound from Example 1188B. H (300MHz, CDCl₃, δ) 8.01 (1H, bt, J=8Hz), 7.46 (1H, dd, J=7&2Hz), 7.40-7.10 (5H, m), 5.90 (1H, m), 4.59 (1H, m), 4.45 (2H, s), 3.68 (2H, t, J=8Hz), 3.22 (2H, bt, J=7Hz), 3.12 (2H, t, J=8Hz), 2.31 (2H, m), 2.20-2.05 (4H, m), 2.03 (3H, s), 1.92 (2H, m), 1.60 (6H, m), 1.40 (2H, m), 1.30-1.00 (4H, m), 0.85 (2H, m). m/e (ESI) 621 (MH⁻) Anal.calc. for C₃₁H₄₃Cl₁N₂O₅S₂·0.50 H₂O C 58.89, H 7.01, N 4.43 Found C 58.96, H 7.04, N 4.40

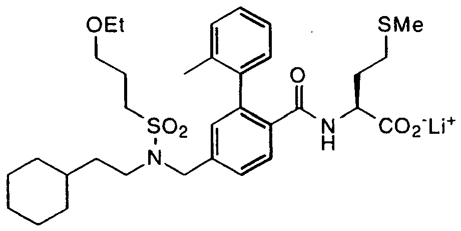

### Example 1189

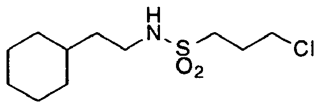

### Example 1189A

### N-(3-Chloropropanesulfonyl)-N-(2-cyclohexylethyl)amine

The desired compound was prepared using the method described in Example 1174A starting with cyclohexylethylamine and 3-chloropropanesulfonyl chloride.

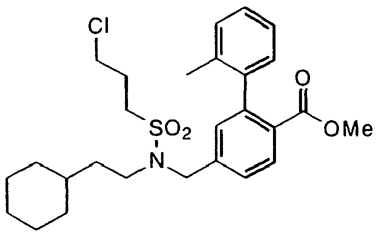

### Example 1189B

### -N-(3-Chloropropanesulfonyl)-N-(2-cyclohexyl)aminomethyl-2-(2-ethylphenyl)benzoic acid methyl ester

The desired product was prepared using the method described in Example 1174B starting with the compound from Example 1189A and 4-bromomethyl-2-(2-methylphenyl)benzoic acid methyl ester, prepared as in Example 1178A-D. m/e (ESI) 506 (MH⁺)

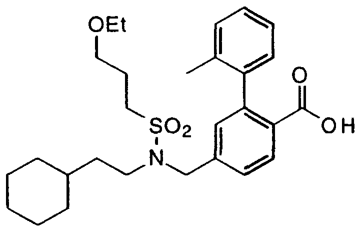

### Example 1189C

### N-[4-N-(2-Cyclohexylethyl)-N-(3-ethoxypropanesulfonyl)aminomethyl-2-(2-methylphenyl)benzoic acid

The acid was prepared using the method described in Example 403E starting with the product from Example 1189B. Chloride was displaced by ethoxide ion.

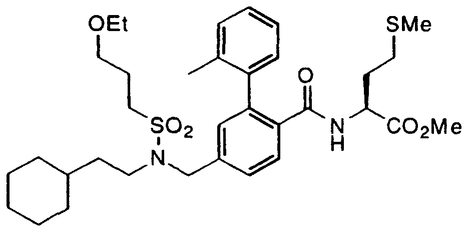

### Example 1189D

### -[4-N-(2-Cyclohexylethyl)-N-(3-ethoxypropanesulfonyl)aminomethyl-2-(2-methylphenyl)benzoyl]methionine methyl ester

The compound was prepared using the method described in Example 403F starting with the product from Example 1189C. m/e (ESI) 645 (MH⁻)

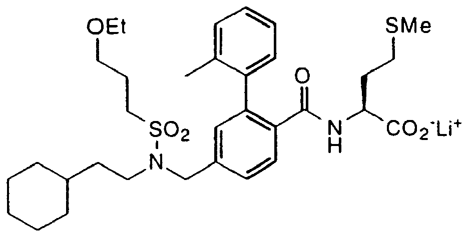

### Example 1189E

### N-[4-N-(2-Cyclohexylethyl)-N-(3-ethoxypropanesulfonyl)aminomethyl-2-(2-methylphenyl)benzoyl]methionine lithium salt

The desired compound was prepared according to the method of Example 403I starting with the compound from Example 1189D. ¹H (300MHz, DMSO-d6, δ) 7.54 (1H, d, J=8Hz), 7.41 (1H, dd, J=7&2Hz), 7.30-7.10 (5H, m), 6.97 (1H, d, J=7Hz), 4.42 (2H, bs), 3.68 (1H, m), 3.43 (2H, q, J=7Hz), 3.40 (2H, m), 3.16 (4H, m), 2.20-1.95 (5H, m), 1.95 (3H, s), 1.90-1.65 (3H, m), 1.55 (6H, m), 1.27 (2H, m), 1.10 (7H, bt, J=8Hz), 0.78 (2H, m). m/e (ESI) 631 (MH⁻) Anal.calc. for C₃₃H₄₇LiN₂O₆S₂·0.50 H₂O C 61.18, H 7.47, N 4.32 Found C 61.15, H 7.53, N 4.15

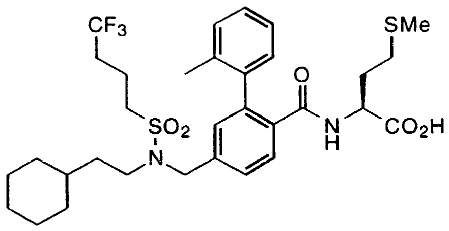

### Example 1190

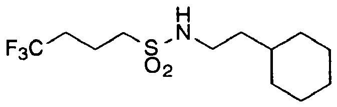

### Example 1190A

### N-(2-Cyclohexylethyl)-N-(3-trifluoromethylpropanesulfonyl)amine

To a stirred solution at 0°C under N2 of 4,4,4-trifluoro-1-bromobutane (2.00 g. 10.5 mmol) in anhydrous DMF (10 mL) was added dropwise a slurry of t-butanethiol sodium salt (1.29 g, 11.5 mmol) in anhydrous DMF (25 mL) such that the temperature was maintained below 5°C. Mixture stirred one hour at 0°C, and then, diluted with water and extracted with ether. Extracts dried with Na₂SO₄, filtered, and concentrated in vacuo. Residue dissolved in 1:1 water/EtOH at 0°C, and to this was bubbled in chlorine gas for 45 minutes. After the chlorine addition, N₂ was bubbled into the black-green mixture until the green color disappeared (30 minutes). The mixture was made a more homogeneous solution by addition of CH₂Cl₂, and to this was added carefully an aqueous 2M Na₂CO₃ solution until mixture was basic (pH 10). Cyclohexylethylamine (1.14 g, 9.00 mmol) was added, and this two-phase solution was stirred at room temperature overnight. Reaction diluted with water and extracted with CHCl₃ (2x). Extracts dried with Na₂SO₄, filtered, and concentrated. Residue purified by flash chromatography on silica gel eluting with 20% EtOAc/Hexanes to afford the desired product as a light brown oil (1.02 g, 32%). m/e (DCl) 319 (MH+NH₃⁺)

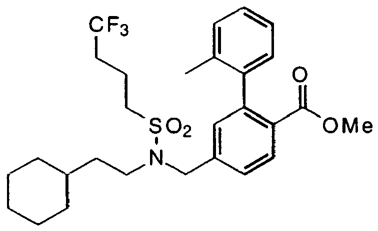

### Example 1190B

### 4-(N-(2-Cyclohexylethyl)-N-(3-trifluoromethylpropanesulfonyl)aminomethyl)-2-(2-methylphenyl)benzoic acid methyl ester

The desired product was prepared using the method described in Example 1174B starting with N-(2-cyclohexylethyl)-N-(3-trifluoromethylpropanesulfonyl)amine, prepared as in Example 1190A, and 4-bromomethyl-2-(2-methylphenyl)benzoic acid methyl ester, prepared as in Example 1178A-D.

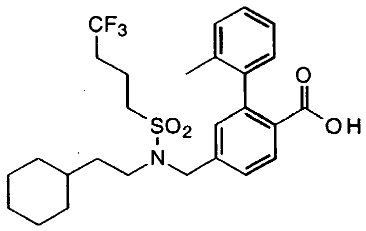

### Example 1190C

### 4-(N-(2-Cyclohexylelthyl)-N-(3-trifluoromethylpropanesulfonyl)aminomethyl)-2-(2-methylphenyl)benzoic acid

The desired acid was prepared using the method described in Example 403E starting with the product from Example 1190B. m/e (ESI) 524 (MH⁻)

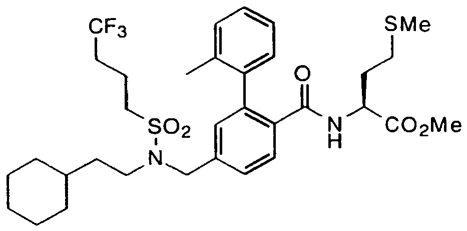

### Example 1190D

### N-[4-N-(2-Cyclohexylethyl)-N-(3-trifluoromethylpropanesulfonyl)aminomethyl-2-(2-methylphenyl)benzoyl]methionine methyl ester

The desired compound was prepared using the method described in Example 403F starting with the product from Example 1190C. m/e (ESI) 669 (MH⁻)

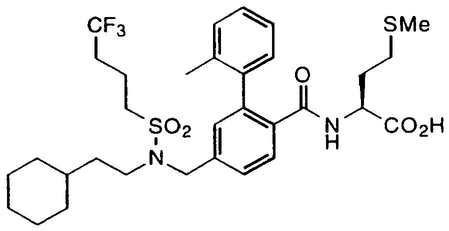

### Example 1190E

### N-[4-N-(2-Cyclohexylethyl)-N-(3-trifluoromethylpropanesulfonyl)aminomethyl-2-(2-methylphenyl)benzoyl]methionine

The desired compound was prepared according to the method of Example 4031 starting with the compound in Example 1190D. ¹H (300MHz, CDCl₃, δ) (rotamer) 8.01 (7.98) (1H, d, J=8Hz), 7.46 (1H, dd, J=7&2Hz), 7.40-7.10 (5H, m), 5.92 (1H, m), 4.80 (1H, bs), 4.58 (1H, m), 4.45 (2H, s), 3.22 (2H, bt, J=7Hz), 3.03 (2H, t, J=8Hz), 2.30 (2H, m), 2.20-2.00 (10H, m), 1.92 (1H, m), 1.62 (6H, m), 1.40 (2H, m), 1.30-1.00 (4H, m), 0.87 (2H, m). m/e (ESI) 655 (MH⁻) Anal.calc. for C₃₂H₄₃F₃N₂O₅S₂ C 58.52, H 6.60, N 4.26 Found C 58.27, H 6.63, N 4.13

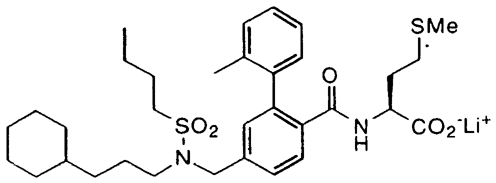

### Example 1191

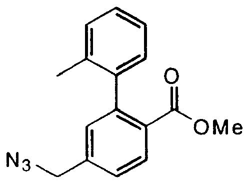

### Example 1191A

### 4-Azidomethyl-2-(2-methylphenyl)benzoic acid methyl ester

To a stirred mixture at 0°C under N₂ of sodium azide (1.47 g, 22.6 mmol) in anhydrous DMF (30 mL) was added a solution of 4-bromomethyl-2-(2-methylphenyl)benzoic acid methyl ester (6.00 g, 18.8 mmol), prepared as in Example 1178A-D, in anhydrous DMF (10 mL). Reaction stirred overnight at room temperature. Reaction diluted with EtOAc and washed with water and brine. Organic layer dried with Na₂SO₄, filtered, and concentrated in vacuo.

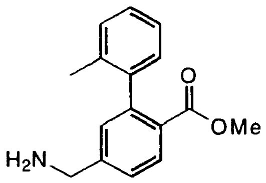

### Example 1191B

### 4-Aminomethyl-2-(2-methylphenyl)benzoic acid methyl ester

To a flask at ambient temperature under N₂ containing 10% palladium on carbon catalyst (1.0 g) was added a solution of 4-azidomethyl-2-(2-methylphenyl)benzoic acid methyl ester (5.00 g, 17.8 mmol), prepared as in Example 1191A, in MeOH (75 mL). Two drops of conc. HCl added, and reaction stirred vigorously overnight under an atmosphere of H₂. Catalyst filtered off through celite and washed with MeOH. Filtrate concentrated in vacuo, and residue taken up in an aqueous 4N NaOH solution. Aqueous solution extracted with CHCl₃ (3x), and extracts dried with Na₂SO₄ filtered, and concentrated in vacuo to afford the desired product (1.37 g, 30%). m/e (DCI) 256 (MH⁺)

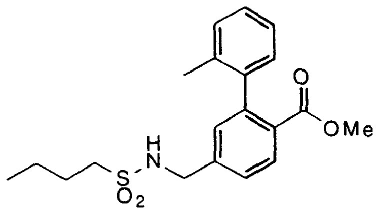

### Example 1191C

### 4-N-Butanesulfonylminomethyl-2-(2-methylphenyl)benzoic acid methyl ester

The desired compound was prepared using the method described in Example 1174A starting with 4-aminomethyl-2-(2-methylphenyl)benzoic acid methyl ester, prepared as in Example 1191B, and butanesulfonyl chloride. m/e (ESI) 374 (MH⁻)

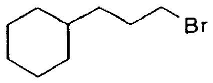

### Example 1191D

### 1-Bromo-3-cyclohexylpropane

The desired compound was prepared according to the method of Example 1178D starting with 3-cyclohexyl-1-propanol. ¹H (300MHz, CDCl₃, δ) 3.40 (2H, t, J=8Hz), 1.85 (2H, m), 1.80-1.50 (6H, m), 1.40-1.10 (5H, m), 0.90 (2H, m).

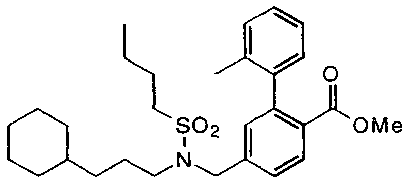

### Example 1191E

### N-[4-N-(Butanesulfonyl)-N-(3-cyclohexylpropyl)aminomethyl-2-(2-methylphenyl)benzoic acid methyl ester

The desired compound was prepared using the method described in Example 1174B starting with the compounds from Example 1191C and Example 1191D. m/e (ESI) 500 (MH⁺)

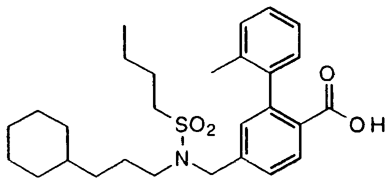

### Example 1191F

### N-[4-N-(Butanesulfonyl)-N-(3-cyclohexylpropyl)aminomethyl-2-(2-methylphenyl)benzoic acid

The acid was prepared using the method described in Example 403E starting with the compound from Example 1191 E.

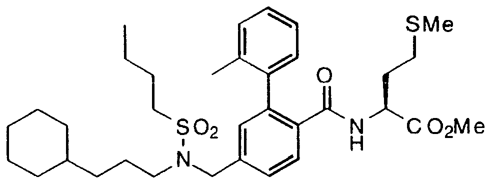

### Example 1191G

### N-[4-N-(Butanesulfonyl)-N-(3-cyclohexylpropyl)aminomethyl-2-(2-methylphenyl)benzoyl]methionine methyl esther

The compound was prepared using the method described in Example 403F starting with the compound from Example 1191F. m/e (ESI) 629 (MH⁻)

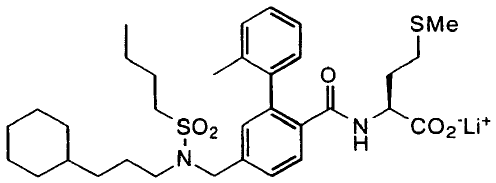

### Example 1191H

### N-[4-N-(Butanesulfonyl)-N-(3-cyclohexylpropyl)aminomethyl-2-(2-methylphenyl)benzoyl]methionine lithium salt

The desired compound was prepared according to the method of Example 4031 starting with the compound from Example 1191G.¹H (300MHz, DMSO-d6, δ) 7.54 (1H, d, J=8Hz), 7.41 (1H, bd, J=7Hz), 7.30-7.05 (5H, m), 6.97 (1H, d, J=7Hz), 4.42 (2H, s), 3.68 (1H, m), 3.10 (4H, bt, J=7Hz), 2.20-1.95 (5H, m), 1.91 (3H, s), 1.90-1.45 (9H, m), 1.45-1.20 (4H, m), 1.20-0.90 (6H, m), 0.88 (3H, t, J=8Hz), 0.73 (2H, m). m/e (ESI) 615 (MH⁻) Anal.calc. for C₃₃H₄₇LiN₂O₅S₂·0.75 H₂O C 62.29, H 7.68, N 4.40 Found C 62.18, H 7.75, N 4.36

## Claims

1. A compound having Formula I 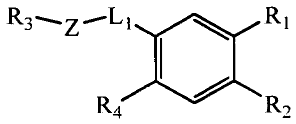 or a pharmaceutically acceptable salt thereof, wherein R₁ is 2-methylphenyl;
R₂ is -L₁₁ - C(R₁₄)(Rᵥ) C(O)OR₁₅, wherein L₁₁ is -C(W)N(R) - wherein R is hydrogen and W is O, Rᵥ is selected from the group consisting of hydrogen and lower alkyl, R₁₅ is selected from the group consisting of (a) hydrogen, (b) alkanoyloxyalkyl, (c) C₁-to-C₁₀ alkyl and (d) a carboxy-protecting group, and R₁₄ is thioalkoxyalkyl;
L₁ is -L₄-N(R₅)-L₅- wherein L₄ is absent or C₁-to-C₁₀-alkylene, L₅ is absent or C₁-to-C₁₀-alkylene, and R₅ is selected from the group consisting of hydrogen, alkanoyl, alkoxy, alkoxyalkyl, alkoxycarbonyl wherein the alkoxycarbonyl is unsubstituted or substituted with 1, 2 or 3 halogens, alkylaminocarbonylalkyl, cycloalkoxycarbonyl, cycloalkylaminocarbonyl, cycloalkylaminothiocarbonyl, cycloalkylalkyl, (cyclolalkyl)oyl, haloalkyl, C₁-to-C₁₀ alkyl, wherein the C₁-to-C₁₀ alkyl is unsubstituted or substituted with -NRR' , wherein R and R' are independently: hydrogen; C₁-to-C₁₀ alkyl;- SO₂-A, wherein A is C₁-to-C₁₀ alkyl optionally substituted with alkoxy or 1-5 halogen(s); or thioalkoxyalkyl;
Z is a covalent bond;
R₃ is cycloalkyl, wherein the cycloalkyl is unsubstituted or substituted with substituents selected from the group consisting of (a) alkoxy, (b) C₁-to-C₁₀ alkyl, and (c) halogen;
and R₄ is hydrogen; selected from the group consisting of:
N-[4-N-(1-cyclohexyl-6-methylheptan-2-yl)aminomethyl-2-(2-methylphenyl)benzoyl]methionine, lithium salt,
N-[4-(N-2-cyclohexylethylaminomethyl)-2-(2-methylphenyl)benzoyl]-methionine, trifluoroacetate salt,
N-[4-(N-(2-cyclohexylethyl)-N-methylaminomethyl)-2-(2-methylphenyl)-benzoyl]methionine, lithium salt,
N-[4(N-acetyl-N-(2-cyclohexylethyl)aminomethyl)-2-(2-methylphenyl)-benzoyl]methionine, lithium salt,
N-[4-(N-(N,N-dimethylamino(carbonyl)-N-(2-cyclohexylethyl-aminomethyl)-2-(2-methylphenyl)benzoyl]methionine,
N-[4-(N-(2-cyclohexylethyl)-N-methanesulfonylaminomethyl)-2-(2-methylphenyl)benzoyl]methionine, lithium salt,
N-[4-(3-cyclohexylpropan-2-ylaminomethyl)-2-(2-methylphenyl)benzoyl]-methionine,
N-[4-(4-cyclohexylbutan-3-ylaminomethyl)-2-(2-methylphenyl)benzoyl]-methionine, lithium salt,
N-[4-(6-cyclohexylhexan-5-y[aminomethyl)-2-(2-methy[phenyl)-benzoyl]methionine, lithium salt,
N-[4-3-cyclohexyl-1-t-butylthiopropan-2-ylaminomethyl)-2-(2-methylphenyl)-benzoyl]methionine, lithium salt,
N-[4-N-t-butyloxycarbonyl-N-2-cyclohexylethylaminomethyl-2-(2-methylphenyl)benzoyl]methionine, lithium salt,
N-[4-(N-2-cyclohexylethyl-N-cyclopropylaminomethyl)-2-(2-methylphenyl)benzoyl]methionine,
N-[4-(N-(2-cyclohexylethyl)-N-isopropylaminomethyl)-2-(2-methylphenyl)-benzoyl] methio nine,
N-[4-(N(butanesulfonyl-N-(2-cyclohexylethyl)aminomethyl)-2-(2-methylphenyl)benzoyl]methionine,
N-[4-(N-methyl-N-(1,1-dimethyl-2-cyclohexylethyl)aminomethyl)-2-(2-methylphenyl)-benzoyl]methionine, lithium salt,
N-[4-(N-cyclohexylmethylaminoethyl)-2-(2-methylphenyl)benzoyl]methionine, lithium salt,
N-[4-(N,N-di-(cyclohexylmethyl)aminoethyl)-2-(2-methylphenyl)benzoyl]-methionine, lithium salt,
N-[4-(N-cyclohexylmethyl-N-1-adamantanoylaminoethyl)-2-(2-methylphenyl)-benzoyl]methionine, lithium salt,
N-[4-(N-cyclohexylmethyl-N-t-butoxycarbonylaminoethyl)-2-(2-methylphenyl)-benzoyl]methionine, lithium salt,
N-[4-(N-cyclohexylmethyl-N-2-ethylhexyloxycarbonylaminoethyl)-2-(2-methylphenyl]benzoyl]methionine, lithium salt,
N-[4-(N-cyclohexylmethyl-N-2,2,2-trichloroethoxycarbonylaminoethyl)-2-(2-methylphenyl)benzoyl]methionine, lithium salt,
N-[4-(N-cyclohexylmethyl-N-cyclohexyloxycarbonylaminoethyl)-2-(2-methylphenyl)benzoyl]methionine, lithium salt,
N-[4-(N-cyclohexylmethyl-N-adamantyloxycarbonylaminoethyl)-2-(2-methylphenyl)benzoyl]methionine, lithium salt,
N-[4-(N-cyclohexylmethyl-N-adamant-1-aminocarbonylaminoethyl)2-(2-methylphenyl)benzoyl]methionine, lithium salt,
N-[4-(N-cyc[ohexylmethy[-N-adamant-1-aminothiocarbony[aminoethyl)-2-(2-methylphenyl)benzoyl]methionine, lithium salt,
N-[4-(N-(2-cyclohexyl-2-methylpropyl)-N-methylaminomethyl)-2-(2-methylphenyl)benzoyl]methionine, lithium salt,
N-[4-(N-(2-cyclohexylethyl)-N-2-fluoroethylaminomethyl)-2-(2-methylphenyl)benzoyl]methionine,
N-[4-(N-(2-cyclohexylethyl)-N-2,2,2-trifluoroethylaminomethyl)-2-(2-methylphenyl)benzoyl]methionine, lithium salt,
N-[4-(N-(2-cyclohexylethyl)-N-2-methoxyethylaminomethyl)-2-(2-methylphenyl)benzoyl]methionine,
N-[4(N-(2-cyclohexylethyl)-N-2-methylthioethylaminomethyl)-2-(2-methylphenyl)benzoyl]methionine,
N-[4-(N-(2-cyclohexylethyl)-N-1-methyl-2(S)-methylthioethylaminomethyl)-2-(2-methylphenyl)benzoy]methionine,
N-[4-(N-(2-cyclohexylethyl)-N-2,N,N-dimethylaminomethyl)-2-(2-methylphenyl)benzoyl]methionine,
N-[4-(N-cyclohexylmethyl-N-1)butylaminoethyl)-2-(2-methylphenyl)benzoyl]-methionine, lithium salt,
N-[4-(N-cyclohexylpropyl)-2-(2-methylphenyl)benzoyl]methionine, lithium salt,
N-[4-(N-cyclohexyl-N-propanoylaminopropyl)-2-(2-methylphenyl)benzoyl]-methionine,
N-[4-(N-cyclohexyl-N-butylaminopropyl)-2-(2-methylphenyl)benzoyl]methionine, lithium salt,
3-[4-(N-cyclohexyl-N-methylaminomethyl)-2-(2-methylphenyl)benzoyl-methyl]4-methylthiobutyric acid,
N-[4-(N-(-2-cyclohexylethyl)-N-butylaminomethyl)-2-(2-methylphenyl)benzoyl]methionine, lithium salt,
N-[4-N-t-butyl-N-(2-cyclohexylethyl)aminomethyl-2-(2-methylphenyl)benzoyl]methionine,
N-[4-N-(2-cyclohexylethyl)-N-(pent-2-yl)aminomethyl-2-(2-methylphenyl)benzoyl]methionine,
N-[4-N-(2-cyclohexylethyl)-N-(pent-2-yl)aminomethyl-2-(2-methylphenyl)]benzoyl] methio nine,
N-[4-N-(2-cyclohexylethyl)-N-[propyloxyaminomethyl-2-(2-methylphenyl)benzoyl]methionine, lithium salt,
N-[4-N-(2-cyclohexylethyl)-N-propanesulfonylaminomethyl-2-(2-methylphenyl)benzoyl]methionine,
N-[4-N-(3-chloropropanesulfonyl)-N-(2-cyclohexylethyl)aminomethyl-2-(2-methylphenyl)benzoyl] methio nine,
N-[4-N-(2-cyclohexylethyl)-N-3-ethoxypropanesulfonyl)-aminomethyl-2-(2-methylphenyl)benzoyl]methionine, lithium salt,
N-[4-N-(2-cyclohexylethyl)-N-(3-trifluoromethylpropanesulfonyl)-aminomethyl-2-(2-methylphenyl)benzoyl]methionine,
N-[4-N-(butanesulfonyl)1-N-(3-cyclohexylpropyl)aminomethyl-2-(2-methylphenyl)benzoyl]methionine, lithium salt,
N-[4-(N-(2-cyclohexylethyl)-N-methylaminomethyl)-2-phenylbenzoyl]methionine, lithium salt.

2. A compound according to claim 1 which is N-[4-N-(-2-cyclohexylethyl)-N-butylaminomethyl)2-(2-methylphenyl)-benzoyl]methionine, lithium salt.

3. A compound in accordance with claim 1 for use in the inhibition of protein isoprenyltransferases in mammals.

4. A compound in accordance with claim 1, alone or in combination with another chemotherapeutic agent, for use in the inhibition or treatment of cancer in a mammal.

5. A composition for use in the treatment of cancer comprising a compound of claim 1 in combination with another chemotherapeutic agent and a pharmaceutically acceptable carrier.

6. A compound in accordance with claim 1 for use in inhibiting post-translational modification of the oncogenic Ras protein by protein farnesyltransferase, protein geranylgeranyltransferase, or both, in a mammal.

7. A composition for use in inhibiting post-translation modification of the oncogenic Ras protein by protein farnesyltransferase, protein geranylgeranyltransferase, or both, comprising a compound of claim 1 in combination with a pharmaceutical carrier.

8. A compound in accordance with claim 1 for use in the treatment of intimal hyperplasia associated with restenosis and atherosclerosis in a mammal.

9. A composition for use in treating restenosis in a mammal comprising a compound of claim 1 in combination with a pharmaceutically acceptable carrier.

## Patentansprüche

1. Verbindungen der Formel I 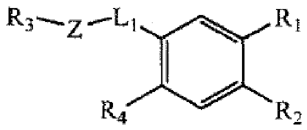
und deren pharmazeutisch unbedenkliche Salze, wobei R₁ für 2-Methylphenyl steht;
R₂ für -L₁₁-C(R₁₄) (Rᵥ)C(O)OR₁₅ steht, wobei L₁₁ für -C(W)N(R)- steht, wobei R für Wasserstoff steht und W für O steht, Rᵥ aus der aus Wasserstoff und Niederalkyl bestehenden Gruppe ausgewählt ist, R₁₅ aus der aus (a) Wasserstoff, (b) Alkanoyloxyalkyl, (c) C₁-C₁₀-Alkyl und (d) einer Carboxylschutzgruppe bestehenden Gruppe ausgewählt ist und R₁₄ für Thioalkoxyalkyl steht;
L₁ für -L₄-N(R₅)-L₅- steht, wobei L₄ fehlt oder für C₁-C₁₀-Alkylen steht, L₅ fehlt oder für C₁-C₁₀-Alkylen steht und R₅ aus der aus Wasserstoff, Alkanoyl, Alkoxy, Alkoxyalkyl, Alkoxycarbonyl, wobei das Alkoxycarbonyl unsubstituiert oder durch 1, 2 oder 3 Halogene substituiert ist, Alkylaminocarbonylalkyl, Cycloalkoxycarbonyl, Cycloalkylaminocarbonyl, Cycloalkylaminothiocarbonyl, Cycloalkylalkyl, (Cycloalkyl)oyl, Halogenalkyl, C₁-C₁₀-Alkyl, wobei C₁-C₁₀-Alkyl unsubstituiert oder durch -NRR' substituiert ist, wobei R und R' unabhängig voneinander für Wasserstoff, C₁-C₁₀-Alkyl, -SO₂-A, wobei A für C₁-C₁₀-Alkyl, welches gegebenenfalls durch Alkoxy oder 1-5 Halogene substituiert ist, oder Thioalkoxyalkyl stehen, bestehenden Gruppe ausgewählt ist;
Z für eine kovalente Bindung steht;
R₃ für Cycloalkyl steht, wobei Cycloalkyl unsubstituiert oder durch Substituenten ausgewählt aus der aus (a) Alkoxy, (b) C₁-C₁₀-Alkyl und (c) Halogen bestehenden Gruppe substituiert ist;
und R₄ für Wasserstoff steht, ausgewählt aus der Gruppe bestehend aus:
N-[4-N-(1-Cyclohexyl-6-methylheptan-2-yl)aminomethyl-2-(2-methylphenyl)benzoyl]methionin, Lithiumsalz,
N-[4-(N-2-Cyclohexylethylaminomethyl)-2-(2-methylphenyl)benzoyl]methionin, Trifluoracetatsalz,
N-[4-(N-(2-Cyclohexylethyl)-N-methylaminomethyl)-2-(2-methylphenyl)benzoyl]methionin, Lithiumsalz,
N-[4(N-Acetyl-N-(2-cyclohexylethyl)aminomethyl)-2-(2-methylphenyl)benzoyl]methionin, Lithiumsalz,
N-[4-(N-(N,N-Dimethylamino(carbonyl)-N-(2-cyclohexylethylaminomethyl)-2-(2-methylphenyl)benzoyl]-methionin,
N-[4-(N-(2-Cyclohexylethyl)-N-methansulfonylaminomethyl)-2-(2-methylphenyl)benzoyl]methionin, Lithiumsalz,
N-[4-(3-Cyclohexylpropan-2-ylaminomethyl)-2-(2-methylphenyl)benzoyl]methionin,
N-[4-(4-Cyclohexylbutan-3-ylaminomethyl)-2-(2-methylphenyl)benzoyl]methionin, Lithiumsalz,
N-[4-(6-Cyclohexylhexan-5-ylaminomethyl)-2-(2-methylphenyl)benzoyl]methionin, Lithiumsalz,
N-[4-(3-Cyclohexyl-1-t-butylthiopropan-2-ylaminomethyl)-2-(2-methylphenyl)benzoyl]methionin, Lithiumsalz,
N-[4-N-t-Butyloxycarbonyl-N-2-cyclohexylethylaminomethyl-2-(2-methylphenyl)benzoyl]methionin, Lithiumsalz,
N-[4-(N-2-Cyclohexylethyl-N-cyclopropylaminomethyl)-2-(2-methylphenyl)benzoyl]methionin,
N-[4-(N-(2-Cyclohexylethyl)-N-isopropylaminomethyl)-2-(2-methylphenyl)benzoyl]methionin,
N-[4-(N(Butansulfonyl-N-(2-cyclohexylethyl)-aminomethyl)-2-(2-methylphenyl)benzoyl]methionin,
N-[4-(N-Methyl-N-(1,1-dimethyl-2-cyclohexylethyl)aminomethyl)-2-(2-methylphenyl)benzoyl]-methionin, Lithiumsalz,
N-[4-(N-Cyclohexylmethylaminoethyl)-2-(2-methylphenyl)benzoyl]methionin, Lithiumsalz,
N-[4-(N,N-Di(cyclohexylmethyl)aminoethyl)-2-(2-methylphenyl)benzoyl]methionin, Lithiumsalz,
N-[4-(N-Cyclohexylmethyl-N-1-adamantanoylaminoethyl)-2-(2-methylphenyl)benzoyl]methionin, Lithiumsalz,
N-[4-(N-Cyclohexylmethyl-N-t-butoxycarbonylaminoethyl)-2-(2-methylphenyl)benzoyl]methionin, Lithiumsalz,
N-[4-(N-Cyclohexylmethyl-N-2-ethylhexyloxycarbonylaminoethyl)-2-(2-methylphenyl)benzoyl]-methionin, Lithiumsalz,
N-[4-(N-Cyclohexylmethyl-N-2,2,2-trichlorethoxycarbonylaminoethyl)-2-(2-methylphenyl)benzoyl]-methionin, Lithiumsalz,
N-[4-(N-Cyclohexylmethyl-N-cyclohexyloxycarbonylaminoethyl)-2-(2-methylphenyl)benzoyl]methionin, Lithiumsalz,
N-[4-(N-Cyclohexylmethyl-N-adamantyloxycarbonylaminoethyl)-2-(2-methylphenyl)benzoyl]methionin, Lithiumsalz,
N-[4-(N-Cyclohexylmethyl-N-adamant-1-aminocarbonylaminoethyl)2-(2-methylphenyl)benzoyl]methionin, Lithiumsalz,
N-[4-(N-Cyclohexylmethyl-N-adamant-1-aminothiocarbonylaminoethyl)-2-(2-methylphenyl)benzoyl]-methionin, Lithiumsalz,
N-[4-(N-(2-Cyclohexyl-2-methylpropyl)-N-methylaminomethyl)-2-(2-methylphenyl)benzoyl]methionin, Lithiumsalz,
N-[4-(N-(2-Cyclohexylethyl)-N-2-fluorethylaminomethyl)-2-(2-methylphenyl)benzoyl]methionin,
N-[4-(N-(2-Cyclohexylethyl)-N-2,2,2-trifluorethylaminomethyl)-2-(2-methylphenyl)benzoyl]methionin, Lithiumsalz,
N-[4-(N-(2-Cyclohexylethyl)-N-2-methoxyethylaminomethyl)-2-(2-methylphenylbenzoyl]methionin,
N-[4(N-(2-Cyclohexylethyl)-N-2-methylthioethylaminomethyl)-2-(2-methylphenyl)benzoyl]methionin,
N-[4-(N-(2-Cyclohexylethyl)-N-1-methyl-2(S)-methylthioethylaminomethyl)-2-(2-methylphenyl)benzoyl]-methionin,
N-[4-(N-(2-Cyclohexylethyl)-N-2,N,N-dimethylaminomethyl)-2-(2-methylphenyl)benzoyl]methionin,
N-[4-(N-Cyclohexylmethyl-N-1)butylaminoethyl)-2-(2-methylphenyl)benzoyl]methionin, Lithiumsalz,
N-[4-(N-Cyclohexylpropyl)-2-(2-methylphenyl)-benzoyl]methionin, Lithiumsalz,
N-[4-(N-Cyclohexyl-N-propanoylaminopropyl)-2-(2-methylphenyl)benzoyl]methionin,
N-[4-(N-Cyclohexyl-N-butylaminopropyl)-2-(2-methylphenyl)benzoyl]methionin, Lithiumsalz,
3-[4-(N-Cyclohexyl-N-methylaminomethyl)-2-(2-methylphenyl)benzoylmethyl]-4-methylthiobuttersäure N-[4-(N-(2-Cyclohexylethyl)-N-butylaminomethyl)-2-(2-methylphenyl)benzoyl]methionin, Lithiumsalz,
N-[4-N-t-Butyl-N-(2-cyclohexylethyl)aminomethyl-2-(2-methylphenyl)benzoyl]methionin,
N-[4-N-(2-Cyclohexylethyl)-N-(pent-2-yl)aminomethyl-2-(2-methylphenyl)benzoyl]methionin,
N-[4-N-(2-Cyclohexylethyl)-N-(pent-2-yl)aminomethyl-2-(2-methylphenyl)]benzoyl]methionin,
N-[4-N-(2-Cyclohexylethyl)-N-[propyloxyaminomethyl-2-(2-methylphenyl)benzoyl]methionin, Lithiumsalz,
N-[4-N-(2-Cyclohexylethyl)-N-propansulfonylaminomethyl-2-(2-methylphenyl)benzoyl]methionin,
N-[4-N-(3-Chlorpropansulfonyl)-N-(2-cyclohexylethyl)aminomethyl-2-(2-methylphenyl)benzoyl]methionin,
N-[4-N-(2-Cyclohexylethyl)-N-3-ethoxypropansulfonyl)aminomethyl-2-(2-methylphenyl)benzoyl]-methionin, Lithiumsalz,
N-[4-N-(2-Cyclohexylethyl)-N-(3-trifluormethylpropansulfonyl)aminomethyl-2-(2-methylphenyl)benzoyl]-methionin,
N-[4-N-(Butansulfonyl)-1-N-(3-cyclohexylpropyl)-aminomethyl-2-(2-methylphenyl)benzoyl]methionin, Lithiumsalz,
N-[4-(N-(2-Cyclohexylethyl)-N-methylaminomethyl)-2-phenylbenzoyl]methionin, Lithiumsalz.

2. Verbindung nach Anspruch 1, bei der es sich um N-[4-(N-(2-Cyclohexylethyl)-N-butylaminomethyl)-2-(2-methylphenyl)benzoyl]methionin, Lithiumsalz handelt.

3. Verbindungen nach Anspruch 1, zur Verwendung beim Inhibieren von Proteinisoprenyltransferasen in Säugetieren.

4. Verbindungen nach Anspruch 1, alleine oder in Kombination mit einem anderen Chemotherapeutikum, zur Verwendung bei der Inhibierung oder Behandlung von Krebs in einem Säugetier.

5. Zusammensetzung zur Verwendung bei der Behandlung von Krebs, enthaltend eine Verbindung nach Anspruch 1 in Kombination mit einem anderen Chemotherapeutikum und einem pharmazeutisch unbedenklichen Träger.

6. Verbindungen nach Anspruch 1 zur Verwendung beim Inhibieren der posttranslationalen Modifikation des onkogenen Ras-Proteins durch Proteinfarnesyltransferase, Proteingeranylgeranyltransferase oder beiden in einem Säugetier.

7. Zusammensetzung zur Verwendung beim Inhibieren der posttranslationalen Modifikation des onkogenen Ras-Proteins durch Proteinfarnesyltransferase, Proteingeranylgeranyltransferase oder beiden, enthaltend eine Verbindung nach Anspruch 1 in Kombination mit einem pharmazeutischen Träger.

8. Verbindungen nach Anspruch 1 zur Verwendung bei der Behandlung von mit Restenose assoziierter Intimahyperplasie und Atherosklerose in einem Säugetier.

9. Zusammensetzung zur Verwendung bei der Behandlung von Restenose in einem Säugetier, enthaltend eine Verbindung nach Anspruch 1 in Kombination mit einem pharmazeutisch unbedenklichen Träger.

## Revendications

1. Composé répondant à la Formule I 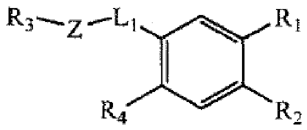
ou un sel pharmaceutiquement acceptable de celui-ci, dans laquelle
R₁ est 2-méthylphényle ;
R₂ est -L₁₁-C(R₁₄) (Rᵥ)C(O)OR₁₅, où L₁₁ est -C(W)N(R) - où R est hydrogène et W est O, Rᵥ est choisi dans le groupe constitué par hydrogène et alkyle inférieur, R₁₅ est choisi dans le groupe constitué par (a) hydrogène, (b) alcanoyloxyalkyle, (c) C₁-C₁₀alkyle et (d) un groupement protecteur de carboxy, et R₁₄ est thioalcoxyalkyle ;
L₁ est -L₄-N(R₅)-L₅, où L₄ est absent ou est C₁-C₁₀-alkylène, L₅ est absent ou est C₁-C₁₀-alkylène, et R₅ est choisi dans le groupe constitué par hydrogène, alcanoyle, alcoxy, alcoxyalkyle, alcoxycarbonyle, où alcoxycarbonyle est non substitué ou substitué par 1, 2 ou 3 halogènes, alkylaminocarbonylalkyle, cycloalcoxycarbonyle, cycloalkylaminocarbonyle, cycloalkylaminothiocarbonyle, cycloalkylalkyle, (cycloalkyl)oyle, halogénoalkyle, C₁-C₁₀alkyle, où C₁-C₁₀alkyle est non substitué ou substitué par -NRR', où R et R' sont indépendamment hydrogène, C₁-C₁₀alkyle ; -SO₂-A, où A est C₁-C₁₀alkyle éventuellement substitué par alcoxy ou 1-5 halogène(s); ou thioalcoxyalkyle ;
Z est une liaison covalente ;
R₃ est cycloalkyle, où cycloalkyle est non substitué ou substitué par des substituants choisis dans le groupe constitué par (a) alcoxy, (b) C₁-C₁₀alkyle, et (c) halogène ;
et R₄ est hydrogène,
choisi dans le groupe constitué par :
la N-[4-N-(1-cyclohexyl-6-méthylheptan-2-yl)aminométhyl-2-(2-méthylphényl)benzoyl]méthionine, sel de lithium,
la N-[4-(N-2-cyclohexyléthylaminométhyl)-2-(2-méthylphényl)benzoyl]méthionine, sel de trifluoroacétate,
la N-[4-(N-(2-cyclohexyléthyl)-N-méthylaminométhyl)-2-(2-méthylphényl)benzoyl]méthionine, sel de lithium,
la N-[4(N-acétyl-N-(2-cyclohexyléthyl)aminométhyl)-2-(2-méthylphényl)benzoyl]méthionine, sel de lithium,
la N-[4-(N-(N,N-diméthylamino(carbonyl)-N-(2-cyclohexyléthylaminométhyl)-2-(2-méthylphényl)benzoyl]-méthionine,
la N-[4-(N-(2-cyclohexyléthyl)-N-méthanesulfonylaminométhyl)-2-(2-méthylphényl)benzoyl]méthionine, sel de lithium,
la N-[4-(3-cyclohexylpropan-2-ylaminométhyl)-2-(2-méthylphényl)benzoyl]méthionine,
la N-[4-(4-cyclohexylbutan-3-ylaminométhyl)-2-(2-méthylphényl)benzoyl]méthionine, sel de lithium,
la N-[4-(6-cyclohexylhexan-5-ylaminométhyl)-2-(2-méthylphényl)benzoyl]méthionine, sel de lithium,
la N-[4-3-cyclohexyl-1-t-butylthiopropan-2-ylaminométhyl)-2-(2-méthylphényl)benzoyl]méthionine. sel de lithium,
la N-[4-N-t-butyloxycarbonyl-N-2-cyclohexyléthylaminométhyl-2-(2-méthylphényl)benzoyl]-méthionine, sel de lithium,
la N-[4-(N-2-cyclohexyléthyl-N-cyclopropylaminométhyl)-2-(2-méthylphényl)benzoyl]méthionine,
la N-[4-(N-(2-cyclohexyléthyl)-N-isopropylaminométhyl)-2-(2-méthylphényl)benzoyl]méthionine,
la N-[4-(N(butanesulfonyl-N-(2-cyclohexyléthyl)-aminométhyl)-2-(2-méthylphényl)benzoyl]méthionine,
la N-[4-(N-méthyl-N-(1,1-diméthyl-2-cyclo hexyléthyl)aminométhyl)-2-(2-méthylphényl)benzoyl]-méthionine, sel de lithium,
la N-[4-(N-cyclohexylméthylaminoéthyl)-2-(2-méthylphényl)benzoyl]méthionine, sel de lithium,
la N-[4-(N,N-di(cyclohexylméthyl)aminoéthyl)-2-(2-méthylphényl)benzoyl]méthionine, sel de lithium,
la N-[4-(N-cyclohexylméthyl-N-1-adamantanoylaminoéthyl)-2-(2-méthylphényl)benzoyl]méthionine, sel de lithium,
la N-[4-(N-cyclohexylméthyl-N-t-butoxycarbonylaminoéthyl)-2-(2-méthylphényl)benzoyl]méthionine, sel de lithium,
la N-[4-(N-cyclohexylméthyl-N-2-éthylhexyloxycarbonylaminoéthyl)-2-(2-méthylphényl)benzoyl]-méthionine, sel de lithium,
la N-[4-(N-cyclohexylméthyl-N-2,2,2-trichloroéthoxycarbonylaminoéthyl)-2-(2-méthylphényl)benzoyl]-méthionine, sel de lithium,
la N-[4-(N-cyclohexylméthyl-N-cyclohexyloxycarbonylaminoéthyl)-2-(2-méthylphényl)benzoyl]méthionine, sel de lithium,
la N-[4-(N-cyclohexylméthyl-N-adamantyloxycarbonylaminoéthyl)-2-(2-méthylphényl)benzoyl]méthionine, sel de lithium,
la N-[4-(N-cyclohexylméthyl-N-adamant-1-aminocarbonylaminoéthyl)2-(2-méthylphénylbenzoyl]méthionine, sel de lithium,
la N-[4-(N-cyclohexylméthyl-N-adamant-1-aminothiocarbonylaminoéthyl)-2-(2-méthylphényl)benzoyl]-méthionine, sel de lithium,
la N-[4-(N-(2-cyclohexyl-2-méthylpropyl)-N-méthylaminométhyl)-2-(2-méthylphényl)benzoyl]méthionine, sel de lithium,
la N-[4-(N-(2-cyclohexyléthyl)-N-2-fluoroéthylaminométhyl)-2-(2-méthylphényl)benzoyl]méthionine,
la N-[4-(N-(2-cyclohexyléthyl)-N-2,2,2-trifluoroéthylaminométhyl)-2-(2-méthylphényl)benzoyl]méthionine, sel de lithium,
la N-[4-(N-(2-cyclohexyléthyl)-N-2-méthoxyéthylaminométhyl)-2-(2-méthylphényl)benzoyl]méthionine,
la N-[4(N-(2-cyclohexyléthyl)-N-2-méthylthioéthylaminométhyl)-2-(2-méthylphényl)benzoyl]méthionine,
la N-[4-(N-(2-cyclohexyléthyl)-N-1-méthyl-2(S)-méthylthioéthylaminométhyl)-2-(2-méthylphényl)benzoyl]-méthionine,
la N-[4-(N-(2-cyclohexyléthyl)-N-2,N,N-diméthylaminométhyl)-2-(2-méthylphényl)benzoyl]méthionine,
la N-[4-(N-cyclohexylméthyl-N-1)butylaminoéthyl)-2-(2-méthylphényl)benzoyl]méthionine, sel de lithium,
la N-[4-(N-cyclohexylpropyl)-2-(2-méthylphényl)-benzoyl]méthionine, sel de lithium,
la N-[4-(N-cyclohexyl-N-propanoylaminopropyl)-2-(2-méthylphényl)benzoyl]méthionine,
la N-[4-(N-cyclohexyl-N-butylaminopropyl)-2-(2-méthylphényl)benzoyl]méthionine, sel de lithium,
l'acide 3-[4-(N-cyclohexyl-N-méthylaminométhyl)-2-(2-méthylphényl)benzoylméthyl]-4-méthylthiobutyrique,
la N-[4-(N-(2-cyclohexyléthyl)-N-butylaminométhyl)-2-(2-méthylphényl)benzoyl]méthionine, sel de lithium,
la N-[4-N-t-butyl-N-(2-cyclohexyléthyl)aminométhyl-2-(2-méthylphényl)benzoyl]méthionine,
la N-[4-N-(2-cyclohexyléthyl)-N-(pent-2-yl)aminométhyl-2-(2-méthylphényl)benzoyl]méthionine,
la N-[4-N-(2-cyclohexyléthyl)-N-(pent-2-yl)aminométhyl-2-(2-méthylphényl)]benzoyl]méthionine,
la N-[4-N-(2-cyclohexyléthyl)-N-[propyloxyaminométhyl-2-(2-méthylphényl)benzoyl]méthionine, sel de lithium,
la N-[4-N-(2-cyclohexyléthyl)-N-propanesulfonylaminométhyl-2-(2-méthylphénylbenzoyl]méthionine,
la N-[4-N-(3-chloropropanesulfonyl)-N-(2-cyclohexyléthyl)aminométhyl-2-(2-méthylphényl)benzoyl]méthionine,
la N-[4-N-(2-cyclohexyléthyl)-N-3-éthoxypropanesulfonyl)aminométhyl-2-(2-méthylphényl)benzoyl]-méthionine, sel de lithium,
la N-[4-N-(2-cyclohexyléthyl)-N-(3-trifluorométhylpropanesulfonyl)aminométhyl-2-(2-méthylphényl)benzoyl]-méthionine,
la N-[4-N-(butanesulfonyl)-1-N-(3-cyclohexylpropyl)-aminométhyl-2-(2-méthylphényl)benzoyl]méthionine, sel de lithium,
la N-[4-(N-(2-cyclohexyléthyl)-N-méthylaminométhyl)-2-phénylbenzoyl]méthionine, sel de lithium.

2. Composé selon la revendication 1, qui est la N-[4-(N-(2-cyclohexyléthyl)-N-butylaminométhyl)-2-(2-méthylphényl)benzoyl]méthionine, sel de lithium.

3. Composé selon la revendication 1, pour une utilisation dans l'inhibition des protéine isoprényltransférases chez les mammifères.

4. Composé selon la revendication 1, seul ou en association avec un autre agent chimiothérapeutique, pour une utilisation dans l'inhibition ou le traitement du cancer chez un mammifère.

5. Composition pour une utilisation dans le traitement du cancer, comprenant un composé selon la revendication 1, en association avec un autre agent chimiothérapeutique et un véhicule pharmaceutiquement acceptable.

6. Composé selon la revendication 1, pour une utilisation dans l'inhibition de la modification post-traductionnelle de la protéine Ras oncogène par la protéine farnésyltransférase, la protéine géranylgéranyltransférase, ou les deux, chez un mammifère.

7. Composition pour une utilisation dans l'inhibition de la modification post-traductionnelle de la protéine Ras oncogène par la protéine farnésyltransférase, la protéine géranylgéranyltransférase, ou les deux, comprenant un composé selon la revendication 1 en association avec un véhicule pharmaceutique.

8. Composé selon la revendication 1, pour une utilisation dans le traitement de l'hyperplasie intimale associée à la resténose et l'athérosclérose chez un mammifère.

9. Composition pour une utilisation dans le traitement de la resténose chez un mammifère, comprenant un composé selon la revendication 1 en association avec un véhicule pharmaceutiquement acceptable.
